# EUROPEAN PATENT SPECIFICATION

(11) **EP 2 305 715 B1**
(45) Date of publication and mention of the grant of the patent: **07.12.2016**
(21) Application number: 10013041.8
(22) Date of filing: 23.02.2001
(51) Int. Cl.: C07K 16/28, A61K 39/395, A61P 19/10, C07K 14/705, C07K 14/52, A61K 45/06

(54) **Monoclonal antibody to osteoprotegerin binding protein**
Monoklonaler Antikörper gegen das Osteoprotegerin-bindenden Protein
Anticorps monoclonal contre la protéine de liaison d'ostéoprotégérine

(30) Priority: 23.02.2000 US 511139; 22.02.2001 US 791153
(43) Date of publication of application: 06.04.2011
(62) Divisional of application: 08010991.1
(73) Proprietor: Amgen, Inc, Thousand Oaks, CA 91320-1799 (US)
(72) Inventor: Deshpande, Rajendra V., Thousand Oaks, CA 91320 (US); Hitz, Anna, Burlingame, CA 94030 (US); Boyle, William J., Malibu, CA 90265 (US); Sullivan, John K., Newbury Park, CA 91320 (US)
(74) Representative: Grünecker Patent- und Rechtsanwälte PartG mbB

(56) References cited:
- WO-A1-98/46751
- K. TSUKII ET AL.: "Osteoclast differentiation factor mediates an essential signal for bone resorption induced by 1alpha,25-dihydroxyvitamin D3, prostaglandin E2, or parathyroid hormone in the microenvironment of bone.", BIOCHEMICAL AND BIOPHYSICAL RESEARCH COMMUNICATIONS, vol. 246, no. 2, 19 May 1998 (1998-05-19), pages 337-341, XP002169940, USA
- N. TAKAHASHI ET AL.: "A new member of tumor necrosis factor ligand family, ODF/OPGL/TRANCE/RANKL, regulates osteoclast differentiation and function.", BIOCHEMICAL AND BIOPHYSICAL RESEARCH COMMUNICATIONS, vol. 256, no. 3, 24 March 1999 (1999-03-24), pages 449-455, XP000877384, USA
- M. VAN REGENMORTEL: "Structural and functional approaches to the study of protein antigenicity.", IMMUNOLOGY TODAY, vol. 10, no. 8, 1989, pages 266-272, XP025809625,

### SEQUENCE LISTING

<110> Amgen Inc.
<120> SELECTIVE BINDING AGENTS OF OSTEOPROTEGERIN BINDING PROTEIN
<130> EP25150IIIHV015pau
<140> not yet assigned
   <141> herewith
<150> EP 08 010 991.1
   <151> 2008-06-16
<150> EP 01 911 158.2
   <151> 2001-02-23
<150> PCT/US01/05973
   <151> 2001-02-23
<150> 09/791,153
   <151> 2001-02-22
<150> 09/511,139
   <151> 2000-02-23
<160> 155
<170> PatentIn version 3.3
<210> 1
   <211> 11
   <212> PRT
   <213> Homo sapiens
<400> 1
   Arg Ala Ser Gln Ser Ile Ser Arg Tyr Leu Asn
   1 5 10
<210> 2
   <211> 11
   <212> PRT
   <213> Homo sapiens
<400> 2
   Arg Ala Ser Gln Ser Val Gly Ser Tyr Leu Ala
   1 5 10
<210> 3
   <211> 12
   <212> PRT
   <213> Homo sapiens
<400> 3
   Arg Ala Ser Gln Ser Val Ser Ser Ser Ser Leu Ala
   1 5 10
<210> 4
   <211> 9
   <212> PRT
   <213> Homo sapiens
<400> 4
   Ser Gly Asp Ala Leu Pro Lys Gln Tyr
   1 5
<210> 5
   <211> 7
   <212> PRT
   <213> Homo sapiens
<400> 5
   Gly Ala Ser Ser Leu Gln Ser
   1 5
<210> 6
   <211> 7
   <212> PRT
   <213> Homo sapiens
<400> 6
   Asp Ala Thr Asn Arg Ala Thr
   1 5
<210> 7
   <211> 7
   <212> PRT
   <213> Homo sapiens
<400> 7
   Gly Ala Ser Ser Arg Ala Thr
   1 5
<210> 8
   <211> 7
   <212> PRT
   <213> Homo sapiens
<400> 8
   Glu Asp Ser Glu Arg Pro Ser
   1 5
<210> 9
   <211> 5
   <212> PRT
   <213> Homo sapiens
<400> 9
   Gln His Thr Arg Ala
   1 5
<210> 10
   <211> 5
   <212> PRT
   <213> Homo sapiens
<400> 10
   Gln His Arg Arg Thr
   1 5
<210> 11
   <211> 5
   <212> PRT
   <213> Homo sapiens
<400> 11
   Gln Gln Tyr Gly Ala
   1 5
<210> 12
   <211> 11
   <212> PRT
   <213> Homo sapiens
<400> 12
   Gln Ser Thr Asp Ser Ser Gly Thr Tyr Val Val
   1 5 10
<210> 13
   <211> 5
   <212> PRT
   <213> Homo sapiens
<400> 13
   Asn Tyr Ala Ile His
   1               5
<210> 14
   <211> 5
   <212> PRT
   <213> Homo sapiens
<400> 14
   Asn Tyr Pro Met His
   1               5
<210> 15
   <211> 5
   <212> PRT
   <213> Homo sapiens
<400> 15
   Asp Tyr Ala Met His
   1               5
<210> 16
   <211> 17
   <212> PRT
   <213> Homo sapiens
<400> 16
   Trp Ile Asn Ala Gly Asn Gly Asn Thr Lys Phe Ser Gln Lys Phe Gln
   1               5                   10                  15
   Gly
<210> 17
   <211> 17
   <212> PRT
   <213> Homo sapiens
<400> 17
   Val Ile Ser Tyr Asp Gly Asn Asn Lys Tyr Tyr Ala Asp Ser Val Lys
   1               5                   10                  15
   Gly
<210> 18
   <211> 17
   <212> PRT
   <213> Homo sapiens
<400> 18
   Gly Ile Ser Trp Asn Ser Gly Arg Ile Gly Tyr Ala Asp Ser Val Lys
   1               5                   10                  15
   Gly
<210> 19
   <211> 17
   <212> PRT
   <213> Homo sapiens
<400> 19
   Asp Ser Ser Asn Met Val Arg Gly Ile Ile Ile Ala Tyr Tyr Phe Asp
   1               5                   10                  15
   Tyr
<210> 20
   <211> 7
   <212> PRT
   <213> Homo sapiens
<400> 20
   Gly Gly Gly Gly Phe Asp Tyr
   1               5
<210> 21
   <211> 14
   <212> PRT
   <213> Homo sapiens
<400> 21
   Gly Gly Ser Thr Ser Ala Arg Tyr Ser Ser Gly Trp Tyr Tyr
   1               5                   10
<210> 22
   <211> 18
   <212> DNA
   <213> Homo sapiens
<400> 22
   ccgactttgc acctagtt 18
<210> 23
   <211> 24
   <212> DNA
   <213> Homo sapiens
<400> 23
   tttgtcgtct ttccagacgt tagt 24
<210> 24
   <211> 20
   <212> PRT
   <213> Homo sapiens
<400> 24
   Thr Asp Ile Pro Ser Gly Ser His Lys Val Ser Leu Ser Ser Trp Tyr
   1               5                   10                  15
   His Asp Arg Gly
               20
<210> 25
   <211> 19
   <212> PRT
   <213> Homo sapiens
<400> 25
   Met Glu Trp Ser Trp Val Phe Leu Phe Phe Leu Ser Val Thr Thr Gly
   1               5                   10                  15
   Val His Ser
<210> 26
   <211> 45
   <212> DNA
   <213> Homo sapiens
<220>
   <221> CDS
   <222> (18)..(44)
<400> 26
   cagaagctta gaccacc atg gaa tgg agc tgg gtc ttt ctc ttc t        45
                      Met Glu Trp Ser Trp Val Phe Leu Phe
                      1               5
<210> 27
   <211> 9
   <212> PRT
   <213> Homo sapiens
<400> 27
   Met Glu Trp Ser Trp Val Phe Leu Phe
   1               5
<210> 28
   <211> 45
   <212> DNA
   <213> Homo sapiens
<220>
   <221> CDS
   <222> (2)..(43)
<400> 28
   g agc tgg gtc ttt ctc ttc ttc ctg tca gta acg act ggt gtc ca         45
     Ser Trp Val Phe Leu Phe Phe Leu Ser Val Thr Thr Gly Val
     1               5                   10
<210> 29
   <211> 14
   <212> PRT
   <213> Homo sapiens
<400> 29
   Ser Trp Val Phe Leu Phe Phe Leu Ser Val Thr Thr Gly Val
   1               5                   10
<210> 30
   <211> 42
   <212> DNA
   <213> Homo sapiens
<220>
   <221> CDS
   <222> (1)..(42)
<400> 30
   tca gta acg act ggt gtc cac tca cag gtc cag ctg gtg cag         42
   Ser Val Thr Thr Gly Val His Ser Gln Val Gln Leu Val Gln
   1               5                   10
<210> 31
   <211> 14
   <212> PRT
   <213> Homo sapiens
<400> 31
   Ser Val Thr Thr Gly Val His Ser Gln Val Gln Leu Val Gln
   1               5                   10
<210> 32
   <211> 30
   <212> DNA
   <213> Homo sapiens
<400> 32
   gtggaggcac tagagacggt gaccagggtg 30
<210> 33
   <211> 22
   <212> PRT
   <213> Homo sapiens
<400> 33
   Met Asp Met Arg Val Pro Ala Gln Leu Leu Gly Leu Leu Leu Leu Trp
   1               5                   10                  15
   Leu Arg Gly Ala Arg Cys
               20
<210> 34
   <211> 48
   <212> DNA
   <213> Homo sapiens
<220>
   <221> CDS
   <222> (17)..(46)
<400> 34
   cagaagcttg accacc atg gac atg agg gtc ccc gct cag ctc ctg gg          48
                     Met Asp Met Arg Val Pro Ala Gln Leu Leu
                     1               5                   10
<210> 35
   <211> 10
   <212> PRT
   <213> Homo sapiens
<400> 35
   Met Asp Met Arg Val Pro Ala Gln Leu Leu
   1               5                   10
<210> 36
   <211> 48
   <212> DNA
   <213> Homo sapiens
<220>
   <221> CDS
   <222> (3)..(47)
<400> 36
   cc gct cag ctc ctg ggg ctc ctg cta ttg tgg ttg aga ggt gcc aga t      48
      Ala Gln Leu Leu Gly Leu Leu Leu Leu Trp Leu Arg Gly Ala Arg
      1               5                   10                  15
<210> 37
   <211> 15
   <212> PRT
   <213> Homo sapiens
<400> 37
   Ala Gln Leu Leu Gly Leu Leu Leu Leu Trp Leu Arg Gly Ala Arg
   1               5                   10                  15
<210> 38
   <211> 44
   <212> DNA
   <213> Homo sapiens
<220>
   <221> CDS
   <222> (2)..(43)
<400> 38
   g tgg ttg aga ggt gcc aga tgt gaa att gtg atg aca cag tct c         44
     Trp Leu Arg Gly Ala Arg Cys Glu Ile Val Met Thr Gln Ser
     1               5                   10
<210> 39
   <211> 14
   <212> PRT
   <213> Homo sapiens
<400> 39
   Trp Leu Arg Gly Ala Arg Cys Glu Ile Val Met Thr Gln Ser
   1               5                   10
<210> 40
   <211> 32
   <212> DNA
   <213> Homo sapiens
<400> 40
   tttggacgtc gacttattaa cactctcccc tg 32
<210> 41
   <211> 4
   <212> PRT
   <213> Homo sapiens
<400> 41
   Cys Glu Gly Arg
   1
<210> 42
   <211> 30
   <212> PRT
   <213> Homo sapiens
<400> 42
   Met Asp Ala Met Lys Arg Gly Leu Cys Cys Val Leu Leu Leu Cys Gly
   1               5                   10                  15
   Ala Val Phe Val Phe Ser Pro Ser Arg Gly Arg Phe Arg Arg
               20                  25                  30
<210> 43
   <211> 645
   <212> DNA
   <213> Homo sapiens
<220>
   <221> CDS
   <222> (1)..(645)
<400> 43
   tct cac agt gca ctt gaa att gtg atg acg cag tct cca tcc tcc ctg      48
   Ser His Ser Ala Leu Glu Ile Val Met Thr Gln Ser Pro Ser Ser Leu
   1               5                   10                  15
   tct gcg tct gtt gga gac aga gtc acc atc act tgc cgg gca agt cag      96
   Ser Ala Ser Val Gly Asp Arg Val Thr Ile Thr Cys Arg Ala Ser Gln
               20                  25                  30
   agc att agc aga tat tta aat tgg tat cag ctt aaa cca ggg aaa gcc      144
   Ser Ile Ser Arg Tyr Leu Asn Trp Tyr Gln Leu Lys Pro Gly Lys Ala
           35                  40                  45
   cct agg ctc ctg atc tat ggt gca tcc agt ttg caa agt gga gtc cca      192
   Pro Arg Leu Leu Ile Tyr Gly Ala Ser Ser Leu Gln Ser Gly Val Pro
       50                  55                  60
   tca agg ttc agt ggc agt gga tct ggg gca gag ttc act ctc acc atc      240
   Ser Arg Phe Ser Gly Ser Gly Ser Gly Ala Glu Phe Thr Leu Thr Ile
   65                  70                  75                  80
   agc agt cta caa cct gaa gac att gcc act tac tac tgt caa cac act      288
   Ser Ser Leu Gln Pro Glu Asp Ile Ala Thr Tyr Tyr Cys Gln His Thr
                   85                  90                  95
   cgg gcg ttc ggc caa ggg acc aag gtt gaa atc aag cga act gtg gct      336
   Arg Ala Phe Gly Gln Gly Thr Lys Val Glu Ile Lys Arg Thr Val Ala
               100                 105                 110
   gca cca tct gtc ttc atc ttc ccg cca tct gat gag cag ttg aaa tct      384
   Ala Pro Ser Val Phe Ile Phe Pro Pro Ser Asp Glu Gln Leu Lys Ser
           115                 120                 125
   gga act gcc tct gtt gtg tgc ctg ctg aat aac ttc tat ccc aga gag      432
   Gly Thr Ala Ser Val Val Cys Leu Leu Asn Asn Phe Tyr Pro Arg Glu
       130                 135                 140
   gcc aaa gta cag tgg aag gtg gat aac gcc ctc caa tcg ggt aac tcc      480
   Ala Lys Val Gln Trp Lys Val Asp Asn Ala Leu Gln Ser Gly Asn Ser
   145                 150                 155                 160
   cag gag agt gcc aca gag cag gac agc aag gac agc acc tac agc ctc      528
   Gln Glu Ser Ala Thr Glu Gln Asp Ser Lys Asp Ser Thr Tyr Ser Leu
                   165                 170                 175
   agc agc acc ctg acg ctg agc aaa gca gac tac gag aaa cac aaa gtc      576
   Ser Ser Thr Leu Thr Leu Ser Lys Ala Asp Tyr Glu Lys His Lys Val
               180                 185                 190
   tac gcc tgc gaa gtc acc cat cag ggc ctg agc tcg ccc gtc aca aag      624
   Tyr Ala Cys Glu Val Thr His Gln Gly Leu Ser Ser Pro Val Thr Lys
           195                 200                 205
   agc ttc aac agg gga gag tgt                                          645
   Ser Phe Asn Arg Gly Glu Cys
       210                 215
<210> 44
   <211> 215
   <212> PRT
   <213> Homo sapiens
<400> 44
   Ser His Ser Ala Leu Glu Ile Val Met Thr Gln Ser Pro Ser Ser Leu
   1               5                   10                  15
   Ser Ala Ser Val Gly Asp Arg Val Thr Ile Thr Cys Arg Ala Ser Gln
               20                  25                  30
   Ser Ile Ser Arg Tyr Leu Asn Trp Tyr Gln Leu Lys Pro Gly Lys Ala
           35                  40                  45
   Pro Arg Leu Leu Ile Tyr Gly Ala Ser Ser Leu Gln Ser Gly Val Pro
       50                  55                  60
   Ser Arg Phe Ser Gly Ser Gly Ser Gly Ala Glu Phe Thr Leu Thr Ile
   65                  70                  75                  80
   Ser Ser Leu Gln Pro Glu Asp Ile Ala Thr Tyr Tyr Cys Gln His Thr
                   85                  90                  95
   Arg Ala Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Ala Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 45
   <211> 645
   <212> DNA
   <213> Homo sapiens
<220>
   <221> CDS
   <222> (1)..(645)
<400> 45
   tct cac agt gca ctt gaa att gtg ctg act cag tct cca gcc acc ctg      48
   Ser His Ser Ala Leu Glu Ile Val Leu Thr Gln Ser Pro Ala Thr Leu
   1               5                   10                  15
   tct ttt tct ccg ggt gaa aga gcc acc ctc tcc tgc agg gcc agt cag      96
   Ser Phe Ser Pro Gly Glu Arg Ala Thr Leu Ser Cys Arg Ala Ser Gln
               20                  25                  30
   agt gtt ggc agc tac tta gcc tgg tac cag cag aga cct ggc cag gct     144
   Ser Val Gly Ser Tyr Leu Ala Trp Tyr Gln Gln Arg Pro Gly Gln Ala
           35                  40                  45
   ccc agg ccc ctc atc tat gat gca acc aac agg gcc act ggc atc cca     192
   Pro Arg Pro Leu Ile Tyr Asp Ala Thr Asn Arg Ala Thr Gly Ile Pro
       50                  55                  60
   acc agg ttc agt ggc agt ggg tct ggg aca gac ttc act ctc acc atc     240
   Thr Arg Phe Ser Gly Ser Gly Ser Gly Thr Asp Phe Thr Leu Thr Ile
   65                  70                  75                  80
   agc agc cta gag cct gaa gat ttt gca act tat tac tgt caa cac cga     288
   Ser Ser Leu Glu Pro Glu Asp Phe Ala Thr Tyr Tyr Cys Gln His Arg
                   85                  90                  95
   agg act ttt ggc cgg ggg acc aag ttg gag atc aaa cga act gtg gct     336
   Arg Thr Phe Gly Arg Gly Thr Lys Leu Glu Ile Lys Arg Thr Val Ala
               100                 105                 110
   gca cca tct gtc ttc atc ttc ccg cca tct gat gag cag ttg aaa tct     384
   Ala Pro Ser Val Phe Ile Phe Pro Pro Ser Asp Glu Gln Leu Lys Ser
           115                 120                 125
   gga act gcc tct gtt gtg tgc ctg ctg aat aac ttc tat ccc aga gag     432
   Gly Thr Ala Ser Val Val Cys Leu Leu Asn Asn Phe Tyr Pro Arg Glu
       130                 135                 140
   gcc aaa gta cag tgg aag gtg gat aac gcc ctc caa tcg ggt aac tcc     480
   Ala Lys Val Gln Trp Lys Val Asp Asn Ala Leu Gln Ser Gly Asn Ser
   145                 150                 155                 160
   cag gag agt gtc aca gag cag gac agc aag gac agc acc tac agc ctc     528
   Gln Glu Ser Val Thr Glu Gln Asp Ser Lys Asp Ser Thr Tyr Ser Leu
                   165                 170                 175
   agc agc acc ctg acg ctg agc aaa gca gac tac gag aaa cac aaa gtc     576
   Ser Ser Thr Leu Thr Leu Ser Lys Ala Asp Tyr Glu Lys His Lys Val
               180                 185                 190
   tac gcc tgc gaa gtc act cat cag ggc ctg agc tcg ccc gtc aca aag     624
   Tyr Ala Cys Glu Val Thr His Gln Gly Leu Ser Ser Pro Val Thr Lys
           195                 200                 205
   agc ttc aac agg gga gag tgt                                         645
   Ser Phe Asn Arg Gly Glu Cys
       210                 215
<210> 46
   <211> 215
   <212> PRT
   <213> Homo sapiens
<400> 46
   Ser His Ser Ala Leu Glu Ile Val Leu Thr Gln Ser Pro Ala Thr Leu
   1               5                   10                  15
   Ser Phe Ser Pro Gly Glu Arg Ala Thr Leu Ser Cys Arg Ala Ser Gln
               20                  25                  30
   Ser Val Gly Ser Tyr Leu Ala Trp Tyr Gln Gln Arg Pro Gly Gln Ala
           35                  40                  45
   Pro Arg Pro Leu Ile Tyr Asp Ala Thr Asn Arg Ala Thr Gly Ile Pro
       50                  55                  60
   Thr Arg Phe Ser Gly Ser Gly Ser Gly Thr Asp Phe Thr Leu Thr Ile
   65                  70                  75                  80
   Ser Ser Leu Glu Pro Glu Asp Phe Ala Thr Tyr Tyr Cys Gln His Arg
                   85                  90                  95
   Arg Thr Phe Gly Arg Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 47
   <211> 645
   <212> DNA
   <213> Homo sapiens
<220>
   <221> CDS
   <222> (1)..(645)
<400> 47
   cac agt gca ctt gaa att gtg atg aca cag tct cca ggc acc ctg tct      48
   His Ser Ala Leu Glu Ile Val Met Thr Gln Ser Pro Gly Thr Leu Ser
   1               5                   10                  15
   ttg tct cca ggg gaa aga gcc acc ctc tcc tgc agg gcc agt cag agt      96
   Leu Ser Pro Gly Glu Arg Ala Thr Leu Ser Cys Arg Ala Ser Gln Ser
               20                      25                      30
   gtt agc agc agc tcc tta gcc tgg tac cag cag aaa cct ggc cag gct      144
   Val Ser Ser Ser Ser Leu Ala Trp Tyr Gln Gln Lys Pro Gly Gln Ala
           35                  40                  45
   ccc agg ctc ctc atc tat ggt gca tcc agc agg gcc act ggc atc cca      192
   Pro Arg Leu Leu Ile Tyr Gly Ala Ser Ser Arg Ala Thr Gly Ile Pro
       50                  55                  60
   gac agg ttc agt ggc agt ggg tct ggg aca gac ttc act ctc acc atc      240
   Asp Arg Phe Ser Gly Ser Gly Ser Gly Thr Asp Phe Thr Leu Thr Ile
   65                  70                  75                  80
   agc aga ctg gag cct gaa gat ttt gca gtg tat tac tgt cag cag tat      288
   Ser Arg Leu Glu Pro Glu Asp Phe Ala Val Tyr Tyr Cys Gln Gln Tyr
                   85                  90                  95
   ggt gct ttc ggc gga ggg acc aag gtg gag atc aaa cga act gtg gct      336
   Gly Ala Phe Gly Gly Gly Thr Lys Val Glu Ile Lys Arg Thr Val Ala
               100                 105                 110
   gca cca tct gtc ttc atc ttc ccg cca tct gat gag cag ttg aaa tct      384
   Ala Pro Ser Val Phe Ile Phe Pro Pro Ser Asp Glu Gln Leu Lys Ser
           115                 120                 125
   gga act gcc tct gtt gtg tgc ctg ctg aat aac ttc tat ccc aga gag      432
   Gly Thr Ala Ser Val Val Cys Leu Leu Asn Asn Phe Tyr Pro Arg Glu
       130                 135                 140
   gcc aaa gta cag tgg aag gtg gat aac gcc ctc caa tcg ggt aac tcc      480
   Ala Lys Val Gln Trp Lys Val Asp Asn Ala Leu Gln Ser Gly Asn Ser
   145                 150                 155                 160
   cag gag agt gtc aca gag cag gac agc aag gac agc acc tac agc ctc      528
   Gln Glu Ser Val Thr Glu Gln Asp Ser Lys Asp Ser Thr Tyr Ser Leu
                   165                 170                 175
   agc agc acc ctg acg ctg agc aaa gca gac tac gag aaa cac aaa gtc      576
   Ser Ser Thr Leu Thr Leu Ser Lys Ala Asp Tyr Glu Lys His Lys Val
               180                 185                 190
   tac gcc tgc gaa gtc acc cat cag ggc ctg aac tcg ccc gtc aca aag      624
   Tyr Ala Cys Glu Val Thr His Gln Gly Leu Asn Ser Pro Val Thr Lys
           195                 200                 205
   agc ttc aac agg gga gag tgt                                          645
   Ser Phe Asn Arg Gly Glu Cys
       210                 215
<210> 48
   <211> 215
   <212> PRT
   <213> Homo sapiens
<400> 48
   His Ser Ala Leu Glu Ile Val Met Thr Gln Ser Pro Gly Thr Leu Ser
   1               5                   10                  15
   Leu Ser Pro Gly Glu Arg Ala Thr Leu Ser Cys Arg Ala Ser Gln Ser
               20                  25                  30
   Val Ser Ser Ser Ser Leu Ala Trp Tyr Gln Gln Lys Pro Gly Gln Ala
           35                  40                  45
   Pro Arg Leu Leu Ile Tyr Gly Ala Ser Ser Arg Ala Thr Gly Ile Pro
       50                  55                  60
   Asp Arg Phe Ser Gly Ser Gly Ser Gly Thr Asp Phe Thr Leu Thr Ile
   65                  70                  75                  80
   Ser Arg Leu Glu Pro Glu Asp Phe Ala Val Tyr Tyr Cys Gln Gln Tyr
                   85                  90                  95
   Gly Ala Phe Gly Gly Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Asn Ser Pro Val Thr Lys
           195                 200                 205
   Ser Phe Asn Arg Gly Glu Cys
       210                 215
<210> 49
   <211> 654
   <212> DNA
   <213> Homo sapiens
<220>
   <221> CDS
   <222> (1)..(654)
<400> 49
   tct cac agt gca cag tct gtg ctg act cag cca ccc tcg gtg tca gtg      48
   Ser His Ser Ala Gln Ser Val Leu Thr Gln Pro Pro Ser Val Ser Val
   1               5                   10                  15
   tcc cca gga cag acg gcc acg atc acc tgc tct gga gat gca ttg cca      96
   Ser Pro Gly Gln Thr Ala Thr Ile Thr Cys Ser Gly Asp Ala Leu Pro
               20                  25                  30
   aag caa tat gtt tat tgg tac cgg cag aag cca ggc cag gcc cct cta     144
   Lys Gln Tyr Val Tyr Trp Tyr Arg Gln Lys Pro Gly Gln Ala Pro Leu
           35                  40                  45
   ttg gtg ata tat gaa gac agt gag agg ccc tca ggg atc cct gaa cga     192
   Leu Val Ile Tyr Glu Asp Ser Glu Arg Pro Ser Gly Ile Pro Glu Arg
       50                  55                  60
   ttc tct ggc tcc agt tca ggg act gaa gtc acg ttg agt atc agt gga     240
   Phe Ser Gly Ser Ser Ser Gly Thr Glu Val Thr Leu Ser Ile Ser Gly
   65                  70                  75                  80
   gtc cag gca gaa gac gag gct gac tat tat tgt caa tca aca gac agc     288
   Val Gln Ala Glu Asp Glu Ala Asp Tyr Tyr Cys Gln Ser Thr Asp Ser
                   85                  90                  95
   agt ggg act tat gtc gtc ttc ggc gga ggg acc aag ctg acc gtc cta     336
   Ser Gly Thr Tyr Val Val Phe Gly Gly Gly Thr Lys Leu Thr Val Leu
               100                 105                 110
   agt cag ccc aag gct gcc ccc tcg gtc act ctg ttc ccg ccc tcc tct     384
   Ser Gln Pro Lys Ala Ala Pro Ser Val Thr Leu Phe Pro Pro Ser Ser
           115                 120                 125
   gag gag ctt caa gcc aac aag gcc aca ctg gtg tgt ctc ata agt gac     432
   Glu Glu Leu Gln Ala Asn Lys Ala Thr Leu Val Cys Leu Ile Ser Asp
       130                 135                 140
   ttc tac ccg gga gcc gtg aca gtg gcc tgg aag gca gat agc agc ccc     480
   Phe Tyr Pro Gly Ala Val Thr Val Ala Trp Lys Ala Asp Ser Ser Pro
   145                 150                 155                 160
   gtc aag gcg gga gtg gag acc acc aca ccc tcc aaa caa agc aac aac     528
   Val Lys Ala Gly Val Glu Thr Thr Thr Pro Ser Lys Gln Ser Asn Asn
                   165                 170                 175
   aag tac gcg gcc agc agc tat ctg agc ctg acg cct gag cag tgg aag     576
   Lys Tyr Ala Ala Ser Ser Tyr Leu Ser Leu Thr Pro Glu Gln Trp Lys
               180                 185                 190
   tcc cac aga agc tac agc tgc cag gtc acg cat gaa ggg agc acc gtg     624
   Ser His Arg Ser Tyr Ser Cys Gln Val Thr His Glu Gly Ser Thr Val
           195                 200                 205
   gag aag aca gtg gcc cct aca gaa tgt tca                             654
   Glu Lys Thr Val Ala Pro Thr Glu Cys Ser
       210                 215
<210> 50
   <211> 218
   <212> PRT
   <213> Homo sapiens
<400> 50
   Ser His Ser Ala Gln Ser Val Leu Thr Gln Pro Pro Ser Val Ser Val
   1               5                   10                  15
   Ser Pro Gly Gln Thr Ala Thr Ile Thr Cys Ser Gly Asp Ala Leu Pro
               20                  25                  30
   Lys Gln Tyr Val Tyr Trp Tyr Arg Gln Lys Pro Gly Gln Ala Pro Leu
           35                  40                  45
   Leu Val Ile Tyr Glu Asp Ser Glu Arg Pro Ser Gly Ile Pro Glu Arg
       50                  55                  60
   Phe Ser Gly Ser Ser Ser Gly Thr Glu Val Thr Leu Ser Ile Ser Gly
   65                  70                  75                  80
   Val Gln Ala Glu Asp Glu Ala Asp Tyr Tyr Cys Gln Ser Thr Asp Ser
                   85                  90                  95
   Ser Gly Thr Tyr Val Val Phe Gly Gly Gly Thr Lys Leu Thr Val Leu
               100                 105                 110
   Ser Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
       210                 215
<210> 51
   <211> 690
   <212> DNA
   <213> Homo sapiens
<220>
   <221> CDS
   <222> (1)..(690)
<400> 51
   gcc cag gtc cag ctg gtg cag tct ggg gct gag gtg agg aag cct ggg 48
   Ala Gln Val Gln Leu Val Gln Ser Gly Ala Glu Val Arg Lys Pro Gly
   1 5 10 15
   gcc tca gtg aag gtt tcc tgc aag gct tct gga tac gac ttc agt aat 96
   Ala Ser Val Lys Val Ser Cys Lys Ala Ser Gly Tyr Asp Phe Ser Asn
   20 25 30
   tat gct ata cat tgg gtg cgc cag gcc ccc gga caa agg ctt gag tgg 144
   Tyr Ala Ile His Trp Val Arg Gln Ala Pro Gly Gln Arg Leu Glu Trp
   35 40 45
   atg gga tgg atc aac gct ggc aat ggg aac aca aaa ttt tca cag aag 192
   Met Gly Trp Ile Asn Ala Gly Asn Gly Asn Thr Lys Phe Ser Gln Lys
   50 55 60
   ttc cag ggc aga atc acc gtt acc agg gac aca gcc gcg agc aca gcc 240
   Phe Gln Gly Arg Ile Thr Val Thr Arg Asp Thr Ala Ala Ser Thr Ala
   65 70 75 80
   tac atg gag ctg cgc agt ctg aga tct gaa gac acg gct gtg tat tac 288
   Tyr Met Glu Leu Arg Ser Leu Arg Ser Glu Asp Thr Ala Val Tyr Tyr
   85 90 95
   tgt gcg aga gat tcc tca aat atg gtt cgg gga att att ata gcg tac 336
   Cys Ala Arg Asp Ser Ser Asn Met Val Arg Gly Ile Ile Ile Ala Tyr
   100 105 110
   tat ttt gac tac tgg ggc cag ggc acc ctg gtc acc gtc tca agc gcc 384
   Tyr Phe Asp Tyr Trp Gly Gln Gly Thr Leu Val Thr Val Ser Ser Ala
   115 120 125
   tcc acc aag ggc cca tcg gtc ttc ccc ctg gca ccc tcc tcc aag agc 432
   Ser Thr Lys Gly Pro Ser Val Phe Pro Leu Ala Pro Ser Ser Lys Ser
   130 135 140
   acc tct ggg ggc aca gcg gcc ctg ggc tgc ctg gtc aag gac tac ttc 480
   Thr Ser Gly Gly Thr Ala Ala Leu Gly Cys Leu Val Lys Asp Tyr Phe
   145 150 155 160
   ccc gaa ccg gtg acg gtg tcg tgg aac tca ggc gcc ctg acc agc ggc 528
   Pro Glu Pro Val Thr Val Ser Trp Asn Ser Gly Ala Leu Thr Ser Gly
   165 170 175
   gtc cac acc ttc ccg gct gtc cta cag tcc tca gga ctc tac tcc ctc 576
   Val His Thr Phe Pro Ala Val Leu Gln Ser Ser Gly Leu Tyr Ser Leu
   180 185 190
   agc agc gta gtg acc gtg ccc tcc agc agc ttg ggc acc cag acc tac 624
   Ser Ser Val Val Thr Val Pro Ser Ser Ser Leu Gly Thr Gln Thr Tyr
   195 200 205
   atc tgc aac gtg aat cac aag ccc agc aac acc aag gtg gac aag aaa 672
   Ile Cys Asn Val Asn His Lys Pro Ser Asn Thr Lys Val Asp Lys Lys
   210 215 220
   gtt gag ccc aaa tct tgt 690
   Val Glu Pro Lys Ser Cys
   225 230
<210> 52
   <211> 230
   <212> PRT
   <213> Homo sapiens
<400> 52
   Ala Gln Val Gln Leu Val Gln Ser Gly Ala Glu Val Arg Lys Pro Gly
   1               5                   10                  15
   Ala Ser Val Lys Val Ser Cys Lys Ala Ser Gly Tyr Asp Phe Ser Asn
               20                  25                  30
   Tyr Ala Ile His Trp Val Arg Gln Ala Pro Gly Gln Arg Leu Glu Trp
           35                  40                  45
   Met Gly Trp Ile Asn Ala Gly Asn Gly Asn Thr Lys Phe Ser Gln Lys
       50                  55                  60
   Phe Gln Gly Arg Ile Thr Val Thr Arg Asp Thr Ala Ala Ser Thr Ala
   65                  70                  75                  80
   Tyr Met Glu Leu Arg Ser Leu Arg Ser Glu Asp Thr Ala Val Tyr Tyr
                   85                  90                  95
   Cys Ala Arg Asp Ser Ser Asn Met Val Arg Gly Ile Ile Ile Ala Tyr
               100                 105                 110
   Tyr Phe Asp Tyr Trp Gly Gln Gly Thr Leu Val Thr Val Ser Ser Ala
           115                 120                 125
   Ser Thr Lys Gly Pro Ser Val Phe Pro Leu Ala Pro Ser Ser Lys Ser
       130                 135                 140
   Thr Ser Gly Gly Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Gln Thr Tyr
           195                 200                 205
   Ile Cys Asn Val Asn His Lys Pro Ser Asn Thr Lys Val Asp Lys Lys
       210                 215                 220
   Val Glu Pro Lys Ser Cys
   225                 230
<210> 53
   <211> 690
   <212> DNA
   <213> Homo sapiens
<220>
   <221> CDS
   <222> (1)..(690)
<400> 53
   gcc gag gtc cag ctg gtg cag tct ggg gct gag gtg agg aag cct ggg      48
   Ala Glu Val Gln Leu Val Gln Ser Gly Ala Glu Val Arg Lys Pro Gly
   1               5                   10                  15
   gcc tca gtg aag gtt tcc tgc aag gct tct gga tac gac ttc agt aat      96
   Ala Ser Val Lys Val Ser Cys Lys Ala Ser Gly Tyr Asp Phe Ser Asn
               20                  25                  30
   tat gct ata cat tgg gtg cgc cag gcc ccc gga caa agg ctt gag tgg      144
   Tyr Ala Ile His Trp Val Arg Gln Ala Pro Gly Gln Arg Leu Glu Trp
           35                  40                  45
   atg gga tgg atc aac gct ggc aat ggg aac aca aaa ttt tca cag aag      192
   Met Gly Trp Ile Asn Ala Gly Asn Gly Asn Thr Lys Phe Ser Gln Lys
       50                  55                  60
   ttc cag ggc aga atc acc gtt acc agg gac aca gcc gcg agc aca gcc      240
   Phe Gln Gly Arg Ile Thr Val Thr Arg Asp Thr Ala Ala Ser Thr Ala
   65                  70                  75                  80
   tac atg gag ctg cgc agt ctg aga tct gaa gac acg gct gtg tat tac      288
   Tyr Met Glu Leu Arg Ser Leu Arg Ser Glu Asp Thr Ala Val Tyr Tyr
                   85                  90                  95
   tgt gcg aga gat tcc tca aat atg gtt cgg gga att att ata gcg tac      336
   Cys Ala Arg Asp Ser Ser Asn Met Val Arg Gly Ile Ile Ile Ala Tyr
               100                 105                 110
   tat ttt gac tac tgg ggc cag ggc acc ctg gtc acc gtc tca agc gcc      384
   Tyr Phe Asp Tyr Trp Gly Gln Gly Thr Leu Val Thr Val Ser Ser Ala
           115                 120                 125
   tcc acc aag ggc cca tcg gtc ttc ccc ctg gca ccc tcc tcc aag agc      432
   Ser Thr Lys Gly Pro Ser Val Phe Pro Leu Ala Pro Ser Ser Lys Ser
       130                 135                 140
   acc tct ggg ggc aca gcg gcc ctg ggc tgc ctg gtc aag gac tac ttc      480
   Thr Ser Gly Gly Thr Ala Ala Leu Gly Cys Leu Val Lys Asp Tyr Phe
   145                 150                 155                 160
   ccc gaa ccg gtg acg gtg tcg tgg aac tca ggc gcc ctg acc agc ggc      528
   Pro Glu Pro Val Thr Val Ser Trp Asn Ser Gly Ala Leu Thr Ser Gly
                   165                 170                 175
   gtc cac acc ttc ccg gct gtc cta cag tcc tca gga ctc tac tcc ctc      576
   Val His Thr Phe Pro Ala Val Leu Gln Ser Ser Gly Leu Tyr Ser Leu
               180                 185                 190
   agc agc gta gtg acc gtg ccc tcc agc agc ttg ggc acc cag acc tac      624
   Ser Ser Val Val Thr Val Pro Ser Ser Ser Leu Gly Thr Gln Thr Tyr
           195                 200                 205
   atc tgc aac gtg aat cac aag ccc agc aac acc aag gtg gac aag aaa      672
   Ile Cys Asn Val Asn His Lys Pro Ser Asn Thr Lys Val Asp Lys Lys
       210                 215                 220
   gtt gag ccc aaa tct tgt                                              690
   Val Glu Pro Lys Ser Cys
   225                 230
<210> 54
   <211> 230
   <212> PRT
   <213> Homo sapiens
<400> 54
   Ala Glu Val Gln Leu Val Gln Ser Gly Ala Glu Val Arg Lys Pro Gly
   1               5                   10                  15
   Ala Ser Val Lys Val Ser Cys Lys Ala Ser Gly Tyr Asp Phe Ser Asn
               20                  25                  30
   Tyr Ala Ile His Trp Val Arg Gln Ala Pro Gly Gln Arg Leu Glu Trp
           35                  40                  45
   Met Gly Trp Ile Asn Ala Gly Asn Gly Asn Thr Lys Phe Ser Gln Lys
       50                  55                  60
   Phe Gln Gly Arg Ile Thr Val Thr Arg Asp Thr Ala Ala Ser Thr Ala
   65                  70                  75                  80
   Tyr Met Glu Leu Arg Ser Leu Arg Ser Glu Asp Thr Ala Val Tyr Tyr
                   85                  90                  95
   Cys Ala Arg Asp Ser Ser Asn Met Val Arg Gly Ile Ile Ile Ala Tyr
               100                 105                 110
   Tyr Phe Asp Tyr Trp Gly Gln Gly Thr Leu Val Thr Val Ser Ser Ala
           115                 120                 125
   Ser Thr Lys Gly Pro Ser Val Phe Pro Leu Ala Pro Ser Ser Lys Ser
       130                 135                 140
   Thr Ser Gly Gly Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Gln Thr Tyr
           195                 200                 205
   Ile Cys Asn Val Asn His Lys Pro Ser Asn Thr Lys Val Asp Lys Lys
       210                 215                 220
   Val Glu Pro Lys Ser Cys
   225                 230
<210> 55
   <211> 660
   <212> DNA
   <213> Homo sapiens
<220>
   <221> CDS
   <222> (1)..(660)
<400> 55
   gcc gag gtc cag ctg gtg cag tct ggg gga ggc ttg gtc cag cct ggg      48
   Ala Glu Val Gln Leu Val Gln Ser Gly Gly Gly Leu Val Gln Pro Gly
   1               5                   10                  15
   ggg tcc ctg aga ctc tcc tgt tta gtc tct gga ttc acc ttc aat aac      96
   Gly Ser Leu Arg Leu Ser Cys Leu Val Ser Gly Phe Thr Phe Asn Asn
               20                  25                  30
   tat cct atg cac tgg gtc cgc cag gct cca ggc aag ggg ctg gag tgg     144
   Tyr Pro Met His Trp Val Arg Gln Ala Pro Gly Lys Gly Leu Glu Trp
           35                  40                  45
   gtg gca gtt ata tca tat gat gga aat aat aaa tac tac gca gac tcc     192
   Val Ala Val Ile Ser Tyr Asp Gly Asn Asn Lys Tyr Tyr Ala Asp Ser
       50                  55                  60
   gtg aag ggc cga ttc acc atc tcc aga gac aat tcc aag aac acg ctg     240
   Val Lys Gly Arg Phe Thr Ile Ser Arg Asp Asn Ser Lys Asn Thr Leu
   65                  70                  75                  80
   tat ttg caa atg aac agc ctg aga tct gag gac acg gcc gtg tat tac     288
   Tyr Leu Gln Met Asn Ser Leu Arg Ser Glu Asp Thr Ala Val Tyr Tyr
                   85                  90                  95
   tgt gcg agg ggg ggc ggt ggc ttt gac tac tgg ggc cag gga acc ctg     336
   Cys Ala Arg Gly Gly Gly Gly Phe Asp Tyr Trp Gly Gln Gly Thr Leu
               100                 105                 110
   gtc acc gtc tca agc gcc tcc acc aag ggc cca tcg gtc ttc ccc ctg     384
   Val Thr Val Ser Ser Ala Ser Thr Lys Gly Pro Ser Val Phe Pro Leu
           115                 120                 125
   gca ccc tcc tcc aag agc acc tct ggg ggc aca gcg gcc ctg ggc tgc     432
   Ala Pro Ser Ser Lys Ser Thr Ser Gly Gly Thr Ala Ala Leu Gly Cys
       130                 135                 140
   ctg gtc aag gac tac ttc ccc gaa ccg gtg acg gtg tcg tgg aac tca     480
   Leu Val Lys Asp Tyr Phe Pro Glu Pro Val Thr Val Ser Trp Asn Ser
   145                 150                 155                 160
   ggc gcc ctg acc agc ggc gtc cac acc ttc ccg gct gtc cta cag tcc     528
   Gly Ala Leu Thr Ser Gly Val His Thr Phe Pro Ala Val Leu Gln Ser
                   165                 170                 175
   tca gga ctc tac tcc ctc agc agc gta gtg acc gtg ccc tcc agc agc     576
   Ser Gly Leu Tyr Ser Leu Ser Ser Val Val Thr Val Pro Ser Ser Ser
               180                 185                 190
   ttg ggc acc cag acc tac atc tgc aac gtg aat cac aag ccc agc aac     624
   Leu Gly Thr Gln Thr Tyr Ile Cys Asn Val Asn His Lys Pro Ser Asn
           195                 200                 205
   acc aag gtg gac aag aaa gtt gag ccc aaa tct tgt                     660
   Thr Lys Val Asp Lys Lys Val Glu Pro Lys Ser Cys
       210                 215                 220
<210> 56
   <211> 220
   <212> PRT
   <213> Homo sapiens
<400> 56
   Ala Glu Val Gln Leu Val Gln Ser Gly Gly Gly Leu Val Gln Pro Gly
   1               5                   10                  15
   Gly Ser Leu Arg Leu Ser Cys Leu Val Ser Gly Phe Thr Phe Asn Asn
   20 25 30
   Tyr Pro Met His Trp Val Arg Gln Ala Pro Gly Lys Gly Leu Glu Trp
   35 40 45
   Val Ala Val Ile Ser Tyr Asp Gly Asn Asn Lys Tyr Tyr Ala Asp Ser
   50 55 60
   Val Lys Gly Arg Phe Thr Ile Ser Arg Asp Asn Ser Lys Asn Thr Leu
   65 70 75 80
   Tyr Leu Gln Met Asn Ser Leu Arg Ser Glu Asp Thr Ala Val Tyr Tyr
   85 90 95
   Cys Ala Arg Gly Gly Gly Gly Phe Asp Tyr Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys
   210 215 220
<210> 57
   <211> 681
   <212> DNA
   <213> Homo sapiens
<220>
   <221> CDS
   <222> (1)..(681)
<400> 57
   gcc gag gtg cag ctg ctg gag tct ggg gga ggc ttg gta caa cct ggc 48
   Ala Glu Val Gln Leu Leu Glu Ser Gly Gly Gly Leu Val Gln Pro Gly
   1 5 10 15
   agg tcc ctg aga ctc tcc tgt gca gcc tct gga ttc acc ttt gat gat 96
   Arg Ser Leu Arg Leu Ser Cys Ala Ala Ser Gly Phe Thr Phe Asp Asp
   20 25 30
   tat gcc atg cac tgg gtc cgg caa gct cca ggg aag ggc ctg gag tgg 144
   Tyr Ala Met His Trp Val Arg Gln Ala Pro Gly Lys Gly Leu Glu Trp
   35 40 45
   gtc tca ggt att agt tgg aat agt ggt agg ata ggc tat gcg gac tct 192
   Val Ser Gly Ile Ser Trp Asn Ser Gly Arg Ile Gly Tyr Ala Asp Ser
       50                  55                  60
   gtg aag ggc cga ttc acc atc tcc aga gac aac gcc aag aac tcc ctg 240
   Val Lys Gly Arg Phe Thr Ile Ser Arg Asp Asn Ala Lys Asn Ser Leu
   65                  70                  75                  80
   tat ctg caa atg aac agt ctg aga cct gag gac acg gcc ttc tat tac 288
   Tyr Leu Gln Met Asn Ser Leu Arg Pro Glu Asp Thr Ala Phe Tyr Tyr
                   85                  90                  95
   tgt gca aaa ggg ggt tct aca agc gcg agg tat agc agt ggc tgg tac 336
   Cys Ala Lys Gly Gly Ser Thr Ser Ala Arg Tyr Ser Ser Gly Trp Tyr
               100                 105                 110
   tac tgg ggc cag ggc acc ctg gtc acc gtc tca agc gcc tcc acc aag 384
   Tyr Trp Gly Gln Gly Thr Leu Val Thr Val Ser Ser Ala Ser Thr Lys
           115                 120                 125
   ggc cca tcg gtc ttc ccc ctg gca ccc tcc tcc aag agc acc tct ggg 432
   Gly Pro Ser Val Phe Pro Leu Ala Pro Ser Ser Lys Ser Thr Ser Gly
       130                 135                 140
   ggc aca gcg gcc ctg ggc tgc ctg gtc aag gac tac ttc ccc gaa ccg 480
   Gly Thr Ala Ala Leu Gly Cys Leu Val Lys Asp Tyr Phe Pro Glu Pro
   145                 150                 155                 160
   gtg acg gtg tcg tgg aac tca ggc gcc ctg acc agc ggc gtc cac acc 528
   Val Thr Val Ser Trp Asn Ser Gly Ala Leu Thr Ser Gly Val His Thr
                   165                 170                 175
   ttc ccg gct gtc cta cag tcc tca gga ctc tac tcc ctc agc agc gta 576
   Phe Pro Ala Val Leu Gln Ser Ser Gly Leu Tyr Ser Leu Ser Ser Val
               180                 185                 190
   gtg acc gtg ccc tcc agc agc ttg ggc acc cag acc tac atc tgc aac 624
   Val Thr Val Pro Ser Ser Ser Leu Gly Thr Gln Thr Tyr Ile Cys Asn
           195                 200                 205
   gtg aat cac aag ccc agc aac acc aag gtg gac aag aaa gtt gag ccc 672
   Val Asn His Lys Pro Ser Asn Thr Lys Val Asp Lys Lys Val Glu Pro
       210                 215                 220
   aaa tct tgt                                                     681
   Lys Ser Cys
   225
<210> 58
   <211> 227
   <212> PRT
   <213> Homo sapiens
<400> 58
   Ala Glu Val Gln Leu Leu Glu Ser Gly Gly Gly Leu Val Gln Pro Gly
   1               5                   10                  15
   Arg Ser Leu Arg Leu Ser Cys Ala Ala Ser Gly Phe Thr Phe Asp Asp
               20                  25                  30
   Tyr Ala Met His Trp Val Arg Gln Ala Pro Gly Lys Gly Leu Glu Trp
           35                  40                  45
   Val Ser Gly Ile Ser Trp Asn Ser Gly Arg Ile Gly Tyr Ala Asp Ser
       50                  55                  60
   Val Lys Gly Arg Phe Thr Ile Ser Arg Asp Asn Ala Lys Asn Ser Leu
   65                  70                  75                  80
   Tyr Leu Gln Met Asn Ser Leu Arg Pro Glu Asp Thr Ala Phe Tyr Tyr
                   85                  90                  95
   Cys Ala Lys Gly Gly Ser Thr Ser Ala Arg Tyr Ser Ser Gly Trp Tyr
               100                 105                 110
   Tyr Trp Gly Gln Gly Thr Leu Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Lys Val Glu Pro
       210                 215                 220
   Lys Ser Cys
   225
<210> 59
   <211> 141
   <212> PRT
   <213> Homo sapiens
<400> 59
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ser Tyr
   20 25 30
   Ala Met His Trp Val Arg Gln Ala Pro Gly Gln Arg Leu Glu Trp Met
   35 40 45
   Gly Trp Ile Asn Ala Gly Asn Gly Asn Thr Lys Tyr Ser Gln Lys Phe
   50 55 60
   Gln Gly Arg Val Thr Ile Thr Arg Asp Thr Ser Ala Ser Thr Ala Tyr
   65 70 75 80
   Met Glu Leu Ser Ser Leu Arg Ser Glu Asp Thr Ala Val Tyr Tyr Cys
   85 90 95
   Ala Arg Val Leu Leu Trp Phe Gly Glu Leu Leu Tyr Tyr Tyr Gly Ser
   100 105 110
   Gly Ser Tyr Tyr Asn Ile Thr Met Val Arg Gly Val Ile Ile Tyr Phe
   115 120 125
   Asp Tyr Trp Gly Gln Gly Thr Leu Val Thr Val Ser Ser
   130 135 140
<210> 60
   <211> 126
   <212> PRT
   <213> Homo sapiens
<400> 60
   Gln Val Gln Leu Val Gln Ser Gly Ala Glu Val Arg Lys Pro Gly Ala
   1               5                   10                  15
   Ser Val Lys Val Ser Cys Lys Ala Ser Gly Tyr Asp Phe Ser Asn Tyr
               20                  25                  30
   Ala Ile His Trp Val Arg Gln Ala Pro Gly Gln Arg Leu Glu Trp Met
           35                  40                  45
   Gly Trp Ile Asn Ala Gly Asn Gly Asn Thr Lys Phe Ser Gln Lys Phe
       50                  55                  60
   Gln Gly Arg Ile Thr Val Thr Arg Asp Thr Ala Ala Ser Thr Ala Tyr
   65                  70                  75                  80
   Met Glu Leu Arg Ser Leu Arg Ser Glu Asp Thr Ala Val Tyr Tyr Cys
                   85                  90                  95
   Ala Arg Asp Ser Ser Asn Met Val Arg Gly Ile Ile Ile Ala Tyr Tyr
               100                 105                 110
   Phe Asp Tyr Trp Gly Gln Gly Thr Leu Val Thr Val Ser Ser
           115                 120                 125
<210> 61
   <211> 126
   <212> PRT
   <213> Homo sapiens
<400> 61
   Glu Val Gln Leu Val Gln Ser Gly Ala Glu Val Arg Lys Pro Gly Ala
   1               5                   10                  15
   Ser Val Lys Val Ser Cys Lys Ala Ser Gly Tyr Asp Phe Ser Asn Tyr
               20                  25                  30
   Ala Ile His Trp Val Arg Gln Ala Pro Gly Gln Arg Leu Glu Trp Met
           35                  40                  45
   Gly Trp Ile Asn Ala Gly Asn Gly Asn Thr Lys Phe Ser Gln Lys Phe
       50                  55                  60
   Gln Gly Arg Ile Thr Val Thr Arg Asp Thr Ala Ala Ser Thr Ala Tyr
   65                  70                  75                  80
   Met Glu Leu Arg Ser Leu Arg Ser Glu Asp Thr Ala Val Tyr Tyr Cys
                   85                  90                  95
   Ala Arg Asp Ser Ser Asn Met Val Arg Gly Ile Ile Ile Ala Tyr Tyr
               100                 105                 110
   Phe Asp Tyr Trp Gly Gln Gly Thr Leu Val Thr Val Ser Ser
           115                 120                 125
<210> 62
   <211> 113
   <212> PRT
   <213> Homo sapiens
<400> 62
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Tyr Phe Asp Tyr Trp Gly Gln Gly Thr Leu Val Thr Val Ser
   100 105 110
   Ser
<210> 63
   <211> 116
   <212> PRT
   <213> Homo sapiens
<400> 63
   Glu Val Gln Leu Val Gln Ser Gly Gly Gly Leu Val Gln Pro Gly Gly
   1 5 10 15
   Ser Leu Arg Leu Ser Cys Leu Val Ser Gly Phe Thr Phe Asn Asn Tyr
   20 25 30
   Pro Met His Trp Val Arg Gln Ala Pro Gly Lys Gly Leu Glu Trp Val
   35 40 45
   Ala Val Ile Ser Tyr Asp Gly Asn Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ser Glu Asp Thr Ala Val Tyr Tyr Cys
   85 90 95
   Ala Arg Gly Gly Gly Gly Phe Asp Tyr Trp Gly Gln Gly Thr Leu Val
   100 105 110
   Thr Val Ser Ser
   115
<210> 64
   <211> 132
   <212> PRT
   <213> Homo sapiens
<400> 64
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Gly Ile Ser Trp Asn Ser Gly Ser Ile Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr Tyr Cys
   85 90 95
   Ala Lys Asp Gly Tyr Ser Ser Gly Trp Tyr Gly Ile Ala Val Ala Gly
   100 105 110
   Val Gln Trp Leu Val Tyr Phe Asp Tyr Trp Gly Gln Gly Thr Leu Val
   115 120 125
   Thr Val Ser Ser
   130
<210> 65
   <211> 123
   <212> PRT
   <213> Homo sapiens
<400> 65
   Glu Val Gln Leu Leu Glu Ser Gly Gly Gly Leu Val Gln Pro Gly Arg
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
           35                  40                  45
   Ser Gly Ile Ser Trp Asn Ser Gly Arg Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Pro Glu Asp Thr Ala Phe Tyr Tyr Cys
                   85                  90                  95
   Ala Lys Gly Gly Ser Thr Ser Ala Arg Tyr Ser Ser Gly Trp Tyr Tyr
               100                 105                 110
   Trp Gly Gln Gly Thr Leu Val Thr Val Ser Ser
           115                 120
<210> 66
   <211> 107
   <212> PRT
   <213> Homo sapiens
<400> 66
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Trp
                   85                  90                  95
   Thr Phe Gly Gln Gly Thr Lys Val Glu Ile Lys
               100                 105
<210> 67
   <211> 103
   <212> PRT
   <213> Homo sapiens
<400> 67
   Glu Ile Val Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Arg Tyr
               20                  25                  30
   Leu Asn Trp Tyr Gln Leu Lys Pro Gly Lys Ala Pro Arg Leu Leu Ile
           35                  40                  45
   Tyr Gly Ala Ser Ser Leu Gln Ser Gly Val Pro Ser Arg Phe Ser Gly
       50                  55                  60
   Ser Gly Ser Gly Ala Glu Phe Thr Leu Thr Ile Ser Ser Leu Gln Pro
   65                  70                  75                  80
   Glu Asp Ile Ala Thr Tyr Tyr Cys Gln His Thr Arg Ala Phe Gly Gln
                   85                  90                  95
   Gly Thr Lys Val Glu Ile Lys
               100
<210> 68
   <211> 107
   <212> PRT
   <213> Homo sapiens
<400> 68
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Tyr
                   85                  90                  95
   Thr Phe Gly Gln Gly Thr Lys Leu Glu Ile Lys
               100                 105
<210> 69
   <211> 103
   <212> PRT
   <213> Homo sapiens
<400> 69
   Glu Ile Val Leu Thr Gln Ser Pro Ala Thr Leu Ser Phe Ser Pro Gly
   1 5 10 15
   Glu Arg Ala Thr Leu Ser Cys Arg Ala Ser Gln Ser Val Gly Ser Tyr
   20 25 30
   Leu Ala Trp Tyr Gln Gln Arg Pro Gly Gln Ala Pro Arg Pro Leu Ile
   35 40 45
   Tyr Asp Ala Thr Asn Arg Ala Thr Gly Ile Pro Thr Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Thr Tyr Tyr Cys Gln His Arg Arg Thr Phe Gly Arg
   85 90 95
   Gly Thr Lys Leu Glu Ile Lys
   100
<210> 70
   <211> 107
   <212> PRT
   <213> Homo sapiens
<400> 70
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp Pro Leu
   85 90 95
   Thr Phe Gly Gly Gly Thr Lys Val Glu Ile Lys
   100 105
<210> 71
   <211> 104
   <212> PRT
   <213> Homo sapiens
<400> 71
   Glu Ile Val Met Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ala Phe Gly
                   85                  90                  95
   Gly Gly Thr Lys Val Glu Ile Lys
               100
<210> 72
   <211> 108
   <212> PRT
   <213> Homo sapiens
<400> 72
   Ser Tyr Glu Leu Met Gln Pro Pro Ser Val Ser Val Ser Pro Gly Gln
   1               5                   10                  15
   Thr Ala Arg Ile Thr Cys Ser Gly Asp Ala Leu Pro Lys Gln Tyr Ala
               20                  25                  30
   Tyr Trp Tyr Gln Gln Lys Pro Gly Gln Ala Pro Val Leu Val Ile Tyr
           35                  40                  45
   Lys Asp Ser Glu Arg Pro Ser Gly Ile Pro Glu Arg Phe Ser Gly Ser
       50                  55                  60
   Ser Ser Gly Thr Thr Val Thr Leu Thr Ile Ser Gly Val Gln Ala Glu
   65                  70                  75                  80
   Asp Glu Ala Asp Tyr Tyr Cys Gln Ser Ala Asp Ser Ser Gly Thr Tyr
                   85                  90                  95
   Val Val Phe Gly Gly Gly Thr Lys Leu Thr Val Leu
               100                 105
<210> 73
   <211> 108
   <212> PRT
   <213> Homo sapiens
<400> 73
   Gln Ser Val Leu Thr Gln Pro Pro Ser Val Ser Val Ser Pro Gly Gln
   1               5                   10                  15
   Thr Ala Thr Ile Thr Cys Ser Gly Asp Ala Leu Pro Lys Gln Tyr Val
               20                  25                  30
   Tyr Trp Tyr Arg Gln Lys Pro Gly Gln Ala Pro Leu Leu Val Ile Tyr
           35                  40                  45
   Glu Asp Ser Glu Arg Pro Ser Gly Ile Pro Glu Arg Phe Ser Gly Ser
       50                  55                  60
   Ser Ser Gly Thr Glu Val Thr Leu Ser Ile Ser Gly Val Gln Ala Glu
   65                  70                  75                  80
   Asp Glu Ala Asp Tyr Tyr Cys Gln Ser Thr Asp Ser Ser Gly Thr Tyr
                   85                  90                  95
   Val Val Phe Gly Gly Gly Thr Lys Leu Thr Val Leu
               100                 105
<210> 74
   <211> 522
   <212> DNA
   <213> Homo sapiens
<220>
   <221> CDS
   <222> (4)..(513)
<400> 74
   cat atg gac tac aag gac gac gat gac aag aag ctt aag cct gag gcc      48
       Met Asp Tyr Lys Asp Asp Asp Asp Lys Lys Leu Lys Pro Glu Ala
       1               5                   10                  15
   cag cca ttt gca cac ctc acc atc aat gct gcc agc atc cca tcg ggt      96
   Gln Pro Phe Ala His Leu Thr Ile Asn Ala Ala Ser Ile Pro Ser Gly
                   20                  25                  30
   tcc cat aaa gtc act ctg tcc tct tgg tac cac gat cga ggc tgg gcc     144
   Ser His Lys Val Thr Leu Ser Ser Trp Tyr His Asp Arg Gly Trp Ala
               35                  40                  45
   aag atc tct aac atg acg tta agc aac gga aaa cta agg gtt aac caa     192
   Lys Ile Ser Asn Met Thr Leu Ser Asn Gly Lys Leu Arg Val Asn Gln
           50                  55                  60
   gat ggc ttc tat tac ctg tac gct aac att tgc ttt cgg cat cat gaa     240
   Asp Gly Phe Tyr Tyr Leu Tyr Ala Asn Ile Cys Phe Arg His His Glu
       65                  70                  75
   aca tcg gga gac ctg gct act gaa tat ctt cag ctg atg gtg tat gtc     288
   Thr Ser Gly Asp Leu Ala Thr Glu Tyr Leu Gln Leu Met Val Tyr Val
   80                  85                  90                  95
   gtt aaa acc agc atc aaa atc cca agt tct cat aac ctg atg aaa gga     336
   Val Lys Thr Ser Ile Lys Ile Pro Ser Ser His Asn Leu Met Lys Gly
                   100                 105                 110
   ggg agc acg aaa aac tgg tcg ggc aat tct gaa ttc cac ttt tat tcc     384
   Gly Ser Thr Lys Asn Trp Ser Gly Asn Ser Glu Phe His Phe Tyr Ser
               115                 120                 125
   ata aat gtt ggg gga ttt ttc aag ctc cga gct ggt gaa gaa att agc     432
   Ile Asn Val Gly Gly Phe Phe Lys Leu Arg Ala Gly Glu Glu Ile Ser
           130                 135                 140
   att cag gtg tcc aac cct tcc ctg ctg gat ccg gat caa gat gcg acg     480
   Ile Gln Val Ser Asn Pro Ser Leu Leu Asp Pro Asp Gln Asp Ala Thr
       145                 150                 155
   tac ttt ggg gct ttc aaa gtt cag gac ata gac taactcgag               522
   Tyr Phe Gly Ala Phe Lys Val Gln Asp Ile Asp
   160                 165                 170
<210> 75
   <211> 170
   <212> PRT
   <213> Homo sapiens
<400> 75
   Met Asp Tyr Lys Asp Asp Asp Asp Lys Lys Leu Lys Pro Glu Ala Gln
   1               5                   10                  15
   Pro Phe Ala His Leu Thr Ile Asn Ala Ala Ser Ile Pro Ser Gly Ser
               20                  25                  30
   His Lys Val Thr Leu Ser Ser Trp Tyr His Asp Arg Gly Trp Ala Lys
   35 40 45
   Ile Ser Asn Met Thr Leu Ser Asn Gly Lys Leu Arg Val Asn Gln Asp
   50 55 60
   Gly Phe Tyr Tyr Leu Tyr Ala Asn Ile Cys Phe Arg His His Glu Thr
   65 70 75 80
   Ser Gly Asp Leu Ala Thr Glu Tyr Leu Gln Leu Met Val Tyr Val Val
   85 90 95
   Lys Thr Ser Ile Lys Ile Pro Ser Ser His Asn Leu Met Lys Gly Gly
   100 105 110
   Ser Thr Lys Asn Trp Ser Gly Asn Ser Glu Phe His Phe Tyr Ser Ile
   115 120 125
   Asn Val Gly Gly Phe Phe Lys Leu Arg Ala Gly Glu Glu Ile Ser Ile
   130 135 140
   Gln Val Ser Asn Pro Ser Leu Leu Asp Pro Asp Gln Asp Ala Thr Tyr
   145 150 155 160
   Phe Gly Ala Phe Lys Val Gln Asp Ile Asp
   165 170
<210> 76
   <211> 39
   <212> PRT
   <213> Homo sapiens
<400> 76
   Gly Phe Tyr Tyr Leu Tyr Ala Asn Ile Cys Phe Arg His His Glu Thr
   1 5 10 15
   Ser Gly Asp Leu Ala Thr Glu Tyr Leu Gln Leu Met Val Tyr Val Thr
   20 25 30
   Lys Thr Ser Ile Lys Ile Pro
   35
<210> 77
   <211> 39
   <212> PRT
   <213> Mus musculus
<400> 77
   Gly Phe Tyr Tyr Leu Tyr Ala Asn Ile Cys Phe Arg His His Glu Thr
   1 5 10 15
   Ser Gly Ser Val Pro Thr Asp Tyr Leu Gln Leu Met Val Tyr Val Val
   20 25 30
   Lys Thr Ser Ile Lys Ile Pro
   35
<210> 78
   <211> 39
   <212> PRT
   <213> Mus musculus
<400> 78
   Gly Phe Tyr Tyr Leu Tyr Ala Asn Ile Cys Phe Arg His His Glu Thr
   1 5 10 15
   Ser Gly Asp Leu Ala Thr Glu Tyr Leu Gln Leu Met Val Tyr Val Val
   20 25 30
   Lys Thr Ser Ile Lys Ile Pro
   35
<210> 79
   <211> 5
   <212> PRT
   <213> Homo sapiens
<400> 79
   Asp Leu Ala Thr Glu
   1 5
<210> 80
   <211> 17
   <212> PRT
   <213> Homo sapiens
<220>
   <221> misc_feature
   <222> (1)..(1)
   <223> xaa is any amino acid
<400> 80
   Xaa Ser Ser Asn Met Val Arg Gly Ile Ile Ile Ala Tyr Tyr Phe Asp
   1 5 10 15
   Tyr
<210> 81
   <211> 17
   <212> PRT
   <213> Homo sapiens
<220>
   <221> misc_feature
   <222> (2)..(2)
   <223> xaa is any amino acid
<400> 81
   Asp Xaa Ser Asn Met Val Arg Gly Ile Ile Ile Ala Tyr Tyr Phe Asp
   1 5 10 15
   Tyr
<210> 82
   <211> 17
   <212> PRT
   <213> Homo sapiens
<220>
   <221> misc_feature
   <222> (3)..(3)
   <223> xaa is any amino acid
<400> 82
   Asp Ser Xaa Asn Met Val Arg Gly Ile Ile Ile Ala Tyr Tyr Phe Asp
   1 5 10 15
   Tyr
<210> 83
   <211> 17
   <212> PRT
   <213> Homo sapiens
<220>
   <221> misc_feature
   <222> (4)..(4)
   <223> xaa is any amino acid
<400> 83
   Asp Ser Ser Xaa Met Val Arg Gly Ile Ile Ile Ala Tyr Tyr Phe Asp
   1 5 10 15
   Tyr
<210> 84
   <211> 17
   <212> PRT
   <213> Homo sapiens
<220>
   <221> misc_feature
   <222> (5)..(5)
   <223> xaa is any amino acid
<400> 84
   Asp Ser Ser Asn Xaa Val Arg Gly Ile Ile Ile Ala Tyr Tyr Phe Asp
   1               5                   10                  15
   Tyr
<210> 85
   <211> 17
   <212> PRT
   <213> Homo sapiens
<220>
   <221> misc_feature
   <222> (6)..(6)
   <223> xaa is any amino acid
<400> 85
   Asp Ser Ser Asn Met Xaa Arg Gly Ile Ile Ile Ala Tyr Tyr Phe Asp
   1               5                   10                  15
   Tyr
<210> 86
   <211> 17
   <212> PRT
   <213> Homo sapiens
<220>
   <221> misc_feature
   <222> (7)..(7)
   <223> xaa is any amino acid
<400> 86
   Asp Ser Ser Asn Met Val Xaa Gly Ile Ile Ile Ala Tyr Tyr Phe Asp
   1               5                   10                  15
   Tyr
<210> 87
   <211> 17
   <212> PRT
   <213> Homo sapiens
<220>
   <221> misc_feature
   <222> (8)..(8)
   <223> xaa is any amino acid
<400> 87
   Asp Ser Ser Asn Met Val Arg Xaa Ile Ile Ile Ala Tyr Tyr Phe Asp
   1 5 10 15
   Tyr
<210> 88
   <211> 17
   <212> PRT
   <213> Homo sapiens
<220>
   <221> misc_feature
   <222> (9)..(9)
   <223> xaa is any amino acid
<400> 88
   Asp Ser Ser Asn Met Val Arg Gly Xaa Ile Ile Ala Tyr Tyr Phe Asp
   1 5 10 15
   Tyr
<210> 89
   <211> 17
   <212> PRT
   <213> Homo sapiens
<220>
   <221> misc_feature
   <222> (10)..(10)
   <223> xaa is any amino acid
<400> 89
   Asp Ser Ser Asn Met Val Arg Gly Ile Xaa Ile Ala Tyr Tyr Phe Asp
   1 5 10 15
   Tyr
<210> 90
   <211> 17
   <212> PRT
   <213> Homo sapiens
<220>
   <221> misc_feature
   <222> (11)..(11)
   <223> xaa is any amino acid
<400> 90
   Asp Ser Ser Asn Met Val Arg Gly Ile Ile Xaa Ala Tyr Tyr Phe Asp
   1               5                   10                  15
   Tyr
<210> 91
   <211> 17
   <212> PRT
   <213> Homo sapiens
<220>
   <221> misc_feature
   <222> (12)..(12)
   <223> xaa is any amino acid
<400> 91
   Asp Ser Ser Asn Met Val Arg Gly Ile Ile Ile Xaa Tyr Tyr Phe Asp
   1               5                   10                  15
   Tyr
<210> 92
   <211> 17
   <212> PRT
   <213> Homo sapiens
<220>
   <221> misc_feature
   <222> (13)..(13)
   <223> xaa is any amino acid
<400> 92
   Asp Ser Ser Asn Met Val Arg Gly Ile Ile Ile Ala Xaa Tyr Phe Asp
   1               5                   10                  15
   Tyr
<210> 93
   <211> 17
   <212> PRT
   <213> Homo sapiens
<220>
   <221> misc_feature
   <222> (14)..(14)
   <223> xaa is any amino acid
<400> 93
   Asp Ser Ser Asn Met Val Arg Gly Ile Ile Ile Ala Tyr Xaa Phe Asp
   1 5 10 15
   Tyr
<210> 94
   <211> 17
   <212> PRT
   <213> Homo sapiens
<220>
   <221> misc_feature
   <222> (15)..(15)
   <223> xaa is any amino acid
<400> 94
   Asp Ser Ser Asn Met Val Arg Gly Ile Ile Ile Ala Tyr Tyr Xaa Asp
   1 5 10 15
   Tyr
<210> 95
   <211> 17
   <212> PRT
   <213> Homo sapiens
<220>
   <221> misc_feature
   <222> (16)..(16)
   <223> xaa is any amino acid
<400> 95
   Asp Ser Ser Asn Met Val Arg Gly Ile Ile Ile Ala Tyr Tyr Phe Xaa
   1 5 10 15
   Tyr
<210> 96
   <211> 17
   <212> PRT
   <213> Homo sapiens
<220>
   <221> misc_feature
   <222> (17)..(17)
   <223> xaa is any amino acid
<400> 96
   Asp Ser Ser Asn Met Val Arg Gly Ile Ile Ile Ala Tyr Tyr Phe Asp
   1               5                   10                  15
   Xaa
<210> 97
   <211> 9
   <212> PRT
   <213> Homo sapiens
<220>
   <221> misc_feature
   <222> (4)..(4)
   <223> xaa in position 4 is any amino acid other than arginine.
<220>
   <221> misc_feature
   <222> (5)..(7)
   <223> xaa in positions 5 - 7 are any amino acid.
<400> 97
   Gln His Thr Xaa Xaa Xaa Xaa Arg Ala
   1               5
<210> 98
   <211> 9
   <212> PRT
   <213> Homo sapiens
<220>
   <221> misc_feature
   <222> (4)..(4)
   <223> xaa is any amino acid other than arginine.
<400> 98
   Gln His Thr Xaa Ala Ala Ala Arg Ala
   1               5
<210> 99
   <211> 10
   <212> PRT
   <213> Homo sapiens
<400> 99
   Asp Tyr Lys Asp Asp Asp Asp Lys Lys Leu
   1               5                   10
<210> 100
   <211> 23
   <212> DNA
   <213> Homo sapiens
<400> 100
   cctctcatat ggactacaag gac 23
<210> 101
   <211> 30
   <212> DNA
   <213> Homo sapiens
<400> 101
   agtagccagg tctcccgatg tttcatgatg 30
<210> 102
   <211> 30
   <212> DNA
   <213> Homo sapiens
<400> 102
   ctggctactg aatatcttca gctgatggtg 30
<210> 103
   <211> 25
   <212> DNA
   <213> Homo sapiens
<400> 103
   cctctcctcg agttagtcta tgtcc 25
<210> 104
   <211> 17
   <212> PRT
   <213> Homo sapiens
<400> 104
   Asp Ser Ser Asn Met Val Arg Gly Ile Ile Ile Ala Tyr Tyr Phe Asp
   1               5                   10                  15
   Tyr
<210> 105
   <211> 39
   <212> DNA
   <213> Homo sapiens
<400> 105
   agagattcct caaatatggt tcggggaatt attatagcg 39
<210> 106
   <211> 36
   <212> DNA
   <213> Homo sapiens
<400> 106
   gtagtcaaaa tagtacgcta taataattcc ccgaac 36
<210> 107
   <211> 33
   <212> DNA
   <213> Homo sapiens
<400> 107
   gtgtattact gtgcgagaga ttcctcaaat atg 33
<210> 108
   <211> 36
   <212> DNA
   <213> Homo sapiens
<400> 108
   cagggtgccc tggccccagt agtcaaaata gtacgc 36
<210> 109
   <211> 39
   <212> DNA
   <213> Homo sapiens
<400> 109
   gtgtattact gtgcgagagc ttcctcaaat atggttcgg 39
<210> 110
   <211> 39
   <212> DNA
   <213> Homo sapiens
<400> 110
   gtgtattact gtgcgagaga tgcctcaaat atggttcgg 39
<210> 111
   <211> 42
   <212> DNA
   <213> Homo sapiens
<400> 111
   aataattccc cgaaccatat ttgcggaatc tctcgcacag ta 42
<210> 112
   <211> 39
   <212> DNA
   <213> Homo sapiens
<400> 112
   aataattccc cgaaccatag ctgaggaatc tctcgcaca 39
<210> 113
   <211> 36
   <212> DNA
   <213> Homo sapiens
<400> 113
   aataattccc cgaacggcat ttgaggaatc tctcgc 36
<210> 114
   <211> 36
   <212> DNA
   <213> Homo sapiens
<400> 114
   gattcctcaa atatggctcg gggaattatt atagcg 36
<210> 115
   <211> 38
   <212> DNA
   <213> Homo sapiens
<400> 115
   gattcctcaa atatggttgc cggaattatt atagcgta 38
<210> 116
   <211> 45
   <212> DNA
   <213> Homo sapiens
<400> 116
   gtagtcaaaa tagtacgcta taataattgc ccgaaccata tttga 45
<210> 117
   <211> 42
   <212> DNA
   <213> Homo sapiens
<400> 117
   gtagtcaaaa tagtacgcta taatggctcc ccgaaccata tt 42
<210> 118
   <211> 39
   <212> DNA
   <213> Homo sapiens
<400> 118
   gtagtcaaaa tagtacgcta tggcaattcc ccgaaccat 39
<210> 119
   <211> 36
   <212> DNA
   <213> Homo sapiens
<400> 119
   gtagtcaaaa tagtacgctg caataattcc ccgaac 36
<210> 120
   <211> 45
   <212> DNA
   <213> Homo sapiens
<400> 120
   ggtgccctgg ccccagtagt caaaataggc cgctataata attcc 45
<210> 121
   <211> 45
   <212> DNA
   <213> Homo sapiens
<400> 121
   ggtgccctgg ccccagtagt caaaagcgta cgctataata attcc 45
<210> 122
   <211> 42
   <212> DNA
   <213> Homo sapiens
<400> 122
   cagggtgccc tggccccagt agtcagcata gtacgctata at 42
<210> 123
   <211> 39
   <212> DNA
   <213> Homo sapiens
<400> 123
   cagggtgccc tggccccagt aggcaaaata gtacgctat 39
<210> 124
   <211> 39
   <212> DNA
   <213> Homo sapiens
<400> 124
   cagggtgccc tggccccagg cgtcaaaata gtacgctat 39
<210> 125
   <211> 42
   <212> DNA
   <213> Homo sapiens
<400> 125
   agtctgagat ctgaagacac ggctgtgtat tactgtgcga ga 42
<210> 126
   <211> 33
   <212> DNA
   <213> Homo sapiens
<400> 126
   gtgtattact gtgcgagaga ttcctcaaat atg 33
<210> 127
   <211> 39
   <212> DNA
   <213> Homo sapiens
<400> 127
   agagattcct caaatatggt tcggggaatt attatagcg 39
<210> 128
   <211> 32
   <212> DNA
   <213> Homo sapiens
<400> 128
   cttgagacgg tgaccagggt gccctggccc ca 32
<210> 129
   <211> 36
   <212> DNA
   <213> Homo sapiens
<400> 129
   cagggtgccc tggccccagt agtcaaaata gtacgc 36
<210> 130
   <211> 36
   <212> DNA
   <213> Homo sapiens
<400> 130
   gtagtcaaaa tagtacgcta taataattcc ccgaac 36
<210> 131
   <211> 46
   <212> DNA
   <213> Homo sapiens
<400> 131
   aataattccc cgaaccatat ttgagatacg tatctctcgc acagta 46
<210> 132
   <211> 46
   <212> DNA
   <213> Homo sapiens
<400> 132
   aataattccc cgaaccatat ttgagcgacg tatctctcgc acagta 46
<210> 133
   <211> 44
   <212> DNA
   <213> Homo sapiens
<400> 133
   aataattccc cgaaccatat ttgactcgta tctctcgcac agta 44
<210> 134
   <211> 44
   <212> DNA
   <213> Homo sapiens
<400> 134
   aataattccc cgaaccatat ttgacacata tctctcgcac agta 44
<210> 135
   <211> 46
   <212> DNA
   <213> Homo sapiens
<400> 135
   aataattccc cgaaccatat tgatacgtgg aatctctcgc acagta 46
<210> 136
   <211> 46
   <212> DNA
   <213> Homo sapiens
<400> 136
   aataattccc cgaaccatat tgcgacgtgg aatctctcgc acagta 46
<210> 137
   <211> 44
   <212> DNA
   <213> Homo sapiens
<400> 137
   aataattccc cgaaccatat tctcgtggaa tctctcgcac agta 44
<210> 138
   <211> 44
   <212> DNA
   <213> Homo sapiens
<400> 138
   aataattccc cgaaccatat tcacatggaa tctctcgcac agta 44
<210> 139
   <211> 43
   <212> DNA
   <213> Homo sapiens
<400> 139
   aataattccc cgaaccatga tacgttgagg aatctctcgc aca 43
<210> 140
   <211> 43
   <212> DNA
   <213> Homo sapiens
<400> 140
   aataattccc cgaaccatgc gacgttgagg aatctctcgc aca 43
<210> 141
   <211> 41
   <212> DNA
   <213> Homo sapiens
<400> 141
   aataattccc cgaaccatct cgttgaggaa tctctcgcac a 41
<210> 142
   <211> 41
   <212> DNA
   <213> Homo sapiens
<400> 142
   aataattccc cgaaccatca cattgaggaa tctctcgcac a 41
<210> 143
   <21 > 42
   <212> DNA
   <213> Homo sapiens
<400> 143
   agtctgagat ctgaagacac ggctgtgtat tactgtgcga ga 42
<210> 144
   <211> 52
   <212> DNA
   <213> Homo sapiens
<400> 144
   cagcagaagc ttagaccacc atggacatga gggtccccgc ttagctcctg gg 52
<210> 145
   <211> 36
   <212> DNA
   <213> Homo sapiens
<400> 145
   cacagccgtg tcttcagatc tcagactgcg cagctc 36
<210> 146
   <211> 30
   <212> DNA
   <213> Homo sapiens
<400> 146
   gtggaggcac tagagacggt gaccagggtg 30
<210> 147
   <211> 48
   <212> DNA
   <213> Homo sapiens
<400> 147
   gtgtattact gtgcgagaga tgccgcaaat atggttcggg gaattatt 48
<210> 148
   <211> 53
   <212> DNA
   <213> Homo sapiens
<400> 148
   gtgtattact gtgcgagaga tgcctcagct atggttcggg gaattattat agc 53
<210> 149
   <211> 53
   <212> DNA
   <213> Homo sapiens
<400> 149
   gtgtattact gtgcgagaga ttccgcagct atggttcggg gaattattat agc 53
<210> 150
   <211> 53
   <212> DNA
   <213> Homo sapiens
<400> 150
   gtgtattact gtgcgagaga tgccgcagct atggttcggg gaattattat agc 53
<210> 151
   <211> 40
   <212> DNA
   <213> Homo sapiens
<400> 151
   gtggttgaga ggtgccagat gtcaggtcca gctggtgcag 40
<210> 152
   <211> 48
   <212> DNA
   <213> Homo sapiens
<400> 152
   ccgctcagct cctggggctc ctgctattgt ggttgagagg tgccagat 48
<210> 153
   <211> 51
   <212> DNA
   <213> Homo sapiens
<400> 153
   ccggtcaaca cactacgtac gtgtgcggcg gcgcgggcgt tcggccaagg g 51
<210> 154
   <211> 23
   <212> DNA
   <213> Homo sapiens
<400> 154
   ccgggcgcgc cttattaaca ctc 23
<210> 155
   <211> 9
   <212> PRT
   <213> Homo sapiens
<220>
   <221> misc_feature
   <222> (4)..(4)
   <223> xaa is any amino acid
<400> 155
   Gly His Thr Xaa Ala Ala Ala Arg Ala
   1 5

## Description

### Field of the Invention

The invention relates to monoclonal antibodies and antigen binding domains which bind selectively to OPGbp and may be used to prevent or treat conditions relating to loss of bone mass.

### Background of the Invention

Living bone tissue exhibits a dynamic equilibrium between deposition and resorption of bone. These processes are mediated primarily by two cell types: osteoblasts, which secrete molecules that comprise the organic matrix of bone; and osteoclasts, which promote dissolution of the bone matrix and solubilization of bone salts. In young individuals with growing bone, the rate of bone deposition exceeds the rate of bone resorption, while in older individuals the rate of resorption can exceed deposition. In the latter situation, the increased breakdown of bone leads to reduced bone mass and strength, increased risk of fractures, and slow or incomplete repair of broken bones.

Osteoclasts are large phagocytic multinucleated cells which are formed from hematopoietic precursor cells in the bone marrow. Although the growth and formation of mature functional osteoclasts is not well understood, it is thought that osteoclasts mature along the monocyte/macrophage cell lineage in response to exposure to various growth-promoting factors. Early development of bone marrow precursor cells to preosteoclasts are believed to mediated by soluble factors such as tumor necrosis factor-α (TNF-α), tumor necrosis factor-β (TNF-β), interleukin-1 (IL-1), interleukin-4 (IL-4), interleukin-6 (IL-6), and leukemia inhibitory factor (LIF). In culture, preosteoclasts are formed in the presence of added macrophage colony stimulating factor (M-CSF). These factors act primarily in early steps of osteoclast development.

A polypeptide factor, osteoprotegerin binding protein (OPGbp), has been described which stimulates osteoclast formation and bone resorption and appears to act at a late stage of development. See PCT WO98/46751. OPGbp stimulates osteoclast formation from bone marrow precursor cells without the requirement for coculturing in the presence of a stromal cell line. Stimulation of bone resorption by OPGbp required interaction with its cognate receptor, osteoprotegerin differentiation and activation receptor (ODAR) and inhibition of the ODAR/OPGbp interaction by osteoprotegerin (OPG) also inhibited bone resorption. Consequently, the regulation of OPGbp binding to ODAR affects osteoclast formation and loss of bone.

It is an object of the invention to identify selective binding agents which regulate the interaction of OPGbp and ODAR, especially those agents which block the interaction of OPGbp and ODAR and/or inhibit at least one activity of OPGbp, such as bone resorption. It is a further object of the invention to identify those selective binding agents that may be used to prevent and treat loss of bone mass. It is a further object of the invention to identify an antibody, or an antigen binding domain, or a fragment or variant thereof, which regulates the interaction of OPGbp and ODAR and neutralizes at least one activity of OPGbp. The antibodies may be used to prevent and treat loss of bone mass.

Tsukii et al., Biochemical and Biophysical Research Communications, 2646, 1998, 337-341 discloses rabbit anti- ODF polyclonal antibodies and activities thereof.

### Summary of the Invention

The invention provides
1. A monoclonal antibody or antigen binding domain thereof which binds to a portion of the amino acid sequence:
   GGFYYLYANICFRHHETSGDLATEYLQLMVYVTKTSIKIP (SEQ ID NO: 76) from amino acid residue 212 to amino acid residue 250 of human OPGbp (osteoprotegerin binding protein), wherein the antibody or antigen binding domain thereof inhibits the formation or activation of osteoclasts mediated by human OPGbp.
2. A monoclonal antibody or antigen binding domain thereof of Embodiment 1 which binds to the amino acid sequence DLATE within SEQ ID NO: 76.
3. The monoclonal antibody or antigen binding domain thereof of Embodiments 1 or 2 which is a recombinant antibody or antigen binding domain.
4. The monoclonal antibody or antigen binding domain thereof of Embodiments 1 or 2 which is a chimeric antibody or antigen binding domain.
5. The monoclonal antibody or antigen binding domain thereof of Embodiments 1 or 2 which is a humanized antibody or antigen binding domain.
6. The monoclonal antibody or antigen binding domain thereof of Embodiments 1 or 2 which is a fully human antibody or antigen binding domain.
7. The monoclonal antibody or antigen binding domain thereof of Embodiments 1 or 2 which is a single chain antibody or antigen binding domain.
8. The monoclonal antibody or antigen binding domain thereof of Embodiments 1 or 2 which is a bispecific antibody or antigen binding domain.
9. The monoclonal antibody or antigen binding domain thereof of Embodiments 1 or 2 which is a fragment thereof.
10. The monoclonal antibody or antigen binding domain thereof of Embodiment 9 wherein the fragment is selected from Fab, Fab', F(ab')2, Fv and scFv.
11. A method of producing the monoclonal antibody or antigen binding domain thereof of Embodiment 1 or 2 comprising culturing a host cell expressing the antibody or antigen binding domain and recovering the antibody or antigen binding domain from the culture medium or from the host cell.
12. The method of Embodiment 11 wherein the host cell is a eukaryotic cell.
13. The method of Embodiment 11 wherein the host cell is a prokaryotic cell.
14. A composition comprising the monoclonal antibody or antigen-binding domain thereof of Embodiments 1 or 2 and a pharmaceutically acceptable carrier.
15. Use of a monoclonal antibody or an antigen-binding domain thereof of Embodiments 1 or 2 for the preparation of a pharmaceutical composition for inhibiting osteoclast formation or activation.
16. Use of a monoclonal antibody or an antigen-binding domain thereof of Embodiments 1 or 2 for the preparation of a pharmaceutical composition for inhibiting bone resorption.
17. Use of a monoclonal antibody or an antigen-binding domain thereof of Embodiments 1 or 2 for the preparation of a pharmaceutical composition for preventing or treating loss of bone mass.
18. The use of Embodiment 17, wherein loss of bone mass results from osteoporosis, metastasis of cancer to bone; rheumatoid arthritis, hypercalcemia of malignancy, and steroid-induced osteoporosis.
19. The use of any of Embodiments 15 to 18, wherein a composition comprising at least one bone anti-resorptive agent is further to be administered.
20. The use of Embodiment 19, wherein the anti-resorptive agent is estrogen, a bisphosphonate, or a selective estrogen receptor modulator.
21. The use of any of Embodiments 15 to 18, wherein a composition comprising an anabolic agent for bone is further to be administered.
22. The use of Embodiment 21, wherein the anabolic agent is parathyroid hormone or a complex of insulin-like growth factor and insulin-like growth factor binding protein.
23. The use of any of Embodiments 15 to 18, wherein an interleukin-1 inhibitor or a tumor necrosis factor-alpha inhibitor is further to be administered.

An antibody may be prepared by immunizing an animal with OPGbp such as murine or human OPGbp, preferably human OPGbp, or with an immunogenic fragment, derivative or variant thereof. In addition, an animal may be immunized with cells transfected with a vector containing a nucleic acid molecule encoding OPGbp such that OPGbp is expressed and associated with the surface of the transfected cells. Alternatively, selective binding agents which are antibodies may be obtained by screening a library comprising antibody or antigen binding domain sequences for binding to OPGbp. Such a library is conveniently prepared in bacteriophage as protein or peptide fusions to a bacteriophage coat protein which are expressed on the surface of assembled phage particles and the encoding DNA sequences contained within the phage particles (so-called "phage displayed library"). In one example, a phage displayed library contains DNA sequences encoding human antibodies, such as variable light and heavy chains.

Selective binding agents which are antibodies or antigen binding domains may be tetrameric glycoproteins similar to native antibodies, or they may be single chain antibodies; Fv, Fab, Fab' or F(ab)' fragments, bispecific antibodies, heteroantibodies, or other fragments, variants, or derivatives thereof, which are capable of binding OPGbp and partially or completely neutralize OPGbp activity. Antibodies or antigen binding domains may be produced in hybridoma cell lines (antibody-producing cells such as spleen cells fused to mouse myeloma cells, for example) or may be produced in heterologous cell lines transfected with nucleic acid molecules encoding said antibody or antigen binding domain.

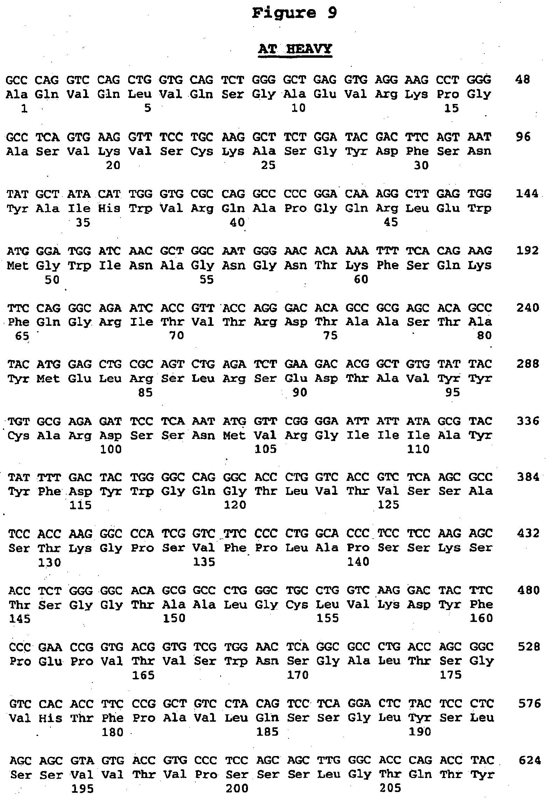

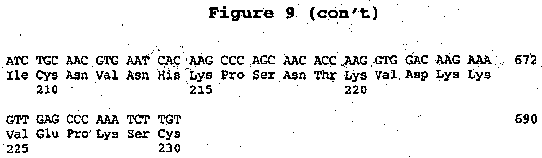

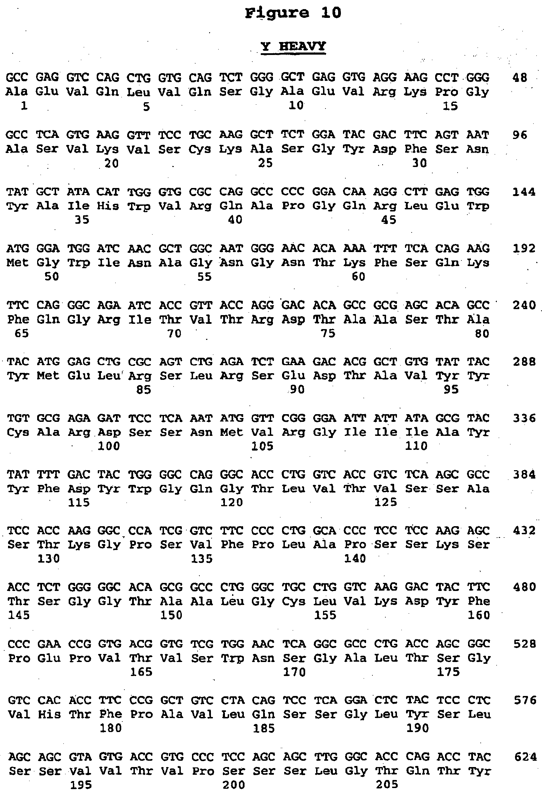

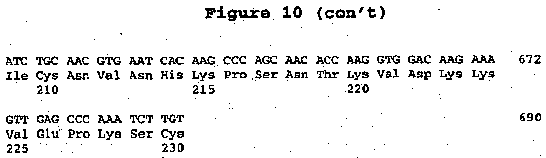

An antibody or antigen binding domain of the invention may comprises:
(a) a Fab heavy chain amino acid sequence as shown in Figure 9 (SEQ ID NO: 51)or Figure 10 (SEQ ID NO: 53);
(b) a heavy chain amino acid sequence comprising conservative amino acid substitutions of the sequence in (a);
(c) a heavy chain amino acid sequence which is at least about 80% identical to the sequence in (a); or
(d) a fragment or derivative of (a), (b) or (c) ;
wherein the antibody or antigen binding domain binds selectively to OPGbp.

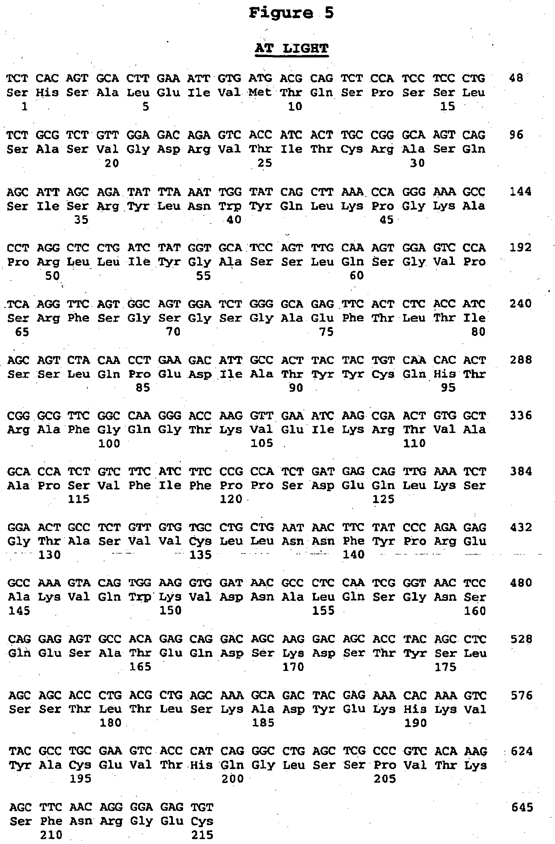

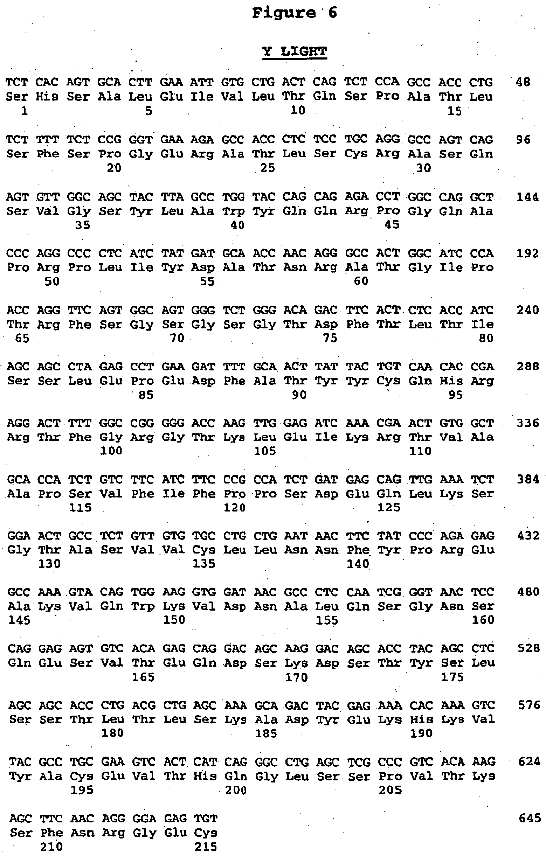

In another embodiment, an antibody or antigen binding domain as defined in claim 1 recognizes an epitope on human OPGbp recognized by an antibody or antigen binding domain comprising a Fab heavy chain amino acid sequence as shown in Figure 9 (SEQ ID NO: 51) or Figure 10 (SEQ ID NO: 53) and a Fab light amino acid sequence as shown in Figure 5 (SEQ ID NO: 43) or Figure 6 (SEQ ID NO: 45). Also provided for is an anti-OPGbp antibody or antigen binding domain which recognizes a DE epitope on OPGbp.

In another embodiment, an antibody or antigen binding domain as defined in claim 1 comprises a V₁ and Vₕ chain:
wherein each V₁ chain comprises CDR amino acid sequences designated CDR1(V₁), CDR2 (V₁) and CDR3(V₁) separated by framework amino acid sequences, CDR1(V₁) being selected from the group consisting of:
   RASQSISRYLN (SEQ ID NO: 01);
   RASQSVGSYLA (SEQ ID NO: 02);
   RASQSVSSSSLA(SEQ ID NO: 03); and
   SGDALPKQY (SEQ ID NO: 04);
   CDR2(V₁) being selected from the group consisting of:
   GASSLQS (SEQ ID NO: 05);
   DATNRAT (SEQ ID NO: 06);
   GASSRAT (SEQ ID NO: 07); and
   EDSERPS (SEQ ID NO: 08);
   and CDR3(V₁) being selected from the group consisting of:
   QHTRA (SEQ ID NO: 09);
   QHRRT (SEQ ID NO: 10);
   QQYGA (SEQ ID NO: 11); and
   QSTDSSGTYW (SEQ ID NO: 12);
   wherein CDR1(V₁), CDR2(V₁) and CDR3(V₁) are selected independently of each other; and
wherein each Vₕ chain comprises CDR amino acid sequences designated CDR1(Vₕ), CDR2(Vₕ) and CDR3(Vₕ) separated by framework amino acid sequences, CDR1(Vₕ) being selected from the group consisting of:
   NYAIH (SEQ ID NO: 13);
   NYPMH (SEQ ID NO: 14); and
   DYAMH (SEQ ID NO: 15)
   CDR2(Vₕ) being selected from the group consisting of:
   WINAGNGNTKFSQKFQG (SEQ ID NO: 16);
   VISYDGNNICYYADSVKG (SEQ ID NO: 17); and
   GISWNSGRIGYADSVKG (SEQ ID NO: 18)
   CDR3(Vₕ) being selected from the group consisting of:
   DSSNMVRGIIIAYYFDY (SEQ ID NO: 19);
   GGGGFDY (SEQ ID NO: 20); and
   GGSTSARYSSGWYY (SEQ ID NO: 21)
   wherein CDR1 (Vₕ), CDR2 (Vₕ) and CDR3 (Vₕ) are selected independently of each other.

In another embodiment, an antibody or antigen binding domain as defined in claim 1 comprises a V₁ and a Vₕ chain wherein:
the V₁ chain comprises CDR1 having the sequence RASQSISRYLN (SEQ ID NO: 01), CDR2 having the sequence GASSLQS (SEQ ID NO: 05), and CDR3 having the sequence QHTRA (SEQ ID NO: 09); and
the Vₕ chain comprises CDR1 having the sequence NYAIH (SEQ ID NO: 13), CDR2 having the sequence WINAGNGNTKFSQKFQG (SEQ ID NO: 16), and CDR3 having the sequence DSSNMVRGIIIAYYFDY (SEQ ID NO: 19);
wherein CDR1, CDR2 and CDR3 on each V₁ and Vₕ chain are separated by framework amino acid sequences.

Antibodies and antigen binding domains of the invention are derived from germ line nucleic acid sequences present in genomic DNA which encode light and heavy chain amino acid sequences. Antibodies are encoded by nucleic acid sequences which are the products of germline sequence rearrangement and somatic mutation.

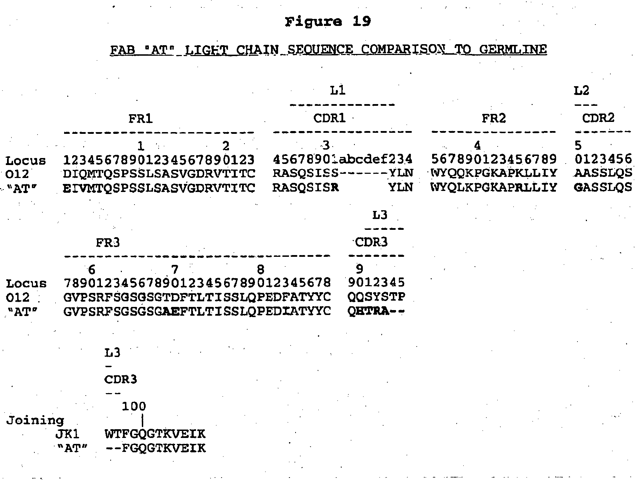

In one embodiment, an antibody or antigen binding domain of the invention comprises a V₁ and a Vₕ chain wherein the V₁ chain is comprises a rearranged or somatic variant of a Vh1 germline genes such as in Figure 19 (SEQ ID NO: 66); and the Vₕ chain comprises a rearranged or somatic variant of a Vh1 germline genes such as in Figure-16 (SEQ ID NO: 59); and the antibody binds selectively to an OPGbp polypeptide.

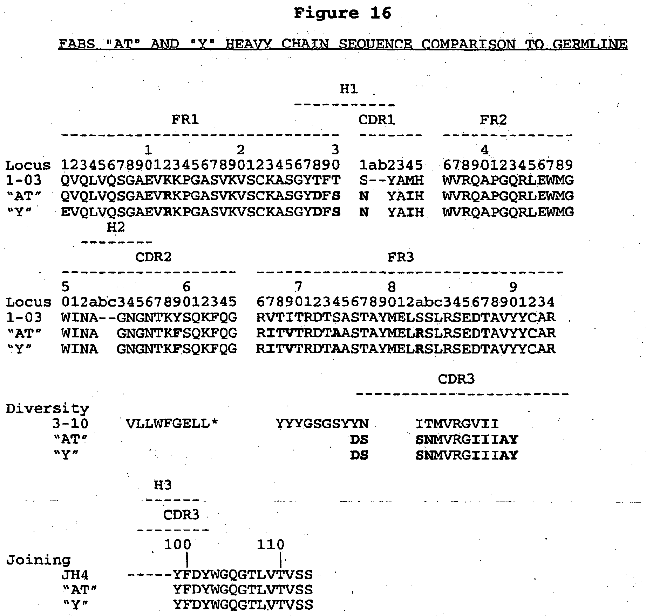

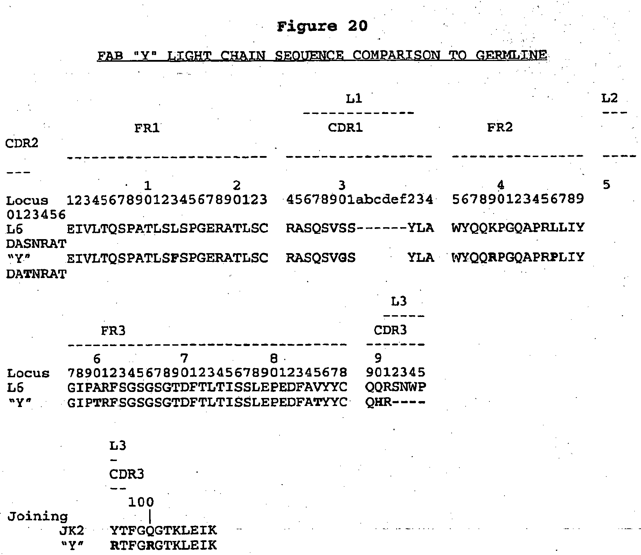

In another embodiment, the V₁ chain comprises or a rearranged or somatic variant of a Vk3 germline genes such as in Figure 20 (SEQ ID NO: 68); and the Vₕ chain comprises a rearranged or somatic variant of a Vh1 germline gene such as in Figure 16 (SEQ ID NO: 59).

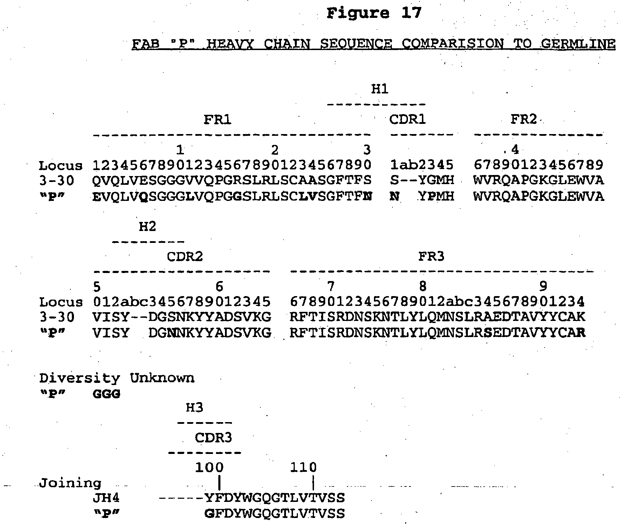

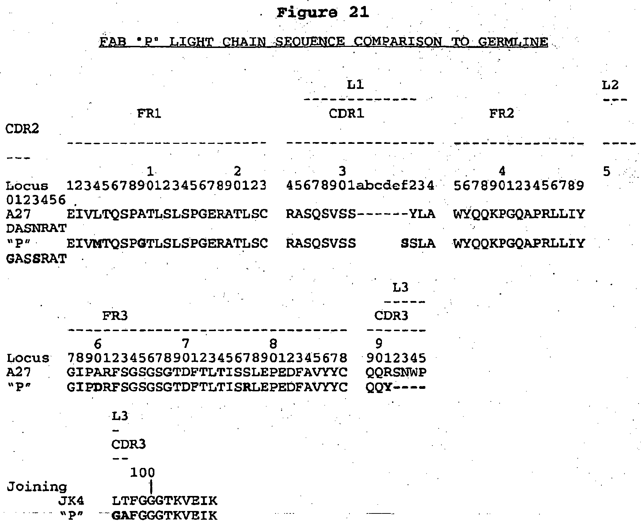

In another embodiment, the V₁ chain comprises a rearranged or somatic variant of a Vk3 germline gene such as in Figure 21 (SEQ ID NO: 70) ; and the Vₕ chain comprises a rearranged or somatic variant of a Vh3 germline gene such as in Figure 17 (SEQ ID NO: 62).

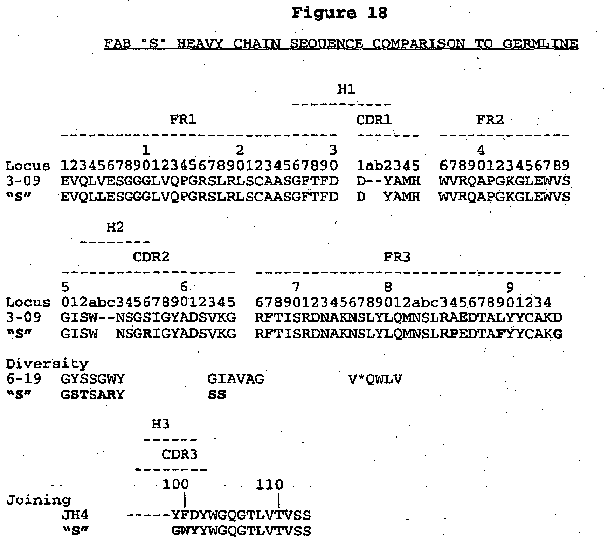

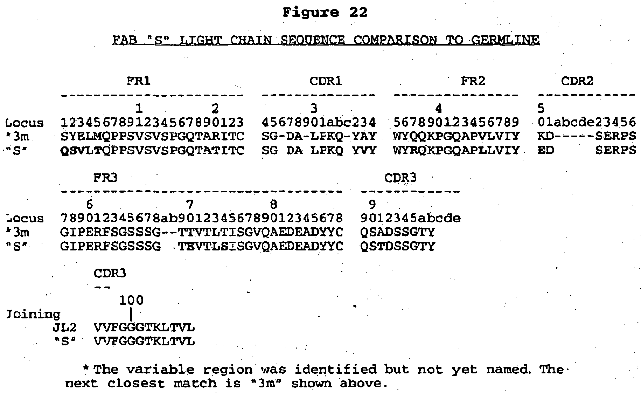

In another embodiment, the V₁ chain comprises a rearranged or somatic variant of a V13 germline gene such as in Figure 22 (SEQ ID NO: 72); and the Vₕ chain comprises or a rearranged or somatic variant of a Vh3 germline gene such as in Figure 18 (SEQ ID NO: 64).

The selective binding agents of the invention (antibody or antigen binding domain) partially or completely inhibit at least one activity of OPGbp, such as binding of OPGbp to ODAR, formation or activation of osteoclasts, or OPGbp-mediated bone resorption and are used to prevent and/or treat bone diseases. In one embodiment, an OPGbp antagonist, such as an antibody or antigen binding domains, is administered to an animal which has experienced loss of bone mass, or is at risk for loss of bone mass, in order to prevent and/or treat loss of bone mass. An OPGbp antagonist may be used to prevent and/or treat osteoporosis, loss of bone mass due to metastasis of cancer to bone; loss-of bone mass due to rheumatoid arthritis, hypercalcemia of malignancy and steroid-induced osteoporosis.

Also provided are compositions comprising the antibodies or antigen binding domains of the invention and a pharmaceutically acceptable carrier.

### Description of the Figures

Figure 1 shows an ELISA of predominant Fab Patterns for reactivity to human OPGbp[143-317]. Titrations were performed using a maximum of 50 *µ*l of phage solution per well to given a typical range 10⁹-10¹¹ phage/well in the ELISA. Phage stocks for ELISA were prepared as described in Example 1. Values were from single point determinations. Patterns "AB" & "X" were superimposed on the same line.
Figure 2 shows inhibition of OPGbp binding to ODAR by Fabs "AT" and "Y". Fabs were purified as described in Example 4 and added to final well concentrations as shown in the figure. Details of the OPGbp/ODAR binding assay are set forth in Example 1. Values were averages of duplicate determinations.
Figure 3 shows bone marrow assays of Fabs "AT" "Y" & "P". The results of one endotoxin-free preparation (0.5 EU/ml or less) each of Fabs "AT", "Y" and "P" were shown. Fabs were purified as described in Example 4 and added to final well dilutions as shown in the figure (Fab stock solutions were 750 *µ*g/ml to 1 mg/ml). The assay format includes a 1 hour preincubation of the anti-hu-OPGbp Fab with 10 ng/ml final cell well concentration of human OPGbp [143-317]. Values were averages of triplicate determinations.
Figure 4 shows Raw cell assays of Fabs "AT" "Y" & "P". The results of one endotoxin-free preparation (0.5 EU/ml or less) each of Fabs "AT", "Y" and "P" were shown. Fabs were purified as described in Example 4. Fabs were preincubated with human OPGbp [143-317] for 1 hour at room temperature before a 1/20 dilution to the final cell well concentration shown on the graph. The final cell well concentration of OPGbp was 20 ng/ml. The cell concentration was 1 x 10⁵ /ml. Values were from triplicate determinations with error bars designating 2 standard deviations (2 STD).
Figure 5 shows the nucleotide and amino acid sequence of Fab "AT" light chain.
Figure 6 shows the nucleotide and amino acid sequence of Fab "Y" light chain.
Figure 7 shows the nucleotide and amino acid sequence of Fab "P" light chain.
Figure 8 shows the nucleotide and amino acid sequence of Fab "S" light chain.
Figure 9 shows the nucleotide and amino acid sequence of Fab "AT" heavy chain.
Figure 10 shows the nucleotide and amino acid sequence of Fab "Y" heavy chain.
Figure 11 shows the nucleotide and amino acid sequence of Fab "P" heavy chain.
Figure 12 shows the nucleotide and amino acid sequence of Fab "S" heavy chain.

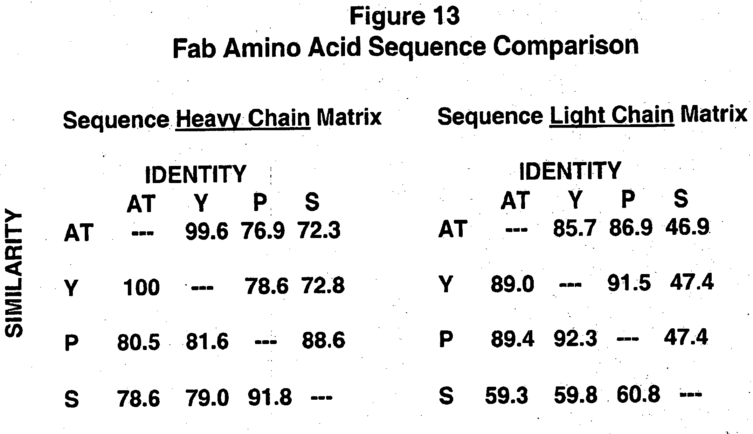

Figure 13 shows a comparison of Fab amino acid sequences shown in Figures 5-12. The predicted amino acid sequences of heavy and light chain Fabs "AT", "Y", "P" and "S" were compared for identity and similarity. Heavy chains "AT" and "Y" differ at only one amino acid position. As library designed, all four Fabs have identical heavy chain CH1 regions comprising the carboxy half of the heavy chain which are included in the calculations of identity and similarity. Light chains "AT", "Y" and "P" share the same or similar V kappa families and therefore differ only at 1 to 2 amino acids in the carboxyl half of the chain, included in the calculations

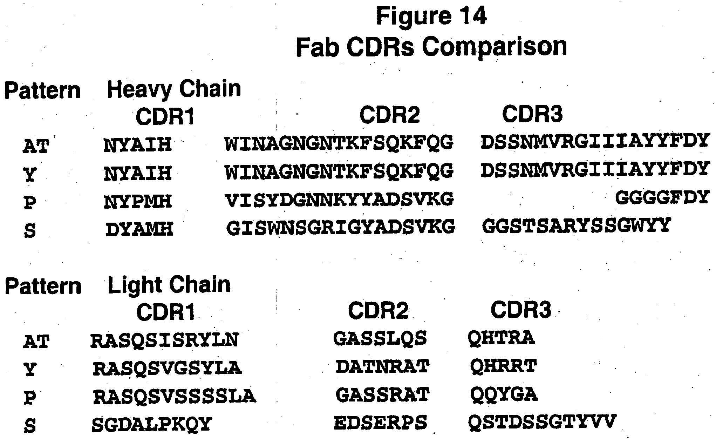

Figure 14 shows a comparison of the predicted heavy and light chain complementarily determining regions (CDRs) of Fabs "AT". "Y" "P" and "S". For heavy chain comparisons, CDR1 includes amino acid residues 32-36 inclusive for all Fabs; CDR2 includes amino acid residues 51-67 inclusive for all Fabs; and CDR3 includes amino acid residues 100-116 inclusive for Fabs "AT" and "Y", 100-106 inclusive for Fab "P", and 100-113 inclusive for Fab "S". For light chain comparisons, CDR1 includes amino acid residues 29-39 inclusive for Fabs "AT" and "Y", 28-39 inclusive for Fab "P", and 27-35 inclusive Fab "S"; CDR2 includes amino acid residues 55-61 inclusive, for Fabs "AT", "Y", and "P", and 53-59 inclusive for Fab "S"; and CDR3 includes amino acid residues 94-98 inclusive for Fabs "AT", "Y" and "P" and 92-102. inclusive for Fab "S".
Figure 15 shows a comparison of Fab classes. Fab class comparison was obtained from V-Base DNA PLOT analysis. The symbol (*) indicates that the closest matching diversity (D) region, although related to known germ line sequences could not be determined. The symbol (**) indicates that the germ line variable (V) region sequence of the closest match has been identified but not formally named to date, being of the rarer lambda family.
Figure 16 shows a comparison of predicted Fab "T" and "Y" heavy chain amino acid sequences (residues 2-127 inclusive in Figures 9 and 10, respectively) with a germline sequence from the VH1 family. The germline sequence comprises the V region sequence 1-03, D region sequence 3-10, and J region sequence JH4 (SEQ. ID NO: 44). FR1, FR2 and FR3 designate the three framework regions, CDR1, CDR2, and CDR3 designate the three complementarily determining regions, and H1, H2 and H3 designate the corresponding junction sequences between framework regions and CDRs. Differences between "AT", "Y" and germline V, D, or J sequences are in boldface. The numbering of germline amino acid residues in Figures 16-22 is as described in Kabat et al. Secuences of Proteins of Immunological Interest, U.S. Department of Health and Human Services, 4th ed. (1991).
Figure 17 shows a comparison of predicted Fab "P" heavy chain amino acid sequences (residues 2-117 inclusive in Figure 11) with a germline sequence from the VH3 family. The sequence comprises the V region sequence 3-30 and the J region sequence JH4. The D region sequence is unknown.
Figure 18 shows a comparison of predicted Fab "S" heavy chain amino acid sequences (residues 2-124 inclusive in Figure 12) with a germline sequence from the VH3 family. The germline sequence comprises the V region sequence 3-09, D region sequence 6-19 and J regions sequence JH4.
Figure 19 shows a comparison of predicted Fab "AT" light chain amino acid sequence (residues 6-108 inclusive in Figure 5) with a germline sequence from the Vkappa1 family. The germline sequence comprises the V region sequence 012 and J region sequence JK1.
Figure 20 shows a comparison of predicted Fab "Y" light chain amino acid sequence (residues 6-108 inclusive in Figure 6) with a germline sequence from the Vkappa3 family. The germline sequence comprises the V region sequence L6 and the J region sequence JK2.
Figure 21 shows a comparison of predicted Fab "P" light chain amino acid sequence (residues 5-108 inclusive in Figure 7) with a germline sequence from the Vkappa3 family. The germline sequence comprises the V region sequence A27 and the J region sequence JK4.
Figure 22 shows a comparison of predicted Fab "S" light chain amino acid sequence (residues 5-112 inclusive in Figure 8) with a germline sequence from the VL3 family. The germline sequence comprises the V region sequence 3m and the J region sequence JL2.

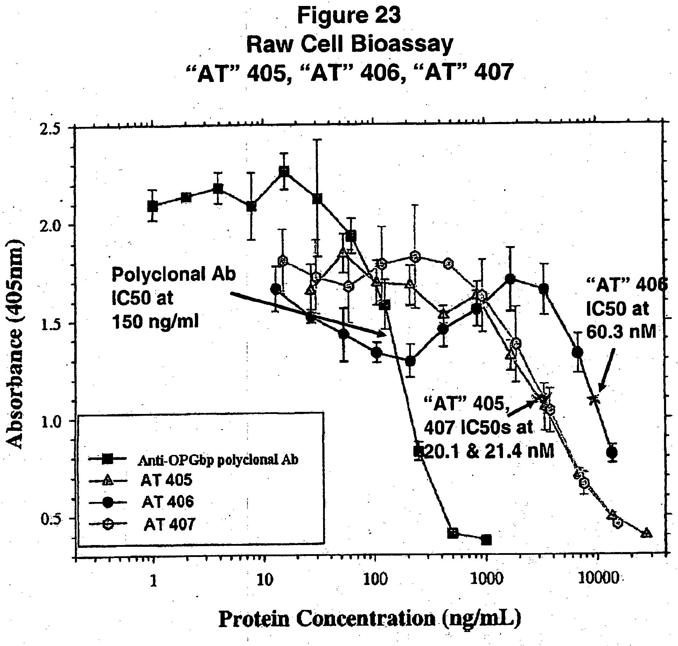

Figure 23 shows RAW cell assays of "AT" 405, "AT" 406 and "AT" 407 isolates. cDNA encoding Fab "AT" was fused to cDNA encoding CH1, CH2 and CH3 regions of human IgG1 as described in Example 7. Different leader sequences were used to produce the resulting isolates designated "AT" 405, "AT" 406 and "AT" 407. "AT" 405-407 were preincubated with OPGbp for 1 hour at room temperature before dilution to the final cell well concentration shown on the graph. The final cell well concentration of OPGbp was 40 ng/ml. Values were from triplicate determinations with error bars designating 2 standard deviations (2 STD).
Figure 24 shows bone marrow assays of "AT" 405 and "AT" 407. The results of one endotoxin-free preparation (0.5 EU/ml or less) of "AT" 405 and "AT" 407 were shown. Samples were pre-incubated with human OPGbp [143-317] for 1 hour at room temperature before addition to the cells. The final cell well dilution for "AT" 405 and "AT" 407 from the sample stock is indicated on the x axis. The final cell well OPGbp concentration was 20 ng/ml.
Figure 25 shows a bone marrow assay of "AT" 406. The results of one endotoxin-free preparation (0.5 EU/ml or less) of "AT" 406 is shown. Samples were pre-incubated with human OPGbp [143-317] for 1 hour at room temperature before addition to the cells. The final cell well concentration of the sample is indicated on the x axis. The final cell well concentration of OPGbp was 20 ng/ml.
Figure 26 shows a bone marrow assay of "S" 435 and "Y". 429. Construction of "S" 435 and "Y" 429 are described in Example 7. The results of one endotoxin-free preparation (0.5 EU/ml or less) of each of "S" 435 and "Y" 429 are shown. Samples were pre-incubated with human OPGbp [143-317] for 1 hour at room temperature before addition to the cells. The final cell well concentration of the sample is indicated on the x axis. The final cell well concentration of OPGbp was 20 ng/ml.
Figure 27 shows a bone marrow assay of "Y" 442 and "P" 444. Construction and expression of "Y" 442 and "P" 444 is described in Example 7. The results of one endotoxin-free preparation (0.5 EU/ml or less) each of "Y" 442 and "P" 444 are shown. Samples were pre-incubated with human OPGbp [143-317] for 1 hour at room temperature before addition to the cells. The final cell well concentration of the sample is shown on the x axis. The final cell well concentration of OPGbp was 20 ng/ml.
Figure 28 shows the nucleic acid and amino acid sequence of FLAG-murine [153-316] OPGbp/DE.
Figure 29 is an alignment of human OPGbp[143-317], murine OPGbp[158-316], and FLAG-murine OPGbp [158-316]/DE amino acid sequences in the region of the DE loop. Underlined are the amino acid residues of human OPGbp introduced into mouse OPGbp to generate the FLAG-mouse OPGbp/DE molecule.
Figure 30 is an enzyme immunoassay examining the binding and reactivity of the AT antibody to plates coated with either human OPGbp[143-317], murine OPGbp[158-316], or FLAG-murine OPGbp [158-316]/DE.

### Detailed Description of the Invention

The present invention provides. a monoclonal antibody which selectively binds OPGbp such that it partially or completely blocks the binding of OPGbp to its cognate receptor and partially or completely inhibits osteoclast formation and/or bone resorption.

The term "selective binding agent" refers to a molecule which preferentially binds OPGbp. A selective binding agent may include a protein, peptide, nucleic acid, carbohydrate, lipid, or small molecular weight compound. In a preferred embodiment, a selective binding agent is an antibody, such as polyclonal antibodies, monoclonal antibodies (mAbs), chimeric antibodies, CDR-grafted antibodies, anti-idiotypic (anti-Id) antibodies to antibodies that can be labeled in soluble or bound form, as well as fragments, regions or derivatives thereof, provided by known techniques, including, but not limited to enzymatic cleavage, peptide synthesis or recombinant techniques. The anti-OPGbp selective binding agents of the present invention are capable of binding portions of OPGbp that inhibit the binding of OPGbp to ODAR receptors.

The antibodies and antigen binding domains of the invention bind selectively to OPGbp, that is they bind preferentially to OPGbp with a greater binding affinity than to other antigens. The antibodies may bind selectively to human OPGbp, but also bind detectably to non-human OPGbp, such as murine OPGbp. Alternatively, the antibodies may bind selectively to non-human OPG, but also bind detectably to human OPG. Alternatively, the antibodies may bind exclusively to human OPGbp, with no detectable binding to non-human OPGbp.

The term "monoclonal antibody" refers to an antibody obtained from a population of substantially homogeneous antibodies wherein each monoclonal antibody will typically recognize a single epitope on the antigen. The term "Monoclonal" is not limited to any particular method for making the antibody. For example, monoclonal antibodies of the invention may be made by the hybridoma method as described in Kohler et al. Nature 256, 495 (1975) or may be isolated from phage libraries using the techniques as described herein, for example.

The term "antigen binding domain" or "antigen binding region" refers to that portion of the selective binding agent (such as an antibody molecule) which contains the amino acid residues that interact with an antigen and confer on the binding agent its specificity and affinity for the antigen. Preferably, the antigen binding region will be of human origin. In other embodiments, the antigen binding region can be derived from other animal species, in particular rodents such as rabbit, rat or hamster.

The term "epitope" refers to that portion of any molecule capable of being recognized by and bound by a selective binding agent (such as an antibody) at one or more of the binding agent's antigen binding regions. Epitopes usually consist of chemically active surface groupings of molecules such as amino acids or sugar side chains and have specific three dimensional structural characteristics as well as specific charge characteristics. By "inhibiting and/or neutralizing epitope" is intended an epitope, which, when bound by a selective binding agent, results in loss of biological activity of the molecule or organism containing the epitope, in *vivo*, in *vitro,* or in *situ*, more preferably *in vivo,* including binding of OPGbp to its receptor.

The term "light chain" when used in reference to an antibody refers to two distinct types, called kappa (k) of lambda (λ) based on the amino acid sequence of the constant domains.

The term "heavy chain" when used in reference to an antibody refers to five distinct types, called alpha, delta, epsilon, gamma and mu, based on the amino acid sequence of the heavy chain constant domain. These distinct types of heavy chains give rise to five classes of antibodies, IgA, IgD, IgE, IgG and IgM, respectively, including four subclasses of IgG, namely IgG₁, IgG₂, IgG₃ and IgG₄.

The term "variable region" or "variable domain" refers to a portion of the light and heavy chains, typically about the amino-terminal 120 to 130 amino acids in the heavy chain and about 100 to 110 amino acids in the light chain, which differ extensively in sequence among antibodies and are used in the binding and specificity of each particular antibody for its particular antigen. The variability in sequence is concentrated in those regions called complimentarily determining regions (CDRs) while the more highly conserved regions in the variable domain are called framework regions (FR). The CDRs of the light and heavy chains are responsible for the interaction of the antibody with antigen.

The term "constant region" or "constant domain" refers to a carboxy terminal portion of the light and heavy chain which is not directly involved in binding of the antibody to antigen but exhibits various effector function, such as interaction with the Fc preceptor.

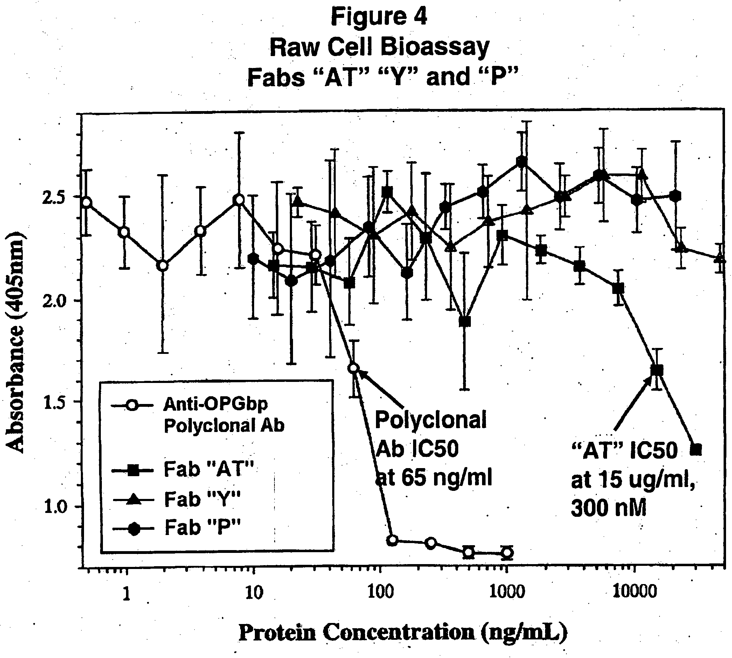

The term "OPGbp" or "OPGbp polypeptide" refers to a polypeptide comprising the amino acid sequence as shown in Figure 4 of PCT publication WO/46757, the disclosure of which is incorporated by reference, and related polypeptides. Related polypeptides include allelic variants; splice variants; fragments; derivatives; substitution, deletion, and insertion variants; fusion polypeptides; and interspecies homologs. Also encompassed are soluble forms of OPGbp, such as residues 69-317 inclusive of human OPGbp (as numbered in WO 98/46757), or a subset thereof which is sufficient to generate an immunological response. In one embodiment, soluble human OPGbp includes residues 140-317 inclusive, 143-317 inclusive, or immunogenic fragments thereof. OPGbp may be a mature polypeptide, as defined herein, and may or may not have an amino terminal methionine residue, depending upon the method by which it is prepared.

The term "fragment" when used in relation to OPGbp or to a proteinaceous selective binding agent of OPGbp refers to a peptide or polypeptide that comprises less than the full length amino acid sequence. Such a fragment may arise, for example, from a truncation at the amino terminus, a truncation at the carboxy terminus, and/or an internal deletion of a residue(s) from the amino acid sequence. Fragments may result from alternative RNA splicing or from in *vivo* protease activity.

The term "variant" when used in relation to OPGbp or to a proteinaceous selective binding agent of OPGbp refers to a peptide or polypeptide comprising one or more amino acid sequence substitutions, deletions, and/or additions as compared to a native or unmodified sequence. For example, an OPGbp variant may result from one or more changes to an amino acid sequence of native OPGbp. Also by way of example, a variant of a selective binding agent of OPGbp may result from one or more changes to an amino acid sequence of a native or previously unmodified selective binding agent. Variants may be naturally occurring, such as allelic or splice variants, or may be artificially constructed. Polypeptide variants may be prepared from the corresponding nucleic acid molecules encoding said variants.

The term "derivative" when used in relation to OPGbp or to a proteinaceous selective binding agent of OPGbp refers to a polypeptide or peptide, or a variant, fragment or derivative thereof, which has been chemically modified. Examples include covalent attachment of one or more polymers, such as water soluble polymers, N-linked, or O-linked carbohydrates, sugars, phosphates, and/or other such molecules. The derivatives are modified in a manner that is different from naturally occurring or starting peptide or polypeptides, either in the type or location of the molecules attached. Derivatives further include deletion of one or more chemical groups which are naturally present on the peptide or polypeptide.

The term "fusion" when used in relation to OPGbp or to a proteinaceous selective binding agent of OPGbp refers to the joining of a peptide or polypeptide, or fragment, variant and/or derivative thereof, with a heterologous peptide or polypeptide.

The term "biologically active" when used in relation to OPGbp or to a proteinaceous selective binding agent refers to a peptide or a polypeptide having at least one activity characteristic of OPGbp or a selective binding agent. A selective binding agent of OPGbp may have agonist, antagonist, or neutralizing or blocking activity with respect to at least one biological activity of OPGbp.

The term "naturally occurring" when used in connection with biological materials such as nucleic acid molecules, polypeptides, host cells, and the like, refers to those which are found in nature and not manipulated by a human being.

The term "isolated" when used in relation to OPGbp or to a proteinaceous selective binding agent of OPGbp refers to a peptide or polypeptide that is free from at least one contaminating polypeptide that is found in its natural environment, and preferably substantially free from any other contaminating mammalian polypeptides which would interfere with its therapeutic or diagnostic use.

The term "mature" when used in relation to OPGbp or to a proteinaceous selective binding agent of OPGbp refers to a peptide or polypeptide lacking a leader sequence. The term may also include other modifications of a peptide or polypeptide such as proteolytic processing of the amino terminus (with or without a leader sequence) and/or the carboxy terminus, cleavage of a smaller polypeptide from a larger precursor, N-linked and/or O-linked glycosylation, and the like.

The terms "effective amount" and "therapeutically effective amount" when used in relation to a selective binding agent of OPGbp refers to an amount of a selective binding agent that is useful or necessary to support an observable change in the level of one or more biological activities of OPGbp. Said change may be either an increase or decrease in the level of OPGbp activity.

The term "conservative amino acid substitution" refers to a substitution of a native amino acid residue with a non-native residue such that there is little or no effect on the polarity or charge of the amino acid residue at that position. For example, a conservative substitution results from the replacement of a non-polar residue in a polypeptide with any other non-polar residue. Furthermore, any native residue in a polypeptide may also be substituted with alanine, as has been previously described for alanine scanning mutagenesis (Cunningham et al. Science 244, 1081-1085 (1989). Exemplary rules for conservative amino acid substitutions are set forth in Table I.

**Table I**

| Conservative Amino Acid Substitutions | | |
|---|---|---|
| Original Residues | Exemplary Substitutions | Preferred Substitutions |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn | Asn |
| Glu | Asp | Asp |
| Gly | Pro, Ala | Ala |
| His | Asn, Gln, Lys, Arg | Arg |
| Ile | Leu,Val,Met,Ala, Phe,Norleucine | Leu |
| Leu | Norleucine, Ile, Val,Met,Ala,Phe | Ile |
| Lys | Arg, Gln, Asn | Arg |
| Met | Leu, Phe, Ile | Leu |
| Phe | Leu, Val, Ile, Ala, Tyr | Leu |
| Pro | Ala | Ala |
| Ser | Thr | Thr |
| Thr | Ser | Ser |
| Trp | Tyr, Phe | Tyr |
| Tyr | Trp,Phe,Thr,Ser | Phe |
| Val | Ile, Met, Leu, Phe, Ala, Norleucine | Leu |

Conservative amino acid substitutions also encompass non-naturally occurring amino acid residues which are typically incorporated by chemical peptide synthesis rather than by synthesis in biological systems. These include peptidomimetics, and other reversed or inverted forms of amino acid moieties.

Conservative modifications to the amino acid sequence (and the corresponding modifications to the encoding nucleotides) are able to produce OPGbp polypeptides (and proteinaceous selective binding agents thereof) having functional and chemical characteristics similar to those of naturally occurring OPGbp or selective binding agents. In contrast, substantial-modifications in the functional and/or chemical characteristics of OPGbp (and protineaceous selective binding agents thereof) may be accomplished by selecting substitutions that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain. Naturally occurring residues may be divided into groups based on common side chain properties:
1) Hydrophobic: norleucine, Met, Ala, Val, Leu, Ile;
2) Neutral hydrophilic: Cys, Ser, Thr;
3) Acidic: Asp, Glu;
4) Basis: Asn, Gln, His, Lys, Arg;
5) Residues that influence chain orientation: Gly, Pro; and
6) Aromatic: Trp, Tyr, Phe.
Non-conservative substitutions may involve the exchange of a member of one of these classes for a member from another class.

The identity or similarity" of two or more nucleic acid molecules and/or polypeptides provides a measure of the relatedness of two or more distinct sequences. The term "identity" refers to amino acids which are identical at corresponding positions in two distinct amino acid sequences. The term "similarity." refers to amino acids which are either identical or are conservative substitutions as defined above at corresponding positions in two distinct amino acid sequences.

The extent of identity or similarity can be readily calculated by known methods, including but not limited to those described in Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math., 48, 1073 (1988).

Preferred methods to determine identity and/or similarity are designed to give the largest match between the sequences tested. Methods to determine identity and similarity are codified in publicly available computer programs. Exemplary computer program methods to determine identity and similarity between two sequences include, but are not limited to, the GCG program package, including GAP (Devereux et al., Nucleic Acids Research 12, 387 (1984); Genetics Computer Group, University of Wisconsin, Madison, WI), BLASTP, BLASTN, and FASTA (Altschul et al., J. Mol. Biol. , 215, 403-410 (1990)). The BLAST X program is publicly available from the National Center for Biotechnology Information (NCBI) and other sources (*BLAST Manual,* Altschul et *al*. NCB NLM NIH Bethesda, MD). The well known Smith Waterman algorithm may also be used to determine identity.

### OPGbp polypeptides

OPGbp polypeptides, and fragments, variants and derivatives thereof, are used as target molecules for screening and identifying the selective binding agents. When it is desired to prepare antibodies as selective binding agents, OPGbp polypeptides are preferably immunogenic, that is they elicit an immune response when administered to an animal. Alternatively, when antibodies are prepared by in vitro techniques, OPGbp polypeptides used as target molecules are capable of detectably binding an antibody or antigen binding domain.

OPG polypeptides are prepared by biological or chemical methods. Biological methods such as expression of DNA sequences encoding recombinant OPGbp are known in the art (see for example Sambrook et al. supra). Chemical synthesis methods such as those set forth by Merrifield et al., J. Am. Chem. Soc., 85:2149 (1963), Houghten et al., Proc Natl Acad. Sci. USA, 82:5132 (1985), and Stewart and Young, Solid Phase Peptide Synthesis, Pierce Chemical Co., Rockford, IL (1984) may also be used to prepare OPGbp polypeptides Such polypeptides may be synthesized with or without a methionine on the amino terminus. Chemically synthesized OPGbp polypeptides, or fragments or variants thereof, may be oxidized using methods set forth in these references to form disulfide bridges. OPGbp polypeptides of the invention prepared by chemical synthesis will have at least one biological activity comparable to the corresponding OPGbp polypeptides produced recombinantly or purified from natural sources.

OPGbp polypeptides may be obtained by isolation from biological samples such as source tissues and/or fluids in which the OPGbp polypeptides are naturally found. Sources for OPGbp polypeptides may be human or non-human in origin. Isolation of naturally-occurring OPGbp polypeptides can be accomplished using methods known in the art, such as separation by electrophoresis followed by electroelution, various types of chromatography (affinity, immunoaffinity, molecular sieve, and/or ion exchange), and/or high pressure liquid chromatography. The presence of the OPGbp polypeptide during purification may be monitored using, for example, an antibody prepared against recombinantly produced OPGbp polypeptide or peptide fragments thereof.

Polypeptides include isolated OPGbp polypeptides and polypeptides related thereto including fragments, variants, fusion polypeptides, and derivatives as defined hereinabove. OPGbp fragments of the invention may result from truncations at the amino terminus (with or without a leader sequence), truncations at the carboxy terminus, and/or deletions internal to the polypeptide. Such OPGbp polypeptides fragments may optionally comprise an amino terminal methionine residue. The polypeptides will be immunogenic in that they will be capable of eliciting an antibody response.

OPGbp polypeptide variants may include one or more amino acid substitutions, additions and/or deletions as compared to the native OPGbp amino acid sequence. Amino acid substitutions may be conservative, as defined above, or non-conservative or any combination thereof. The variants may have additions of amino acid residues either at the carboxy terminus or at the amino terminus (where the amino terminus may or may not comprise a leader sequence). OPGbp glycosylation variants include variants wherein the number and/or type of glycosylation sites has been altered compared to native OPGbp polypeptide. OPGbp glycosylation variants may comprise a greater or a lesser number of N-linked glycosylation sites compared to native OPGbp. Also illustrated are OPGbp glycoyslation variants comprising a rearrangement of N-linked carbohydrate chains wherein one or more N-linked glycosylation sites (typically those that are naturally occurring) are eliminated and one or more new N-linked sites are created. OPGbp cysteine variants comprise a greater number or alternatively a lesser number of cysteine residues compared to native OPGbp. In one embodiment, one or more cysteine residues are deleted or substituted with another amino acid (e.g., serine). Cysteine variants of OPGbp can improve the recovery of biologically active OPGbp by aiding the refolding of OPGbp into a biologically active conformation after isolation from a denatured state.

Preparing OPGbp polypeptide variants is within the level of skill in the art. In one approach, one may introduce one or more amino acid substitutions, deletions and/or additions in native OPGbp wherein the OPGbp variant retains the native structure of OPGbp and/or at least one of the biological activities. One approach is to compare sequences of OPG polypeptides from a variety of different species in order to identify regions of relatively low and high identity and/or similarity. It is appreciated that those regions of an OPGbp polypeptide having relatively low identity and/or similarity, are less likely to be essential for structure and activity and therefore may be more tolerant of amino acid alterations, especially those which are non-conservative. It is also appreciated that even in relatively conserved regions, one could introduce conservative amino acid substitutions while retaining activity.

In another approach, structure-function relationships can be used to identify residues in similar polypeptides that are important for activity or structure. For example, one may compare conserved amino acid residue among OPGbp and other members of the tumor necrosis factor family for which structure-function analyses are available and, based on such a comparison, predict which amino acid residues in OPGbp are important for activity or structure. One skilled in the art may choose chemically similar amino acid substitutions for such predicted important amino acid residues of OPGbp.

In yet another approach, an analysis of a secondary or tertiary structure of OPGbp (either determined by x-ray diffraction of OPGbp crystals or by structure prediction methods) can be undertaken to determine the location of specific amino acid residues in relation to actual or predicted structures within an OPGbp polypeptide. Using this information, one can introduce amino acid changes in a manner that seeks to retain as much as possible the secondary and/or tertiary structure of an OPGbp polypeptide.

In yet another approach, the effects of altering amino acids at specific positions may be tested experimentally by introducing amino acid substitutions and testing the altered OPGbp polypeptides for biological activity using assays described herein. Techniques such as alanine scanning mutagenesis (Cunningham et al., supra) are particularly suited for this approach. Many altered sequence may be conveniently tested by introducing many substitutions at various amino acid positions in OPGbp and screening the population of altered polypeptides as part of a phage display library. Using this approach, those regions of an OPGbp polypeptide that are essential for activity may be readily determined.

The above methods are useful for generating OPGbp variants which retain the native structure. Thus, antibodies raised against each variants are likely to recognize a native structural determinant, or epitope, of OPGbp and are also likely to bind to native OPGbp. However, in some cases is may be desirable to produce OPGbp variants which do not retain native OPGbp structure or are partially or completely unfilled. Antibodies raised against such proteins will recognize buried epitopes on OPGbp.

The invention also illustrates OPGbp fusion polypeptides which comprise OPGbp polypeptides, and fragments, variants, and derivatives thereof, fused to a heterologous peptide or protein. Heterologous peptides and proteins include, but are not limited to: an epitope to allow for detection and/or isolation or a OPGbp fusion polypeptide; a transmembrane receptor protein or a portion thereof, such as an extracellular domain, or a transmembrane and intracellular domain; a ligand or a portion thereof which binds to a transmembrane receptor protein; an enzyme or portion thereof which is catalytically active; a protein or peptide which promotes oligomerization, such as leucine zipper domain;-and a protein or peptide which increases stability, such as an immunoglobulin constant region. A OPGbp polypeptide may be fused to itself or to a fragment, variant, or derivative thereof. Fusions may be made either at the amino terminus or at the carboxy terminus of a OPGbp polypeptide, and may be direct with no linker or adapter molecule or may be through a linker or adapter molecule. A linker or adapter molecule may also be designed with a cleavage site for a DNA restriction endonuclease or for a protease to allow for separation of the fused moieties.

A OPGbp polypeptide, fragment, variant and/or derivative may be fused to an Fc region of human IgG. In one example, a human IgG hinge, CH2 and CH3 region may be fused at either the N-terminus or C-terminus of the OPGbp polypeptides using methods known to the skilled artisan. In another example, a portion of a hinge regions and CH2 and CH3 regions may be fused. The OPGbp Fc-fusion polypeptide so produced may be purified by use of a Protein A affinity column. In addition, peptides and proteins fused to an Fc region have been found to exhibit a substantially greater half-life *in vivo* than the unfused counterpart. Also, a fusion to an Fc region allows for dimerization/multimerization of the fusion polypeptide. The Fc region may be a naturally occurring Fc region, or may be altered to improve certain qualities, such as therapeutic qualities, circulation time, reduce aggregation, etc.

OPGbp polypeptide derivatives are chemically modified OPGbp polypeptide compositions in which OPGbp polypeptide is linked to a polymer. The polymer selected is typically water soluble so that the protein to which it is attached does not precipitate in an aqueous environment, such as a physiological environment. The polymer may be of any molecular weight, and may be branched or unbranched. Included within the scope of OPGbp polypeptide polymers is a mixture of polymers. For therapeutic use of the end-product preparation, the polymer will be pharmaceutically acceptable.

The water soluble polymer or mixture thereof may be for example, polyethylene glycol (PEG), monomethoxy-polyethylene glycol, dextran (such as low molecular weight dextran, of, for example about 6 kD), cellulose, or other carbohydrate based polymers, poly-(N-vinyl pyrrolidone) polyethylene glycol, propylene glycol homopolymers, a polypropylene oxide/ethylene oxide co-polymer, polyoxyethylated polyols (*e*.*g*., glycerol) and polyvinyl alcohol.

A water soluble polymer is polyethylene glycol. As used herein, polyethylene glycol is meant to encompass any of the forms of PEG that have been used to derivatize other proteins, such as mono- (C₁-C₁₀) alkoxy-, or aryloxy-polyethylene glycol. Also encompassed by the invention are bifunctional PEG crosslinking molecules which may be used to prepare covalently attached OPGbp multimers.

Methods for preparing chemically derivatized OPGbp polypeptides are known in the art. By way of example, derivatization of OPGbp polypeptides with PEG may be carried out using procedures described in Francis et al., Focus on Growth Factors. 3, 4-10 (1992); EP 0 154 316; EP 0 401 384, and U.S. Patent No. 4,179,337. In a preferred embodiment, an OPGbp polypeptide derivative will have a single PEG moiety at the amino terminus. See U.S. Patent No. 5, 234, 784

OPGbp polypeptide derivatives disclosed herein may exhibit an enhancement or reduction of at least one biological activity of OPGbp compared to unmodified polypeptide, or may exhibit increased or decreased half-life or stability.

### OPGbp selective binding agents

OPGbp polypeptides, and fragments, variants and derivatives thereof, may be used to identify selective binding agents of OPGbp. As defined above, a selective binding agent of OPGbp encompasses both proteinaceous and non-proteinaceous binding agents and, in one preferred embodiment of the invention, the selective binding agent is proteinaceous. The selective binding agent may be an antibody or fragment thereof which binds OPGbp, preferably human OPGbp. The antibodies of the invention may be agonist antibodies, which enhance the level of at least one biological activity of OPGbp; or antagonist antibodies, which decrease the level of at least one biological activity of OPGbp. Antagonist antibodies of OPGbp may also be referred to as inhibitory or neutralizing antibodies of OPGbp.

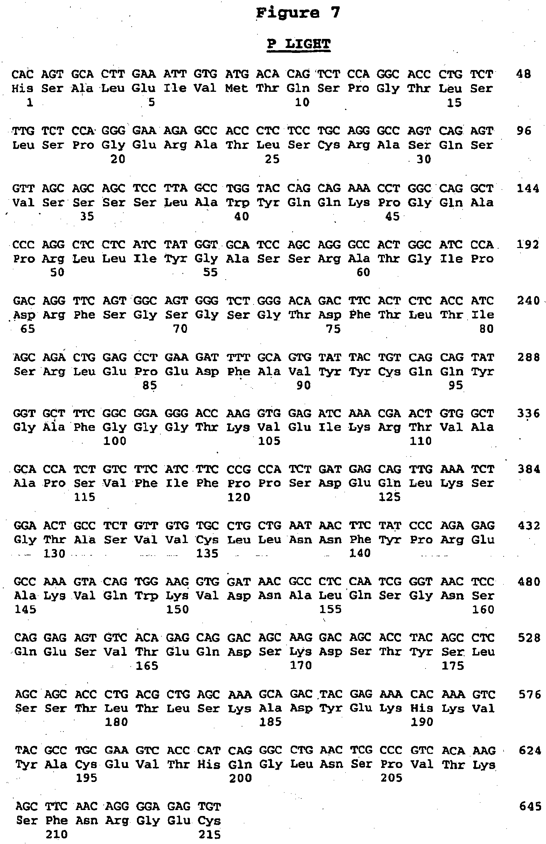

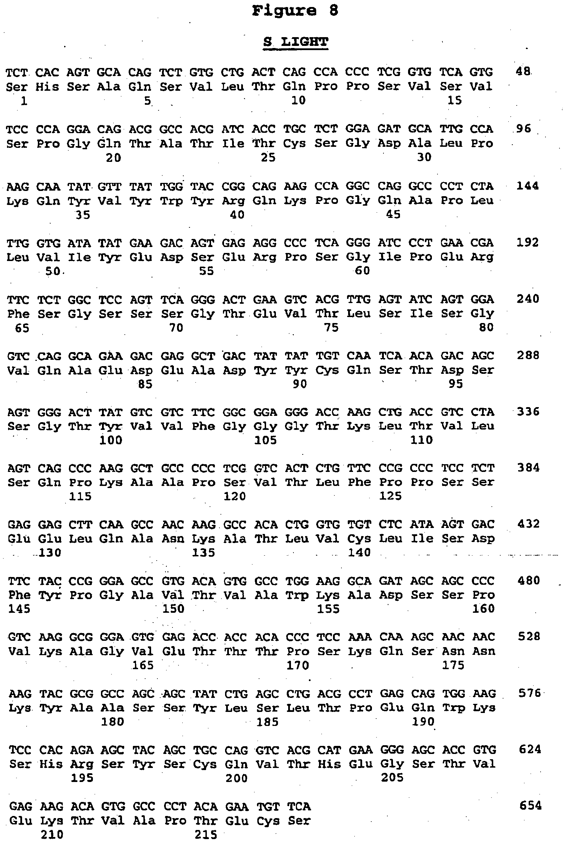

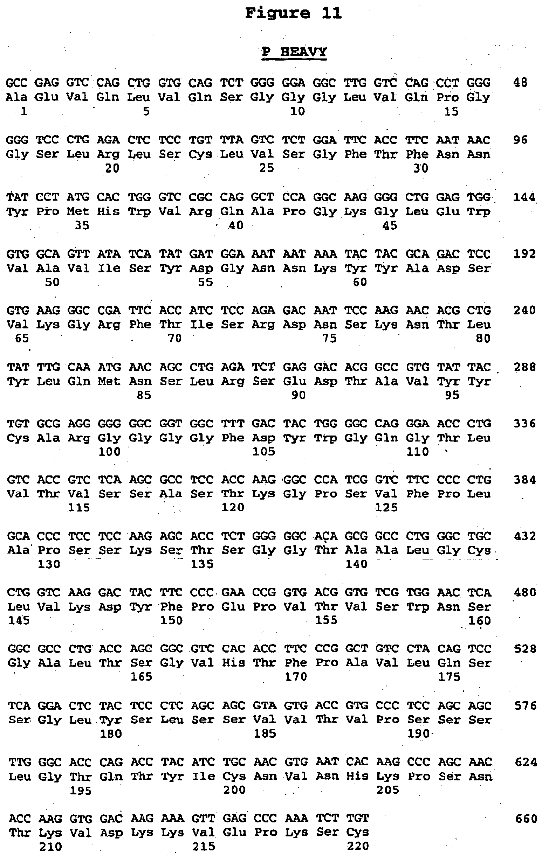

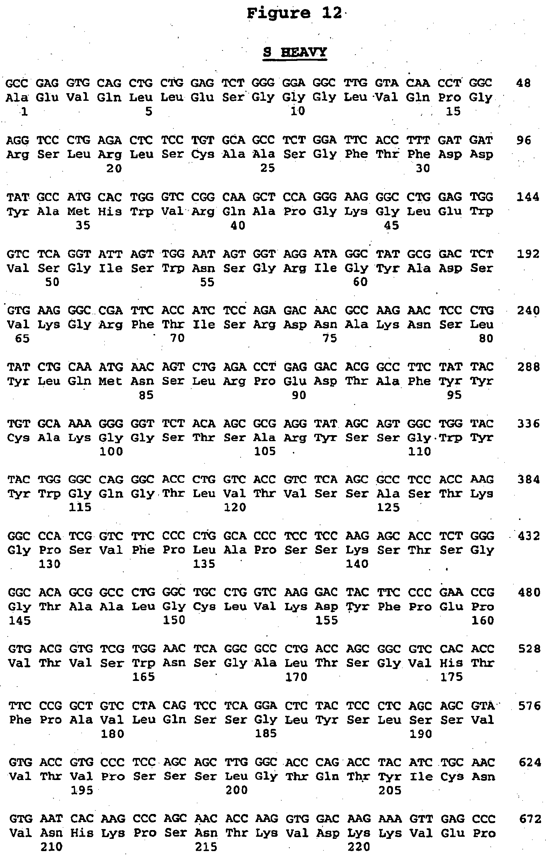

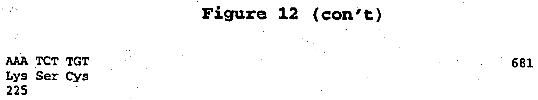

As described in the examples below, anti-OPGbp antibodies and antigen binding domains which inhibit at least one activity of OPGbp have been identified. Embodiments of the invention include antibodies comprising a heavy chain Fab sequence as shown in any of Figures 9, 10, 11 or 12 and further comprising a kappa or lambda light chain sequence. Light chain Fab sequences may be as shown in Figures 5, 6, 7 or 8. For example, "AT" antibody has light and heavy chain sequences in Figures 5 and 9, respectively; "Y" antibody has light and heavy chains sequences of Figures 6 and 10, respectively; "S" antibody has light and heavy chain sequences of Figures 7 and 11, respectively; and "P" antibody has light and heavy chain sequences of Figures 8 and 12, respectively. The antibodies of the invention further comprise a human Fc region from any isotype, either IgG, IgM, IgA, IgE, or IgD. Preferably, the Fc region is from human IgG, such as IgG1, IgG2, IgG3, or IgG4.

The invention also provides for antibodies or antigen binding domains which comprise fragments, variants, or derivatives of the Fab sequences disclosed herein. Fragments include variable domains of either the light or heavy chain Fab sequences which are typically joined to light or heavy constant domains. Variants include antibodies comprising light chain Fab sequences which are at least about 80%, 85%, 90%, 95%, 98% or 99% identical or similar to the Fab sequences, or the corresponding variable domains, in any one of Figures 5-8, or antibodies comprising heavy chain Fab sequences, or the corresponding variable domains, which are at least about 80%, 85%, 90%, 95%, 98% or 99% identical or similar to the Fab sequences in any one of Figures 9-12. The antibodies may be typically associated with constant regions of the heavy and light chains to form full-length antibodies.

Antibodies and antigen binding domains, and fragments, variants and derivatives thereof, of the invention will retain the ability to bind selectively to human OPGbp polypeptide. In one embodiment, an antibody will bind an OPGbp polypeptide with a dissociation constant (KD) of about 1 nM or less, or alternatively 0.1 nM or less, or alternatively 10 pM or less or alternatively less than 10 pM. In Example 8, it was observed that "AT" antibody binds to OPGbp with a KD of about 0.33 to 0.43 nM.

Antibodies of the invention include monoclonal, recombinant, chimeric, humanized, fully human, single chain and/or bispecific antibodies. Antibody fragments include those portions of an anti-OPGbp antibody which bind to an epitope on an OPGbp polypeptide. Examples of such fragments include Fab F(ab'), F(ab)', Fv, and sFv fragments. The antibodies may be generated by enzymatic cleavage of full-length antibodies or by recombinant DNA techniques, such as expression of recombinant plasmids containing nucleic acid sequences encoding antibody variable regions.

Polyclonal antibodies are heterogeneous populations of antibody molecules derived from the sera of animals immunized with an antigen. An antigen is a molecule or a portion of a molecule capable of being bound by an antibody which is additionally capable of inducing an animal to produce antibody capable of binding to an epitope of that antigen. An antigen can have one or more epitope. The specific reaction referred to above is meant to indicate that the antigen will react, in a highly selective manner, with its corresponding antibody and not with the multitude of other antibodies which can be evoked by other antigens.

Polyclonal antibodies directed toward an OPGbp polypeptide generally are raised in animals (*e*.*g*., rabbits or mice) by multiple subcutaneous or intraperitoneal injections of OPGbp and an adjuvant. In accordance with the invention, it may be useful to conjugate an OPGbp polypeptide, or a variant, fragment, or derivative thereof to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-OPGbp antibody titer.

Monoclonal antibodies (mAbs) contain a substantially homogeneous population of antibodies specific to antigens, which population contains substantially similar epitope binding sites. Such antibodies may be of any immunoglobulin class including IgG, IgM, IgE, IgA, IgD and any subclass thereof. A hybridoma producing a monoclonal antibody of the present invention may be cultivated *in vitro, in situ,* or *in vivo.* Production of high titers *in vivo* or in *situ* is a preferred method of production.

Monoclonal antibodies directed toward OPGbp are produced using any method which provides for the production of antibody molecules by continuous cell lines in culture. Examples of suitable methods for preparing monoclonal antibodies include hybridoma methods of Kohler et al., Nature 256, 495-497 (1975), and the human B-cell hybridoma method, Kozbor, J. Immunol. 133, 3001 (1984); Brodeur et al., Monoclonal Antibody Production Techniques and Applications, pp. 51-63 (Marcel Dekker, Inc., New York, 1987); and Harlow and Lane, Antibodies: A Laboratory Manual, Cold Spring Harbor Laboratory (1988).

Preferred anti-OPGbp selective binding agents include monoclonal antibodies which will inhibit partially or completely the binding of human OPGbp to its cognate receptor, ODAR, or an antibody having substantially the same specific binding characteristics, as well as fragments and regions thereof. Preferred methods for determining monoclonal antibody specificity and affinity by competitive inhibition can be found in Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc. and Wiley Interscience, N.Y., (1992, 1993), and Muller, Meth. Enzymol., 92:589-601 (1983).

Also provided are hybridoma cell lines which produce monoclonal antibodies reactive with OPGbp polypeptides.

Chimeric antibodies are molecules in which different portions are derived from different animal species, such as those having a variable region derived from a murine monoclonal antibody and a human immunoglobulin constant region. Chimeric antibodies are primarily used to reduce immunogenicity in application and to increase yields in production, for example, where murine monoclonal antibodies have higher yields from hybridomas but higher immunogenicity in humans, such that human/murine chimeric monoclonal antibodies are used.

Chimeric antibodies and methods for their production are known in the art. Cabilly et al., Proc. Natl. Acad. Sci. USA, 81:3273-3277 (1984); Morrison et al., Proc. Natl. Acad. Sci. USA, 81:6851-6855 (1984); Boulianne et al., Nature, 312:643-646 (1984); Neuberger et al., Nature, 314:268-270 (1985); Liu et al., Proc. Natl. Acad. Sci. USA, 84:3439-3443 (1987); and Harlow and Lane Antibodies: A Laboratory Manual, Cold Spring Harbor Laboratory (1988).

For example, chimeric monoclonal antibodies of the invention may be used as a therapeutic. In such a chimeric antibody, a portion of the heavy and/or light chain is identical with or homologous to corresponding sequence in antibodies derived from a particular species or belonging to one particular antibody class or subclass, while the remainder of the chain(s) is identical with or homologous to corresponding sequence in antibodies derived from another species or belonging to another antibody class or subclass, as well as fragments of such antibodies, so long as they exhibit the desired biological activity (see U.S. Patent No. 4,816;567; Morrison et al., Proc. Natl. Acad. Sci., 81, 6851-6855 (1985).

As used herein, the term "chimeric antibody" includes monovalent, divalent or polyvalent immunoglobulins. A monovalent chimeric antibody is a dimer (HL) formed by a chimeric H chain associated through disulfide bridges with a chimeric L chain. A divalent chimeric antibody is tetramer (H,L,) formed by two HL dimers associated through at least one disulfide bridge. A polyvalent chimeric antibody can also be produced, for example, by employing a C_{E} region that aggregates (*e*.*g*., from an IgM H chain, or µ chain).

Murine and chimeric antibodies, fragments and regions of the present invention may comprise individual heavy (H) and/or light (L) immunoglobulin chains. A chimeric H chain comprises an antigen binding region derived from the H chain of a non-human antibody specific for OPGbp, which is linked to at least a portion of a human H chain C region (C_{H}), such as CH₁ or CH₂.

A chimeric L chain according to the present invention comprises an antigen binding region derived from the L chain of a non-human antibody specific for OPGbp, linked to at least a portion of a human L chain C region (C_{L}).

Selective binding agents, such as antibodies, fragments, or derivatives, having chimeric H chains and L chains of the same or different variable region binding specificity, can also be prepared by appropriate association of the individual polypeptide chains, according to known method steps, *e.g.,* according to Ausubel et al., eds. Current Protocols in Molecular Biology, Wiley Interscience, N.Y. (1993), and Harlow et al., Antibodies: A Laboratory Manual, Cold Spring Harbor Laboratory Press, Cold Spring Harbor, N.Y. (1988). With this approach, hosts expressing chimeric H chains (or their derivatives) are separately cultured from hosts expressing chimeric L chains (or their derivatives), and the immunoglobulin chains are separately recovered and then associated. Alternatively, the hosts can be co-cultured and the chains allowed to associate spontaneously in the culture medium, followed by recovery of the assembled immunoglobulin, fragment or derivative.

As an example, the antigen binding region of the selective binding agent (such as a chimeric antibody) of the present invention is preferably derived from a non-human antibody specific for human OPGbp. Preferred sources for the DNA encoding such a non-human antibody include cell lines which produce antibodies, such as hybrid cell lines commonly known as hybridomas.

The invention also provides for fragments, variants and derivatives, and fusions of anti-OPGbp antibodies, wherein the terms "fragments", "variants", "derivatives" and "fusions" are defined herein. The invention encompasses fragments, variants, derivatives, and fusions of anti-OPGbp antibodies which are functionally similar to the unmodified anti-OPGbp antibody, that is, they retain at least one of the activities of the unmodified antibody. In addition to the modifications set forth above, also included is the addition of genetic sequences coding for cytotoxic proteins such as plant and bacterial toxins. The fragment, variants, derivatives and fusions of anti-OPGbp antibodies can be produced from any of the hosts of this invention.

Suitable fragments include, for example, Fab, Fab', P(ab')₂, Fv and scFv. These fragments lack the Fc fragment of an intact antibody, clear more rapidly from the circulation, and can have less non-specific tissue binding than an intact antibody. See Wahl et al., J. Nucl. Med., 24:316-325 (1983). These fragments are produced from intact antibodies using methods well known in the art, for example by proteolytic cleavage with enzymes such as papain (to produce Fab fragments) or pepsin (to produce F(ab')₂ fragments). The identification of these antigen binding regions and/or epitopes recognized by monoclonal antibodies of the present invention provides the information necessary to generate additional monoclonal antibodies with similar binding characteristics and therapeutic or diagnostic utility that parallel the embodiments of this application.

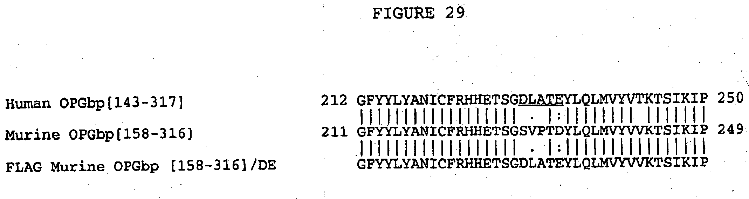

The invention provides for anti-OPGbp antibodies, or antigen binding domains, which recognize and bind to inhibiting and/or neutralizing epitopes on OPGbp. As a result of this binding, an anti-OPGbp antibody may partially or completely inhibit binding of OPGbp to its receptor, or may partially or completely inhibit osteoclast formation, bone resoprtion and/or bone loss. More particularly, the invention provides for anti-OPGbp antibodies which recognize and bind to an epitope comprising a portion of the amino acid sequence of a DE region of OPGbp (a "DE epitope"). A DE region of OPGbp spans approximately the D and E beta sheet regions and intervening loop sequence (a "DE loop"). The DE region in human OPGbp comprises from about amino acid residue 212 to about amino acid residue 250 inclusive (see Figure 29).

While it is contemplated that an anti-OPGbp antibody, or an antigen binding domain, may bind at any location within a DE region, a preferred embodiment is an anti-OPGbp antibody which binds to at least part of a DE loop. The DE loop in human OPGbp spans approximately five amino acids and is located at about residues 230-234 inclusive. The DE loop in human OPGbp has the sequence DLATE. However, the amino acid sequence and endpoints of the DE loop of human OPGbp are merely exemplary and it is understood that DE loops could have sequences and endpoints which vary from those in human OPGbp. The invention encompass antibodies which bind to such variable DE loops.

As shown in Example 10, introduction of the sequence DLATE into the corresponding DE loop of murine OPGbp resulted in binding of "AT" antibody whereas the antibody had no detectable affinity for murine OPGbp with the native DE loop sequence up to an antibody concentration of about 2 *µ*g/ml. In another embodiment, an anti-OPGbp antibody binds to the amino acid sequence DLATE in human OPGbp, or to a portion of said sequence. In another embodiment, an anti-OPGbp antibody, or antigen binding domain, binds to murine OPGbp comprising the amino acid substitutions S229D, V230L, P231A and D233E, but does not bind to murine OPGbp lacking said substitutions under similar conditions.

While the antibodies of the invention are characterized in part by the amino acid sequences on OPGbp to which they bind, it is understood and appreciated by one skilled in the art that a DE epitope on OPGbp recognized by an antibody typically comprises a three dimensional structure which may involve amino acids outside the DE region. In a linear representation of an OPGbp sequence, amino acids comprising the DE epitope may be distant from the DE region, but in a three dimensional structure of OPGbp, amino acids of the DE epitope will likely be in proximity to the DE region. Thus, it is understood that binding of an anti-OPGbp antibody to a DE epitope may involve amino acids other than those in the DE region. Nonetheless, it has been shown that amino acid residues in the DE loop, especially some or all of the residues in the sequence DLATE, are involved in antibody binding to OPGbp and inhibition of OPGbp activity.

Variants of selective binding agents are also provided. In one embodiment, variants of antibodies and antigen binding domains comprise changes in light and/or heavy chain amino acid sequences that are naturally occurring or are introduced by in vitro engineering of native sequences using recombinant DNA techniques. Naturally occurring variants include "somatic" variants which are generated in vivo in the corresponding germ line nucleotide sequences during the generation of an antibody response to a foreign antigen. Variants encoded by somatic mutations in germline variable light and heavy chain sequences which generate the exemplary Fabs of the present invention in sequences are shown in Figures 16 and 19 for Fab "AT", Figures 16 and 20 for Fab "Y", Figures 17 and 21 for Fab "P" and Figures 18 and 22 for Fab "S".

Variants of anti-OPGbp antibodies and antigen binding domains are also prepared by mutagenesis techniques known in the art. In one example, amino acid changes may be introduced at random throughout an antibody coding region and the resulting variants may be screened for a desired activity, such as binding affinity for OPGbp. Alternatively, amino acid changes may be introduced in selected regions of an OPGbp antibody, such as in the light and/or heavy chain CDRs, and framework regions, and the resulting antibodies may be screened for binding to OPGbp or some other activity. Amino acid changes encompass one or more amino acid substitutions in a CDR, ranging from a single amino acid difference to the introduction of all possible permutations of amino acids within a given CDR, such as CDR3. In another method, the contribution of each residue within a CDR to OPGbp binding may be assessed by substituting at least one residue within the CDR with alanine (Lewis et al., Mol. Immunol. 32, 1065-1072 (1995)). Residues which are not optimal for binding to OPGbp may then be changed in order to determine a more optimum sequence. Also encompassed are variants generated by insertion of amino acids to increase the size of a CDR, such as CDR3. For example, most light chain CDR3 sequences are nine amino acids in length. Light chain CDR3 sequences in an antibody which are shorter than nine residues may be optimized for binding to OPGbp by insertion of appropriate amino acids to increase the length of the CDR.

In one embodiment, antibody or antigen binding domain variants comprise one or more amino acid changes in one or more of the heavy or light chain CDR1, CDR2 or CDR3 and optionally one or more of the heavy or light chain framework regions FR1, FR2 or FR3. Amino acid changes comprise substitutions, deletions and/or insertions of amino acid residues. Exemplary variants include an "AT" heavy chain variable region variant with one or more amino acid changes in the sequences NYAIH (SEQ ID NO: 13); WINAGNGNTIKFSQKFQF (SEQ ID NO: 16); or DSSNMVRGIIIAYYFDY (SEQ ID NO: 19), or an "AT" light chain variable region variant with one or more amino acid changes in the sequences RASQSISRYLN (SEQ ID NO: 01); GASSLQS (SEQ ID NO: 05); or QHTRA (SEQ ID NO: 09). The aforementioned "AT" heavy and light chain variable region variants may further comprise one or more amino acid changes in the framework regions. In one example, one or more amino acid changes may be introduced to substitute a somatically mutated framework residue with the germline residue at that position. When the aforementioned amino acid changes are substitutions, the changes may be conservative or non-conservative substitutions.

Examples 11 and 12 provide variants in light and heavy chain CDR3 region of AT antibody. In one embodiment, the invention provides for variants in either SEQ ID NO:19 (heavy chain CDR3) or SEQ ID NO:9 (light chain CDR3) such that the resulting antibodies or antigen binding domains bind selectively to an OPG binding protein. In one embodiment, the OPGbp is human OPGbp.

The invention provides for anti-OPG bp antibodies comprising variable light and variable heavy chains and further comprising a heavy chain CDR3 region having the sequence selected from the group consisting of:
XSSNMVRGIIIAYYFDY (SEQ ID NO: 80);
DXSNMVRGIIIAYYFDY (SEQ ID NO: 81);
DSXNMVRGIIIAYYFDY (SEQ ID NO: 82);
DSSXMVRGIIIAYYFDY (SEQ ID NO: 83);
DSSNXVRGIIIAYYFDY (SEQ ID NO: 84);
DSSNMXRGIIIAYYFDY (SEQ ID NO 85);
DSSNMVXGIIIAYYFDY (SEQ ID NO: 86);
DSSNMVRXIIIAYYFDY (SEQ ID NO: 87);
DSSNMVRGXIIAYYFDY (SEQ ID NO: 88);
DSSNMVRGIXIAYYFDY (SEQ ID NO: 89);
DSSNMVRGIIXAYYFDY (SEQ ID NO: 90);
DSSNMVRGIIIXYYFDY (SEQ ID NO: 91);
DSSNMVRGIIIAXYFDY (SEQ ID NO: 92);
DSSNMVRGIIIAYXFDY (SEQ ID NO: 93):
DSSNMVRGIIIAYYXDY (SEQ ID NO: 94);
DSSNMVRGIIIAYYFXY (SEQ ID NO: 95); and
DSSNMVRGIIIAYYFDX (SEQ ID NO: 96);
wherein X can be any amino acid residue which is different from the amino acid residue normally resident at that position, and wherein the resulting antibody binds selectively to an OPGbp.

The invention also provides for anti-OPGbp antibodies comprising variable light and variable heavy chains and further comprising a light chain CDR3 sequence which is increased from five amino acids to up to nine amino acids. More particularly, the light chain CDR3 sequence is selected from the group consisting of:
QHTXXXXRA (SEQ ID NO: 97)
wherein the first occurrence of X from left to right denotes any amino acid residue other than arginine, the second, third and fourth occurrences of X denote any amino acid residue, but preferably alanine, and wherein the resulting antibody binds selectively to an OPGbp. In another embodiment of the invention, a light chain CDR3 sequence is selected from the group consisting of:
QHTXAAARA (SEQ ID NO: 98)
wherein X is any amino acid residue other than arginine.

In another embodiment, the antibody variants of the invention comprise V₁ chains having a CDR1 sequence as in SEQ ID NO:1 and a CDR2 sequence as in SEQ ID NO:5, and comprise Vₕ chains having Vₕ chains having a CDR1 sequence as in SEQ ID NO:13 and a CDR2 sequence as in SEQ ID NO:16. In another embodiment, the antibody variants comprise a V₁ chain from "AT" antibody with the aforementioned light chain CDR3 variants and a Vₕ chain from "AT" antibody with the aforementioned heavy chain CDR3 variants.Variants may also be prepared by "chain shuffling" of either light or heavy chains (Marks et al. Biotechnology 10, 779-783 (1992)). Typically, a single light (or heavy) chain is combined with a library having a repertoire of heavy (or light) chains and the resulting population is screened for a desired activity, such as binding to OPGbp. This technique permits screening of a greater sample of different heavy (or light) chains in combination with a single light (or heavy) chain than is possible with libraries comprising repertoires of both heavy and light chains. The selective binding agents of the invention can be bispecific. Bispecific selective binding agents of this invention can be of several configurations. For example, bispecific antibodies resemble single antibodies (or antibody fragments) but have two different antigen binding sites (variable regions). Bispecific antibodies can be produced by chemical techniques (see *e*.*g*., Kranz et al., Proc. Natl. Acad. Sci. USA, 78:5807 (1981)), by "polydoma" techniques (see U.S. Pat. No. 4,474,893 to Reading) or by recombinant DNA techniques.

The selective binding agents of the invention may also be heteroantibodies. Heteroantibodies are two or more antibodies, or antibody binding fragments (Fab) linked together, each antibody or fragment having a different specificity.

The invention also relates to "humanized" antibodies. Methods for humanizing non-human antibodies are well known in the art. Generally, a humanized antibody has one or more amino acid residues introduced into a human antibody from a source which is non-human. In general, non-human residues will be present in CDRs. Humanization can be performed following methods known in the art (Jones et al., Nature 321, 522-525 (1986); Riechmann et al., Nature, 332, 323-327 (1988); Verhoeyen et al., Science 239, 1534-1536 (1988)), by substituting rodent complementarily-determining regions (CDRs) for the corresponding regions of a human antibody.

The selective binding agents of the invention, including chimeric, CDR-grafted, and humanized antibodies can be produced by recombinant methods known in the art. Nucleic acids encoding the antibodies are introduced into host cells and expressed using materials and procedures described herein and known in the art. In a preferred embodiment, the antibodies are produced in mammalian host cells, such as CHO cells. Fully human antibodies may be produced by expression of recombinant DNA transfected into host cells or by expression in hybridoma cells as described above.

Techniques for creating recombinant DNA versions of the antigen-binding regions of antibody molecules which bypass the generation of monoclonal antibodies are encompassed within the practice of this invention. To do so, antibody-specific messenger RNA molecules are extracted from immune system cells taken from an immunized animal, and transcribed into complementary DNA (cDNAs/. The cDNA is then cloned into a bacterial expression system. One example of such a technique suitable for the practice of this invention uses a bacteriophage lambda vector system having a leader sequence that causes the expressed Fab protein to migrate to the periplasmic space (between the bacterial cell membrane and the cell wall) or to be secreted. One can rapidly generate and screen great numbers of functional Fab fragments for those which bind the antigen. Such OPGbp selective binding agents (Fab fragments with specificity for an OPGbp polypeptide) are specifically encompassed within the term "antibody" as it is defined, discussed, and claimed herein.

Also within the scope of the invention are techniques developed for the production of chimeric antibodies by splicing the genes from a mouse antibody molecule of appropriate antigen-specificity together with genes from a human antibody molecule of appropriate biological activity, such as the ability to activate human complement and mediate ADCC. (Morrison et al., Proc. Natl. Acad. Sci., 81:6851 (1984); Neuberger et al., Nature, 312:604 (1984)). One example is the replacement of a Fc region with that of a different isotype. Selective binding agents such as antibodies produced by this technique are within the scope of the invention.

In a preferred embodiment of the invention, the anti-OPGbp antibodies are fully human antibodies. Thus encompassed by the invention are antibodies which bind OPGbp polypeptides and are encoded by nucleic acid sequences which are naturally occurring somatic variants of human germline immunoglobulin nucleic acid sequence, and fragments, synthetic variants, derivatives and fusions thereof. Such antibodies may be produced by any method known in the art. Exemplary methods include immunization with a OPGbp antigen (any OPGbp polypeptide capable of elicing an immune response, arid optionally conjugated to a carrier) of transgenic animals (*e.g.*. mice) that are capable of producing a repertoire of human antibodies in the absence of endogenous immunoglobulin production. See, for example, Jakobovits et al., Proc. Natl. Acad. Sci., 90, 2551-2555 (1993); Jakobovits et al., Nature, 362, 255-258 (1993) ; Bruggermann et al., Year in Immunol., 7, 33 (1993).

Alternatively, human antibodies may be generated through the in *vitro* screening of phage display antibody libraries. See Hoogenboom et al., J. Mol. Biol., 227. 381 (1991); Marks et al., J. Mol. Biol., 222, 581 (1991), incorporated herein by reference. Various antibody-containing phage display libraries have been described and may be readily prepared by one skilled in the art. Libraries may contain a diversity of human antibody sequences, such as human Fab, Fv, and scFv fragments, that may be screened against an appropriate target. Example 1 describes the screening of a Fab phage library against OPGbp to identify those molecules which selectively bind OPGbp. It will be appreciated that phage display libraries may comprise peptides or proteins other than antibodies which may be screened to identify selective binding agents of OPGbp.

An anti-idiotypic (anti-Id) antibody is an antibody which recognizes unique determinants generally associated with the antigen-binding site of an antibody. An Id antibody can be prepared by immunizing an animal of the same species and genetic type (e.g., mouse strain) as the source of the monoclonal antibody with the monoclonal antibody to which an anti-Id is being prepared. The immunized animal will recognize and respond to the idiotypic determinants of the immunizing antibody by producing an antibody to these idiotypic determinants (the anti-Id antibody). See, for example, U.S. Pat. No. 4,699,880, which is herein entirety incorporated by reference. The anti-Id antibody may also be used as an "immunogen" to induce an immune response in yet another animal, producing a so-called anti-anti-Id antibody. The anti-anti-Id may be epitopically identical to the original monoclonal antibody which induced the anti-Id. Thus, by using antibodies to the idiotypic determinants of a mAb, it is possible to identify other clones expressing antibodies of identical specificity.

### Production of selective binding agents of OPGbp

When the selective binding agent of OPGbp to be prepared is a proteinaceous selective binding agent, such as an antibody or an antigen binding domain, various biological or chemical methods for producing said agent are available.

Biological methods are preferable for producing sufficient quantities of a selective binding agent for therapeutic use. Standard recombinant DNA techniques are particularly useful for the production of antibodies and antigen binding domains of the invention. Exemplary expression vectors, host cells and methods for recovery of the expressed product are described below.

A nucleic acid molecule encoding an OPGbp antibody or antigen binding domain is inserted into an appropriate expression vector using standard ligation techniques. The vector is typically selected to be functional in the particular host cell employed (*i.e*., the vector is compatible with the host cell machinery such that amplification of the gene and/or expression of the gene can occur). A nucleic acid molecule encoding an anti-OPGbp antibody may be amplified/expressed in prokaryotic, yeast, insect (baculovirus systems) and/or eukaryotic host cells. Selection of the host cell will depend in part on whether an anti-OPGbp antibody is to be post-transitionally modified (e.g., glycosylated and/or phosphorylated). If so, yeast, insect, or mammalian host cells are preferable. For a review of expression vectors, see Meth. Enz. v. 185,-D.V. Goeddel, ed. Academic Press Inc., San Diego, CA (1990),

Typically, expression vectors used in any host cells will contain one or more of the following components: a promoter, one or more enhancer sequences, an origin of replication, a transcriptional termination sequence, a complete intron sequence containing a donor and acceptor splice site, a leader sequence for secretion, a ribosome binding site, a polyadenylation sequence, a polylinker region for inserting the nucleic acid encoding the polypeptide to be expressed, and a selectable marker element. Each of these sequences is discussed in more detail below.

The vector components may be homologous (i.e., from the same species and/or strain as the host cell), heterologous (i.e., from a species other than the host cell species or strain), hybrid (i.e., a combination of different sequences from more than one source), synthetic, or native sequences which normally function to regulate immunoglobulin expression. As such, a source of vector components may be any prokaryotic or eukaryotic organism, any vertebrate or invertebrate organism, or any plant, provided that the components are functional in, and can be activated by, the host cell machinery.

An origin of replication is selected based upon the type of host cell being used for expression. For example, the origin of replication from the plasmid pBR322 (Product No. 303-3s, New England Biolabs, Beverly, MA) is suitable for most Gram-negative bacteria while various origins from SV40, polyoma, adenovirus, vesicular stomatitus virus (VSV) or papillomaviruses (such as HPV or BPV) are useful for cloning vectors in mammalian cells. Generally, the origin of replication component is not needed for mammalian expression vectors (for example, the SV40 origin is often used only because it contains the early promoter).

A transcription termination sequence is typically located 3' of the end of a polypeptide coding regions and serves to terminate transcription. Usually, a transcription termination sequence in prokaryotic cells is a G-C rich fragment followed by a poly T sequence. While the sequence is easily cloned from a library or even purchased commercially as part of a vector, it can also be readily synthesized using methods for nucleic acid synthesis such as those described above.

A selectable marker gene element encodes a protein necessary for the survival and growth of a host cell grown in a selective culture medium. Typical selection marker genes encode proteins that (a) confer resistance to antibiotics or other toxins, *e.g*., ampicillin, tetracycline, or kanamycin for prokaryotic host cells, (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells.

Other selection genes may be used to amplify the gene which will be expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure which only the transformants are uniquely adapted to survive by virtue of the marker present in the vector. Selection pressure is imposed by culturing the transformed cells under conditions in which the concentration of selection agent in the medium is successively changed, thereby leading to amplification of both the selection gene and the DNA that encodes an anti-OPGbp antibody. As a result, increased quantities of an antibody are synthesized from the amplified DNA.

A ribosome binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of the polypeptide to be expressed. The Shine-Dalgarno sequence is varied but is typically a polypurine (i.e., having a high A-G content). Many Shine-Dalgarno sequences have been identified, each of which can be readily synthesized using methods set forth above and used in a prokaryotic vector.

A leader, or signal, sequence is used to direct secretion of a polypeptide. A signal sequence may be positioned within or directly at the 5' end of a polypeptide coding region. Many signal sequences have been identified and may be selected based upon the host cell used for expression. In the present invention, a signal sequence may be homologous (naturally occurring) or heterologous to a nucleic acid sequence encoding an anti-OPGbp antibody or antigen binding domain. A heterologous signal sequence selected should be one that is recognized and processed, i.e., cleaved, by a signal peptidase, by the host cell. For prokaryotic host cells that do not recognize and process a native immunoglobulin signal sequence, the signal sequence is substituted by a prokaryotic signal sequence selected, for example, from the group of the alkaline phosphatase, penicillinase, or heat-stable enterotoxin II leaders. For yeast secretion, a native immunoglobulin signal sequence may be substituted by the yeast invertase, alpha factor, or acid phosphatase leaders. In mammalian cell expression the native signal sequence is satisfactory, although other mammalian signal sequences may be suitable.

In most cases, secretion of an anti-OPGbp antibody or antigen binding domain from a host cell will result in the removal of the signal peptide from the antibody. Thus the mature antibody will lack any leader or signal sequence.

In some cases, such as where glycosylation is desired in a eukaryotic host cell expression system, one may manipulate the various presequences to improve glycosylation or yield. For example, one may alter the peptidase cleavage site of a particular signal peptide, or add prosequences, which also may affect glycosylation. The final protein product may have, in the -1 position (relative to the first amino acid of the mature protein) one or more additional amino acids incident to expression, which may not have been totally removed. For example, the final protein product may have one or two amino acid found in the peptidase cleavage site, attached to the N-terminus. Alternatively, use of some enzyme cleavage sites may result in a slightly truncated form of the desired OPGbp polypeptide, if the enzyme cuts at such area within the mature polypeptide.

The expression vectors of the present invention will typically contain a promoter that is recognized by the host organism and operably linked to a nucleic acid molecule encoding an anti-OPGbp antibody or antigen binding domain. Either a native or heterologous promoter may be used depending the host cell used for expression and the yield of protein desired.

Promoters suitable for use with prokaryotic hosts include the beta-lactamase and lactose promoter systems; alkaline phosphatase, a tryptophan (trp) promoter system; and hybrid promoters such as the tac promoter. Other known bacterial promoters are also suitable. Their sequences have been published, thereby enabling one skilled in the art to ligate them to the desired DNA sequence(s), using linkers or adapters as needed to supply any required restriction sites.

Suitable promoters for use with yeast hosts are also well known in the art. Yeast enhancers are advantageously used with yeast promoters. Suitable promoters for use with mammalian host cells are well known and include those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40). Other suitable mammalian promoters include heterologous mammalian promoters, e.g., heat-shock promoters and the actin promoter.

Additional promoters which may be used for expressing the selective binding agents of the invention include, but are not limited to: the SV40 early promoter region (Bernoist and Chambon, Nature, 290:304-310, 1981); the CMV promoter; the promoter contained in the 3' long terminal repeat of Rous sarcoma virus (Yamamoto, et al., Cell, 22; 787-797, 1980); the herpes thymidine kinase promoter (Wagner et al., Proc. Natl. Acad. Sci. U.S.A., 78; 144-1445, 1981); the regulatory sequences of the metallothionine gene (Brinster et al., Nature, 296; 39-42, 1982); prokaryotic expression vectors such as the beta - lactamase promoter (Villa-Kamaroff, et al., Proc. Natl. Acad. Sci. U.S.A., 75; 3727-3731, 1978); or the tac promoter (DeBoer, et al., Proc. Natl. Acad. Sci. U.S.A., 80; 21-25, 1983). Also of interest are the following animal transcriptional control regions, which exhibit tissue specificity and have been utilized in transgenic animals: the elastase I gene control region which is active in pancreatic acinar cells (Swift et al., Cell, 38; : 639-646, 1984; Ornitz et al., Cold Spring Harbor Symp. Quant. Biol. 50; 399-409, 1986; MacDonald, Hepatology, 7; :425-515, 1987); the insulin gene control region which is active in pancreatic beta cells (Hanahan, Nature, 315; 115-122, 1985); the immunoglobulin gene control region which is active in lymphoid cells (Grosschedl et al., Cell, 38; 647-658, 1984; Adames et al., Nature, 318; 533-538, 1985; Alexander et al., Mol. Cell. Biol., 7; 1436-1444, 1987); the mouse mammary tumor virus control region which is active in testicular, breast, lymphoid and mast cells (Leder et al.. Cell, 45; 485-495, 1986), albumin gene control region which is active in liver (Pinkert et al., Genes and Devel., 1; 268-276, 1987); the alphafetoprotein gene control region which is active in liver (Krumlauf et al., Mol. Cell. Biol., 5; 1639-1648, 1985; Hammer et al., Science, 215; 53-58, 1987); the alpha 1-antitrypsin gene control region which is active in the liver (Kelsey et al., Genes and Devel., 1; 161-171, 1987); the beta-globin gene control region which is active in myeloid cells (Mogram et al., Nature, 315; 338-340, 1985; Kollias et al., Cell, 46; 89-94, 1986); the myelin basic protein gene control region which is active in oligodendrocyte cells in the brain (Readhead et al., Cell, 48; 703-712, 1987); the myosin light chain-2 gene control region which is active in skeletal muscle (Sani, Nature, 314; 283-286, 1985); and the gonadotropic releasing hormone gene control region which is active in the hypothalamus (Mason et al., Science, 23; 1372-1378, 1986).

An enhancer sequence may be inserted into the vector to increase transcription in eucaryotic host cells. Several enhancer sequences available from mammalian genes are known (e.g., globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to the polypeptide coding region, it is typically located at a site 5' from the promoter.

vectors for practicing this invention may be those which are compatible with bacterial, insect, and mammalian host cells. Such vectors include, inter alia, pCRII, pCR3, and pcDNA3.1 (Invitrogen Company, San Diego, CA), pBSII (Stratagene Company, La Jolla, CA), pET15 (Novagen, Madison, WI), pGEX (Pharmacia Biotech, Piscataway, NJ), pEGFP-N2 (Clontech, Palo Alto, CA), pETL (BlueBacII; Invitrogen), pDSR-alpha (PCT Publication No. WO90/14363) and pFastBacDual (Gibco/BRL, Grand Island, NY).

Additional possible vectors include, but are not limited to, cosmids, plasmids or modified viruses, but the vector system must be compatible with the selected host cell. Such vectors include, but are not limited to plasmids such as Bluescript plasmid derivatives (a high copy number ColEl-based phagemid, Stratagene Cloning Systems Inc., La Jolla CA), PCR cloning plasmids designed for cloning Taq-amplified PCR products (e.g., TOPO™ TA Cloning^{®} Kit, PCR2.1^{®} plasmid derivatives, Invitrogen, Carlsbad, CA), and mammalian, yeast or virus vectors such as a baculovirus expression system (pBacPAK plasmid derivatives, Clontech, Palo Alto, CA). The recombinant molecules can be introduced into host cells via transformation, transfection, infection, electroporation, or other known techniques.

Host cells may be prokaryotic host cells (such as *E. coli*) or eukaryotic host cells (such as a yeast cell, an insect cell, or a vertebrate cell). The host cell, when cultured under appropriate conditions, expresses an antibody or antigen binding domain of the invention which can subsequently be collected from the culture medium (if the host cell secretes it into the medium) or directly from the host cell producing it (if it is not secreted). Selection of an appropriate host cell will depend upon various factors, such as desired expression levels, polypeptide modifications that are desirable or necessary for activity, such as glycosylation or phosphorylation, and ease of folding into a biologically active molecule.

A number of suitable host cells are known in the art and many are available from the American Type Culture Collection (ATCC), Manassas, VA. Examples include mammalian cells, such as Chinese hamster ovary cells (CHO) (ATCC No. CCL61) CHO DHFR- cells (Urlaub et al. Proc. Natl. Acad. Sci. USA 97, 4216-4220 (1980)), human embryonic kidney (HEK) 293 or 293T cells (ATCC No. CRL1573), or 3T3 cells (ATCC No. CCL92). The selection of suitable mammalian host cells and methods for transformation, culture, amplification, screening and product production and purification are known in the art. Other suitable mammalian cell lines, are the monkey COS-1 (ATCC No. CRL1650) and COS-7 cell lines (ATCC No. CRL1651), and the CV-1 cell line (ATCC No. CCL70). Further exemplary mammalian host cells include primate cell lines and rodent cell lines, including transformed cell lines. Normal diploid cells, cell strains derived from *in vitro* culture of primary tissue, as well as primary explants, are also suitable. Candidate cells may be genotypically deficient in the selection gene, or may contain a dominantly acting selection gene. Other suitable mammalian cell lines include but are not limited to, mouse neuroblastoma N2A cells, HeLa, mouse L-929 cells, 3T3 lines derived from Swiss, Balb-c or NIH mice, BHK or HaK hamster cell lines, which are available from the American Type Culture Collection, Manassas, VA). Each of these cell lines is known by and available to those skilled in the art of protein expression.

-Similarly useful as host cells suitable for the present invention are bacterial cells. For example, the various strains of *E. coli* (e.g., HB101, (ATCC No. 33694) DH5α, DH10, and MC1061 (ATCC No.. 53338)) are well-known as host cells in the field of biotechnology. Various strains of B. subtilis, Pseudomonas spp., other Bacillus spp., Streptomyces spp., and the like may also be employed in this method.

Many strains of yeast cells known to those skilled in the art are also available as host cells for expression of the polypeptides of the present invention. Preferred yeast cells include, for example, Saccharomyces cerivisae.

Additionally, where desired, insect cell system may be utilized in the methods of the present invention. Such systems are described for example in Kitts et al. (Biotechniques, 14, 810-817 (1993)), Lucklow (Curr. Opin. Biotechnol., A, 564-572 (1993) and Lucklow et al. (J. Virol., 67, 4566-4579 (1993)). Preferred insect cells are Sf-9 and Hi5 (Invitrogen, Carlsbad, CA).

Transformation or transfection of a nucleic acid molecule encoding an anti-OPGbp antibody or antigen binding domain into a selected host cell may be accomplished by well known methods including methods such as calcium chloride, electroporation, microinjection, lipofection or the DEAE-dextran method. The method selected will in part be a function of the type of host cell to be used. These methods and other suitable methods are well known to the skilled artisan, and are set forth, for example, in Sambrook et al., supra.

One may also use transgenic animals to express glycosylated selective binding agents, such as antibodies and antigen binding domain. For example, one may use a transgenic milk-producing animal (a cow or goat, for example) and obtain glycosylated binding agents in the animal milk. Alternatively, one may use plants to produce glycosylated selective binding agents.

Host cells comprising (*i*.*e*., transformed or transfected) an expression vector encoding a selective binding agent of OPGbp may be cultured using standard media well known to the skilled artisan. The media will usually contain all nutrients necessary for the growth and survival of the cells. Suitable media for culturing *E. coli* cells are for example, Luria Broth (LB) and/or Terrific Broth (TB). Suitable media for culturing eukaryotic cells are RPMI 1640, MEM, DMEM, all of which may be supplemented with serum and/or growth factors as required by the particular cell line being cultured. A suitable medium for insect cultures is Grace's medium supplemented with yeastolate, lactalbumin hydrolysate, and/or fetal calf serum as necessary.

Typically, an antibiotic or other compound useful for selective growth of transfected or transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin. Other compounds for selective growth include ampicillin, tetracycline and neomycin The amount of an anti-OPGbp antibody or antigen binding domain produced by a host cell can be evaluated using standard methods known in the art. Such methods include, without limitation, Western blot analysis, SDS-polyacrylamide gel electrophoresis, non-denaturing gel electrophoresis, HPLC separation, immunoprecipitation, and/or activity assays.

Purification of an anti-OPG antibody or antigen binding domain which has been secreted into the cell media can be accomplished using a variety of techniques including affinity, immunoaffinity or ion exchange chromatography, molecular sieve chromatography, preparative gel electrophoresis or isoelectric focusing, chromatofocusing, and high pressure liquid chromatography. For example, antibodies comprising a Fc region may be conveniently purified by affinity chromatography with protein A, which selectively binds the Fc region. Modified forms of an antibody or antigen binding domain may be prepared with affinity tags, such as hexahistidine or other small peptide such as FLAG (Eastman Kodak Co., New Haven, CT) or myc (Invitrogen) at either its carboxyl or amino terminus and purified by a one-step , affinity column. For example, polyhistidine binds with great affinity and specificity to nickel, thus an affinity column of nickel (such as the Qiagen® nickel columns) can be used for purification of polyhistidine-tagged selective binding agents. (See for example, Ausubel et al., eds., Current Protocols in Molecular Biology, Section 10.11.8, John Wiley & Sons, New York (1993)). In some instances, more than one purification step may be required.

Selective binding agents of the invention which are expressed in procaryotic host cells may be present in soluble form either in the periplasmic space or in the cytoplasm or in an insoluble form as part of intracellular inclusion bodies. Selective binding agents can be extracted from the host cell using any standard technique known to the skilled artisan. For example, the host cells can be lysed to release the contents of the periplasm/cytoplasm by French press, homogenization, and/or sonication followed by centrifugation.

Soluble forms of an anti-OPGbp antibody or antigen binding domain present either in the cytoplasm or released from the periplasmic space may be further purified using methods known in the art, for example Fab fragments are released from the bacterial periplasmic space by osmotic shock techniques.

If an antibody or antigen binding domain has formed inclusion bodies, they can often bind to the inner and/or outer cellular membranes and thus will be found primarily in the pellet material after centrifugation. The pellet material can then be treated at pH extremes or with chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The soluble selective binding agent can then be analyzed using gel electrophoresis, immunoprecipitation or the like. If it is desired to isolate a solublized. antibody or antigen binding domain, isolation may be accomplished using standard methods such as those set forth below and in Marston et al. (Meth. Enz., 182:264-275 (1990)).

In some cases, an antibody or antigen binding domain may not be biologically active upon isolation. Various methods for "refolding" or converting the polypeptide to its tertiary structure and generating disulfide linkages, can be used to restore biological activity. Such methods include exposing the solubilized polypeptide to a pH usually above 7 and in the presence of a particular concentration of a chaotrope. The selection of chaotrope is very similar to the choices used for inclusion body solubilization, but usually the chaotrope is used at a lower concentration and is not necessarily the same as chaotropes used for the solubilization. In most cases the refolding/oxidation solution will also contain a reducing agent or the reducing agent plus its oxidized form in a specific ratio to generate a particular redox potential allowing for disulfide shuffling to occur in the formation of the protein's cysteine bridge(s). Some of the commonly used redox couples include cysteine/cystamine, glutathione (GSH)/dithiobis GSH, cupric chloride, dithiothreitol (DTT)/dithiane DTT, and 2-mercaptoethanol(bME)/dithio-b(ME). In many instances, a cosolvent may be used or may be needed to increase the efficiency of the refolding and the more common reagents used for this purpose include glycerol, polyethylene glycol of various molecular weights, arginine and the like.

Antibodies and antigen binding domains of the invention may also be prepared by chemical synthesis methods (such as solid phase peptide synthesis) using techniques known in the art such as those set forth by Merrifield et al., (J. Am. Chem. Soc., 85:2149 [1963]), Houghten et al. (Proc Natl Acad. Sci. USA, 82:5132 [1985]), and Stewart and Young (Solid Phase Peptide Synthesis, Pierce Chemical Co., Rockford, IL [1984]). Such polypeptides may be synthesized with or without a methionine on the amino terminus. Chemically synthesized antibodies and antigen binding domains may be oxidized using methods set forth in these references to form disulfide bridges. Antibodies so prepared will retain at least one biological activity associated with a native or recombinantly produced anti-OPGbp antibody or antigen binding domain.

### Assays for selective binding agents of OPGbp

Screening methods for identifying selective binding agents which partially or completely inhibits at least one biological activity of OPGbp are provided by the invention. Inhibiting the biological activity of OPGbp includes, but is not limited to, inhibiting binding of OPGbp to its cognate receptor, ODAR, inhibiting stimulation of osteoclast formation in vitro or in vivo by OPGbp, and/or inhibiting bone turnover or bone resorption mediated by OPGbp. Selective binding agents of the invention include anti-OPGbp antibodies, and fragments, variants, derivatives and fusion thereof, peptides, peptidomimetic compounds or organo-mimetic compounds.

Screening methods for identifying selective binding agents which can partially or completely inhibit a biological activity of OPGbp can include in vitro or in vivo assays. In vitro assays include those that detect binding of OPGbp to ODAR and may be used to screen selective binding agents of OPGbp for their ability to increase or decrease the rate or extent of OPGbp binding to ODAR. In one type of assay, an OPGbp polypeptide, preferably a soluble form of OPGbp such as an extracellular domain, is immobilized on a solid support (e.g., agarose or acrylic beads) and an ODAR polypetpide is the added either in the presence or absence of a selective binding agent of OPGbp. The extent of binding of OPGbp and ODAR with or without a selective binding agent present is measured. Binding can be detected by for example radioactive labeling, fluorescent labeling or enzymatic reaction. Alternatively, the binding reaction may be carried out using a surface plasmon resonance detector system such as the BIAcore assay system (Pharmacia, Piscataway, NJ). Binding reactions may be carried out according to the manufacturer's protocol.

In vitro assays such as those described above may be used advantageously to screen rapidly large numbers of selective binding agents for effects on binding of OPGbp to ODAR. The assays may be automated to screen compounds generated in phage display, synthetic peptide and chemical synthesis libraries.

Selective binding agents increase or decrease binding of OPGbp to ODAR may also be screened in cell culture using cells and cell lines expressing either polypeptide. Cells and cell lines may be obtained from any mammal, but preferably will be from human or other primate, canine, or rodent sources. As an example, the binding of OPGbp to cells expressing ODAR on the surface is evaluated in the presence or absence of selective binding agents and the extent of binding may be determined by, for example, flow cytometry using a biotinylated antibody to OPGbp.

In vitro activity assays may also be used to identify selective binding agents which inhibit OPGbp activity. Examples of assays include stimulation of cell growth and proliferation which are dependent on OPGbp and OPGbp mediated osteoclast formation from bone marrow cells, the latter of which is described in Example 1 of the present application.

In vivo assays are also available to determine whether a selective binding agent is capable of decreasing or inhibiting bone turnover and/or bone resorption. Bone resorption can be increased in animals by a variety of methods, including ovariectomy and administration of pro-resorptive agents such as OPGbp or IL-1. See WO 97/23614 and WO 98/46751. The effects of OPG inhibitors on bone resorption in human patients may be measured by a variety of known methods such as single photon absorptiometry (SPA), dual photon absorptiometry (DPA), dual energy X-ray absorptiometry (DEXA), quantitative computed tomography (QCT), and ultrasonography (See Johnston et al. in Primer on the Metabolic Bone Disease and Disorders of Mineral Metabolism, 2nd ed., M.J. Favus, ed. Raven Press pp. 137-146). Bone turnover and resorption may also be determined by measuring changes in certain biochemical markers, such as serum osteocalcin, serum alkaline phosphatase, serum procollagen I extension peptides, urinary or serum C-terminal or N-terminal telopeptide of collagen, urinary calcium, hydroxyproline and urinary pyridinoline and deoxypyridinoline. It is generally recognized that a decrease in the levels of the aforementioned biochemical markers indicates that bone resorption is decreased and loss of bone mass is being reduced. Alternatively, effects on bone resorption may also be determined by measuring a change in the mechanical strength of bone, in particular an increase in torsional (twisting) strength of bone.

For diagnostic applications, in certain embodiments, selective binding agents of OPGbp, such as antibodies and antigen binding domains thereof, typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin; or an enzyme, such as alkaline phosphatase, *β-*galactosidase or horseradish peroxidase. Bayer et al., Meth. Enz., 184: 138-163 (1990).

The selective binding agents of the invention may be employed in any known assay method, such as radioimmunoassays, competitive binding assays, direct and indirect sandwich assays (ELISAs), and immunoprecipitation assays (Sola, Monoclonal Antibodies: A Manual of Techniques, pp. 147-158 (CRC Press, 1987)) for detection and quantitation of OPGbp polypeptides. The antibodies will bind OPGbp polypeptides with an affinity which is appropriate for the assay method being employed

The selective binding agents of the invention also are useful for *in vivo* imaging, wherein for example a selective binding agent labeled with a detectable moiety is administered to an animal, preferably into the bloodstream, and the presence and location of the labeled antibody in the host is assayed. The agent may be labeled with any moiety that is detectable in an animal, whether by nuclear magnetic resonance, radiology, or other detection means known in the art.

The invention also relates to a kit comprising a selective binding agent of OPGbp, such as an antibody or antigen binding domain, and other reagents useful for detecting OPGbp levels in biological samples. Such reagents may include a secondary activity, a detectable label, blocking serum, positive and negative control samples, and detection reagents.

Therapeutic Users of OPGbp selective binding agents, Monoclonal antibodies and antigen binding domains as of claim 1 may be used as therapeutics. Therapeutic selective binding agents may be OPGbp agonists or antagonists and, in one embodiment, are anti-OPGbp antagonist antibodies which inhibit at least one of the biological activities of a OPGbp polypeptide in vitro or in vivo. For example, an antagonist of OPGbp will inhibit the binding of OPGbp to ODAR by at least about 100-fold, or about 1000-fold, or greater than 1000-fold. Alternatively, an OPGbp antagonist will inhibit osteoclast formation in vitro as indicated by a measurable IC50 (a concentration giving 50% inhibition) in a bone marrow assay such as that described in Example 1. Alternatively, an OPGbp antagonist will decrease bone turnover markers by at least 20%, or at lease 50% compared to baseline levels. Antagonist OPGbp selective binding agents (such as antibodies) are identified by screening assays described herein.

OPGbp antagonists, such as anti-OPGbp antagonist antibodies and antigen binding domains, may be used to prevent or treat bone diseases characterized by loss of bone mass or by replacement of structurally normal bone with structurally abnormal bone. OPGbp antagonists may be administered to an animal having loss of bone mass or susceptible to having loss of bone mass resulting from any of the following disorders: Osteoporosis, such as primary osteoporosis, endocrine osteoporosis (hyperthyroidism, hyperparathryoidism, Cushing's syndrome, and acromegaly), hereditary and congenital forms of osteoporosis (osteogenesis imperfecta, homocystinuria, Menkes' syndrome, and Riley-Day syndrome) and osteoporosis due to immobilization of extremities; Osteomyelitis, or an infectious lesion in bone, leading to loss of bone mass; Hypercalcemia resulting from solid tumors (breast, lung and kidney) and hematologic malignacies (multiple myeloma, lymphoma and leukemia), idiopathic hypercalcemia, and hypercalcemia associated with hyperthryoidism and renal function disorders; Osteopenia following surgery, induced by steroid administration, and associated with disorders of the small and large intestine and with chronic hepatic and renal diseases; Osteonecrosis, or bone cell death, associated with traumatic injury or nontraumatic necrosis associated with Gaucher's disease, sickle cell anemia, systemic lupus erythematosus and other conditions; Loss of bone mass due to rheumatoid arthritis; Periodontal loss of bone mass; Osteoarthritis; Prosthetic loosening; and Osteolytic metastasis. OPGbp antagonists may also be used to prevent or treat certain bone disorders are characterized by the replacement of structurally sound bone with disorganized structurally incompetent bone, such as Paget's disease of bone (osteitis deformans) in adults and juveniles; hyperparathryoidism, in congenital bone disorders such as fibrous dysplasia, and in osteosclerotic bone metastases.

OPGbp antagonists are advantageously used to treat loss of bone mass resulting from osteolytic destruction of bone caused by malignant or metastatic tumors. OPG polypeptides of the invention may be used to treat loss of bone mass associated with breast, prostate, thyroid, kidney, lung, esophogeal, rectal, bladder, cervical, ovarian and liver cancers as well as cancer of the gastrointestional tract. Also included is loss of bone mass associated with certain hematological malignancies such as multiple myeloma and lymphomas such as Hodgkin's Disease.

OPGbp antagonists of the invention, including antagonist antibodies and antigen binding domains, are administered alone or in combination with other therapeutic agents, in particular, in combination with other cancer therapy agents. Such agents generally include radiation therapy or chemotherapy. Chemotherapy may involve treatment with one or more of the following: anthracyclines, taxol, tamoxifene, doxorubicin, 5-fluorouracil, and other drugs known to the skilled worker. In one embodiment, the cancer therapy agent is a luteinizing hormone-releasing hormone (LHRH) antagonist, preferably a peptide antagonist. More preferably, an LHRH antagonist is a decapeptide comprising the following structure:
A-B-C-D-E-F-G-H-I-J
wherein
A is pyro-glu, Ac-D-Nal, Ac-D-Qal; Ac-Sar, or Ac-D-Pal;
B is His or 4-Cl-D-Phe;
C is Trp, D-Pal, D-Nal, L-Nal-D-Pal(N-O), or D-Trp;
D is Ser;
E is N-Me-Ala, Tyr, N-Me-Tyr, Ser, Lys(iPr), 4-Cl-Phe, His, Asn, Met, Ala, Arg or Ile;
F is 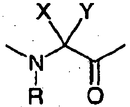 wherein R and X are independently, H and alkyl;
and Y comprises a small polar entity.
G is Leu or Trp;
H is Lys(iPr), Gln, Met, or Arg;
I is Pro; and
J is Gly-NH2 or D-Ala-NH2;
or a pharmaceutically acceptable salt thereof.

In another embodiment, an LHRH antagonist comprises the peptide:
N-Ac-D-Nal-4-Cl-Phe-D-Pal-Ser-N-Me-Tyr-D-Asn-Leu-Lys(iPr)-Pro-D-Ala-NH2.

Standard abbreviations and conventions are used herein and the following non-standard residues and moieties are abbreviated as follows:

| | |
|---|---|
| Nal | 3-(2-napthyl)alaninyl |
| 4-Cl-Phe | (4'-chlorophenyl)alaninyl |
| Pal | 3-(3'-pyridyl)alaninyl |
| Pal(N-O) | 3-(3'-pyridine-N-oxide) alaninyl |
| iPr-Lys | N-epsilon-2-propyl-lysinyl |
| Qal | 3-(2'-quinolinyl)alaninyl |

Alternative forms of LHRH antagonists decapeptides are also encompassed by the invention. Such decapeptides are described in U.S. Patent No. 5,843,901 hereby incorporated by reference.

Also included are combinations of OPGbp antagonists with antibodies which bind to tumor cells and induce a cytotoxic and/or cytostatic effect on tumor growth. Examples of such antibodies include those which bind to cell surface proteins Her2, CDC20, CDC33, mucin-like glycoprotein and epidermal growth factor receptor (EGFR) present on tumor cells and induce a cytostatic and/or cytotoxic effect on tumor cells displaying these proteins. Examples of such antibodies include HERCEPTIN for treatment of breast cancer and RITUXAN for the treatment of non-Hodgkin's lymphoma. Also included as cancer therapy agents are polypeptides which selectively induce apoptosis in tumor cells, such as the TNF-related polypeptide TRAIL. OPGbp antagonists may be administered prior to, concurrent with, or subsequent to treatment with a cancer therapy agent. OPGbp antagonists may be administered prophylactically to prevent or mitigate the onset of loss of bone mass by metastatic cancer or may be given for the treatment of an existing condition of loss of bone mass due to metastasis.

OPGbp antagonists of the invention may be used to prevent and/or treat the growth of tumor cells in bone. Cancer which metastasizes to bone can spread readily as tumor cells stimulate osteoclasts to resorb the internal bone matrix. Treatment with an OPGbp antagonist will maintain bone density by inhibiting resorption and decrease the likelihood of tumor cells spreading throughout the skeleton. Any cancer which metastasizes to bone may be prevented and/or treated with an OPGbp antagonist.

In one embodiment, multiple myeloma may be prevented and/or treated with an OPGbp antagonist, such as an antibody. Multiple myeloma is localized to bone and affected patients typically exhibit a loss of bone mass due to increased osteoclast activation in localized regions. Myeloma cells either directly or indirectly produce OPGbp, which in turn activates osteoclasts resulting in local bone lysis surrounding the myeloma cells embedded in bone marrow spaces. The normal osteoclasts adjacent to the myeloma cell in turn produce IL-6, leading to growth and proliferation of myeloma cells. Myeloma cells expand in a clonal fashion and occupy bone spaces that are being created by inappropriate bone resorption. Treatment of an animal with an OPGbp antagonist blocks activation of osteoclasts which in turn leads to a decrease in IL-6 production by osteoclasts, and a suppression of mycloma all growth and/or proliferation.

OPGbp antagonists may be used alone for the treatment of the above referenced conditions resulting in loss of bone mass or in combination with a therapeutically effective amount of a bone growth promoting (anabolic) agent or a bone anti-resorptive agent including bone morphogenic factors designated BMP-1 to BMP-12, transforming growth factor-β and TGF-β family members, fibroblast growth factors FGF-1 to FGF-10, interleukin-1 inhibitors, TNFα inhibitors, parathyroid hormone, E series prostaglandins, bisphosphonates and bone-enhancing minerals such as fluoride and calcium. Anabolic agents include parathyroid hormone and insulin-like growth factor (IGF), wherein the latter agent is preferably complexed with an IGF binding protein. Preferred embodiments also include the combination of an OPGbp antagonist with a interluekin-1 (IL-1) receptor antagonist or an OPGbp antagonist with a soluble TNF receptor, such as soluble TNF receptor-1 or soluble TNF receptor-2. An exemplary IL-1 receptor antagonist is described in WO89/1154 and an exemplary soluble TNF receptor-1 is described in WO98/01555.

A decrease in the rate of bone resorption can lead to osteopetrosis, a condition marked by excessive bone density. Agonists of OPGbp may increase osteoclast formation and bone resorption and be administered to an animal which has or is susceptible to decreased bone resorption and an abnormal increase in bone mass.

### Pharmaceutical Compositions

Pharmaceutical compositions of OPGbp selective binding agents are within the scope of the present invention. Such compositions comprise a therapeutically or prophylactically effective amount of an OPGbp selective binding agent i.e an antibody, or a fragment, variant, derivative or fusion thereof, in admixture with a pharmaceutically acceptable agent. In a preferred embodiment, pharmaceutical compositions comprise anti-OPGbp antagonist antibodies which inhibit partially or completely at least one biological activity of OPGbp in admixture with a pharmaceutically acceptable agent. Typically, the antibodies will be sufficiently purified for administration to an animal.

Pharmaceutically acceptable agents for use in the compositions of the invention include carriers, excipients, diluents, antioxidants, preservatives, coloring, flavoring and diluting agents, emulsifying agents, suspending agents, solvents, fillers, bulking agents, buffers, delivery vehicles, tonicity agents, cosolvents, wetting agents, complexing agents, buffering agents, antimicrobials and surfactants, as are well known in the art.

Neutral buffered saline or saline mixed with serum albumin are exemplary appropriate carriers. Also included in the compositions are antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, pluronics or polyethylene glycol. Also by way of example, suitable tonicity enhancing agents include alkali metal halides (preferably sodium or potassium chloride), mannitol, sorbitol and the like. Suitable preservatives include, but are not limited to, benzalkonium chloride, thimerosal, phenethyl alcohol, methylparaben, propylparaben, chlorhexidine, sorbic acid and the like. Hydrogen peroxide may also be used as preservative. Suitable cosolvents are for example glycerin, propylene glycol, and polyethylene glycol. Suitable complexing agents are for example caffeine, polyvinylpyrrolidone, beta-cyclodextrin or hydroxy-propyl--beta-cyclodextrin. Suitable surfactants or wetting agents include sorbitan esters, polysorbates such as polysorbate 80, tromethamine, lecithin, cholesterol, tyloxapal and the like. The buffers can be conventional buffers such as acetate, borate, citrate, phosphate, bicarbonate, or Tris-HC1. Acetate buffer may be around pH 4.0-5.5 and Tris buffer may be around pH 7.0-8.5. Additional pharmaceutical agents are set forth in Remington's Pharmaceutical Sciences, 18th Edition, A.R. Gennaro, ed., Mack Publishing Company 1990.

The compositions may be in liquid form or in a lyophilized or freeze-dried form. Lypophilized forms may include excipients such as sucrose.

The compositions of the invention are suitable for parenteral administration. In preferred embodiments, the compositions are suitable for injection or infusion into an animal by any route available to the skilled worker, such as subcutaneous, intravenous, intramuscular, intraperitoneal, intracerebral (intraparenchymal), intracerebroventricular, intramuscular, intraocular, intraarterial, or intralesional routes. A parenteral formulation will typically be a sterile, pyrogen-free, isotonic aqueous solution, optionally containing pharmaceutically acceptable preservatives.

The optimal pharmaceutical formulation may be readily determined by one skilled in the art depending upon the intended route of administration, delivery format and desired dosage.

Other formulations are also contemplated by the invention. The pharmaceutical compositions also may include particulate preparations of polymeric compounds such as polylactic acid, polyglycolic acid, etc. or the introduction of an OPGbp selective binding agent (such as an antibody) into liposomes. Hyaluronic acid may also be used, and this may have the effect of promoting sustained duration in the circulation. Pharmaceutical compositions also include the formulation of OPGbp selective binding agents (such as antibodies) with an agent, such as injectable microspheres, bio-erodible particles or beads, or liposomes, that provides for the controlled or sustained release of a selective binding agent which may then be delivered as a depot injection. Other suitable means for delivery include implantable delivery devices.

A pharmaceutical composition comprising and OPGbp selective binding agent (such as an antibody) may be formulated as a dry powder for inhalation. Such inhalation solutions may also be formulated in a liquefied propellant for aerosol delivery. In yet another formulation, solutions may be nebulized.

It is also contemplated that certain formulations containing OPGbp selective binding agents may be administered orally. Formulations administered in this fashion may be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents may be included to facilitate absorption of a selective binding agent. Diluents, flavorings, low melting point waxes, vegetable oils, lubricants, suspending agents, tablet disintegrating agents, and binders may also be employed.

Another preparation may involve an effective quantity of an OPGbp selective binding agent in a mixture with non-toxic excipients which are suitable for the manufacture of tablets. By dissolving the tablets in sterile water, or another appropriate vehicle, solutions can be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional formulations will be evident to those skilled in the art, including formulations involving OPGbp selective binding agents in combination with one or more other therapeutic agents. Techniques for formulating a variety of other sustained- or controlled-delivery means, such as liposome carriers, bio-erodible microparticles or porous beads and depot injections, are also known to those skilled in the art. See, for example, the Supersaxo *et al.* description of controlled release porous polymeric microparticles for the delivery of pharmaceutical compositions (See WO 93/15722 (PCT/US93/00829).

Regardless of the manner of administration, the specific dose may be calculated according to body weight, body surface area or organ size. Further refinement of the calculations necessary to determine the appropriate dosage for treatment involving each of the above mentioned formulations is routinely made by those of ordinary skill in the art and is within the ambit of tasks routinely performed by them. Appropriate dosages may be ascertained through use of appropriate dose-response data.

One may further administer the present pharmaceutical compositions by pulmonary administration, see, *e.g.,* PCT WO94/20069, which discloses pulmonary delivery of chemically modified proteins, herein incorporated by reference. For pulmonary delivery, the particle size should be suitable for delivery to the distal lung. For example, the particle size may be from 1µm to 5µm, however, larger particles may be used, for example, if each particle is fairly porous.

Alternatively or additionally, the compositions may be administered locally via implantation into the affected area of a membrane, sponge, or other appropriate material on to which an OP an OPGbp selective binding agent has been absorbed or encapsulated. Where an implantation device is used, the device may be implanted into any suitable tissue or organ, and delivery of an OPGbp selective binding agent may be directly through the device via bolus, or via continuous administration, or via catheter using continuous infusion.

Pharmaceutical compositions of the invention may also be administered in a sustained release formulation or preparation. Suitable examples of sustained-release preparations include semipermeable polymer matrices in the form of shaped articles, *e.g.* films, or microcapsules. Sustained release matrices include polyesters, hydrogels, polylactides (See *e.g.*, U.S. Patent No. 3,773,919, EP 58,481), copolymers of L-glutamic acid and gamma ethyl-L-glutamate (Sidman et al, Biopolymers, 22: 547-556 [1983]), poly (2-hydroxyethyl-methacrylate) (Langer et al., J. Biomed. Mater. Res., 15: 167-277 [1981] and Langer, Chem. Tech., 12: 98-105 [1982]), ethylene vinyl acetate, or poly-D(-)-3-hydroxybutyric acid. Sustained-release compositions also may include liposomes, which can be prepared by any of several methods known in the art. *See e.g.,* Eppstein et al., Proc. Natl. Acad. Sci. USA, 82: 3688-3692 (1985); EP 36,676; EP 88,046; and EP 143,949.

OPGbp selective binding agents, i.e. antibodies and fragments, variants, derivatives and fusions thereof, may be employed alone or in combination with other pharmaceutical compositions. For example, pharmaceutical compositions comprising separately or together an OPGbp antagonist and an interleukin-1 receptor antagonist, or an OPGbp antagonist and a soluble TNF receptor-1, or an OPGbp antagonist and a soluble TNF receptor-2 may be used for the treatment of rheumatoid arthritis. Further, compositions comprising separately or together an OPGbp antagonist and a cancer therapy agent may be used for the treatment of cancer and associated loss of bone mass. Other combinations with an OPGbp antagonist or agonist are possible depending upon the condition being treated.

It may be desirable in some instances to use a pharmaceutical composition comprising an OPGbp selective binding agent compositions in an *ex vivo* manner. Here, cells, tissues, or organs that have been removed from the patient are exposed to pharmaceutical compositons comprising OPGbp selective binding agents after which the cells, tissues and/or organs are subsequently implanted back into the patient.

In other cases, a composition comprising an OBGbp selective binding agent may be delivered through implanting into patients certain cells that have been genetically engineered, using methods such as those described herein, to express and secrete the polypeptide, selective binding agents, fragments, variants, or derivatives. Such cells may be animal or human cells, and may be derived from the patient's own tissue or from another source, either human or non-human. Optionally, the cells may be immortalized. However, in order to decrease the chance of an immunological response, it is preferred that the cells be encapsulated to avoid infiltration of surrounding tissues. The encapsulation materials are typically biocompatible, semi-permeable polymeric enclosures or membranes that allow release of the protein product(s) but prevent destruction of the cells by the patient's. immune system or by other detrimental factors from the surrounding tissues.

Methods used for membrane encapsulation of cells are familiar to the skilled artisan, and preparation of encapsulated cells and their implantation in patients may be accomplished without undue experimentation. See, *e.g.,* U.S. Patent Nos. 4,892,538; 5,011,472; and 5,106,627. A system for encapsulating living cells is described in PCT WO 91/10425 (Aebischer et al.). Techniques for formulating a variety of other sustained or controlled delivery means, such as liposome carriers, bio-erodible particles or beads, are also known to those in the art, and are described. The cells, with or without encapsulation, may be implanted into suitable body tissues or organs of the patient.

A therapeutically or prophylactically effective amount of a pharmaceutical composition comprising an OPGbp selective binding agent (such as an anti-OPGbp antibody, or fragment, variant, derivative, and fusion thereof) will depend, for example, upon the therapeutic objectives such as the indication for which the composition is being used, the route of administration, and the condition of the subject. OPGbp antagonist antibodies or antigen binding domains of the invention are administered in a therapeutically or prophylactically effective amount to prevent and/or treat loss of bone associated with metastatic bone disease. A "therapeutically or prophylactically effective amount" of an OPGbp antagonist antibody is that amount which reduces the rate and/or extent of loss of bone mass or prevents the loss of bone mass in a subject having normal bone mass. Changes in bone mass are detected by a variety of known methods such as single photon absorptiometry (SPA), dual photon absorptiomerty (DPA), dual energy X-ray absorptiometry (DEXA), quantitative computed tomography (QCT), and ultrasonography (See Johnston et al. in Primer on the Metabolic Bone Disease and Disorders of Mineral Metabolism, 2nd ed., M.J. Favus, ed. Raven Press pp. 137-146). One skilled in the art can use these methods to determine a therapeutically effective amount of an OPG fusion polypeptide. A therapeutically effective amount may also be determined by measuring changes in biochemical markers for bone turnover, such as serum osteocalcin, serum alkaline phosphatase, serum procollagen I extension peptides, urinary or serum C-terminal or N-terminal telopeptide of collagen, urinary calcium, hydroxyproline and urinary pyridinoline and deoxypyridinoline. It is generally recognized that a decrease in the levels of the aforementioned biochemical markers indicates that bone resorption is decreased and loss of bone mass is being reduced. Alternatively, a therapeutically effective amount of an OPG fusion polypeptide may also be determined by measuring a change in the mechanical strength of bone, in particular an increase in torsional (twisting) strength of bone.

Accordingly, it may be necessary for the caretaker to titer the dosage and modify the route of administration as required to obtain the optimal therapeutic effect. A typical dosage may range from about 0.1 µg/kg to up to about 100 mg/kg or more, depending on the factors mentioned above. In other embodiments, the dosage may range from 1 µg/kg up to about 100 mg/kg; or 5 µg/kg up to about 100 mg/kg; or 0.1 µg/kg up to about 100 mg/kg; or 1 µg/kg up to about 100 mg/kg Typically, a clinician will administer the composition until a dosage is reached that achieves the desired effect. The composition may therefore be administered as a single dose, or as two or more doses (which may or may not contain the same amount of an OPGbp selective binding agent) over time, or as a continuous infusion via implantation device or catheter.

### Example 1

### Reagents and Assays

The screening target used in these studies was prepared from expression of a cDNA encoding human OPGbp of amino acids 140 through 317 inclusive as shown in Figure 4 of PCT WO98/46751 in a CHO host cell and purified as follows. A Q Sepharose column (Pharmacia) was equilibrated with 20mM tris pH 8.5. Conditioned media which had also been titrated to pH 8.5 was applied, the column washed with the Tris buffer, and proteins were eluted with a 100-600mM NaCl gradient over 20 column volumes. Fractions containing OPGL were identified through SDS-PAGE and Western blot analysis. OPGbp containing fractions were then titrated to pH 4.8 and applied to a Sp column (Pharmacia) which had been equilibrated with 20mM sodium acetate pH 4.8. After washing, proteins were eluted with a 0-0.3M NaCl gradient followed by 0.5M and 1M NaCl steps. OPGbp eluted with all buffers however only the 0-0.3M NaCl gradient fractions were found to be active in vitro osteoclast stimulating bioassays. The yield was 40mg/l. Amino-terminal sequencing revealed that about 80% of the purified protein started with amino acid 143 of human OPGbp while the remaining about 20% started with amino acid 147. The final product used for screening phage libraries is referred to as OPGbp[143-31], the predominant purified form.

Anti-OPGbp polyclonal antibodies were prepared as follows. Three white New Zealand rabbits (Western Oregon Rabbit Co., Philomath, OR) were initially injected with equal amounts of Hunter Titer Max (CytRx Corp., Atlanta, GA) and OPGbp[143-317]. 0.2 mgs per rabbit was injected. This was repeated four and six weeks later. A 50 ml bleed was performed at seven weeks and once per week thereafter for a total of six bleeds. The antibodies were affinity purified from sera of immunized rabbits on an OPGbp resin as follows. Three mls of Actigel Ald resin (Sterogene) were added to a 10ml column (Kontes Flex Colum) and washed with 50 mls of PBS. Three mgs of OPGbp[143-317] diluted into 3 mls of PBS was added to the Actigel column and shaken gently to mix. 0.6mls of 1M Na Cyanoborohydride was then added and the mixture shaken overnight at 4°C. The column was washed with 50mls of Pierce Gentle Elution Buffer (Pierce) followed by 150mls of PBS. 50mls of sera from immunized rabbits was sterile filtered through a 0.45µm filter, added to the column and the mixture was shaken overnight at 4°C. The following day the column contents were allowed to settle and the liquid phase was drained. The column was then washed with 150mls of PBS to an OD₂₈₀ of 0.002. Pierce Gentle Elution Buffer with 1% Glacial Acetic Acid was then added to the column and 1 ml fractions were collected at 10 min intervals and analyzed by OD₂₈₀. Fractions containing the highest amount of OD₂₈₀ absorbing material were pooled and dialyzed against two liters of PBS for 48 hours. There was one buffer change during this time.

ELISA assays were performed on eluted phage pools by plating OPGbp[143-317] at 1.5 µg/ml in PBS pH 8.0 for 2 h at room temperature in Nunc Maxisorp Immunoplates on a rocker. A rinse solution of 2% MPBS (Block Buffer) was added to the immunoplates, incubated for 3 min at room temperature and discarded. Blocking was performed for 1 hour at room temperature with 2% MPBS. Washes were performed 5X using TBS-Tween-20 (0.1%) (TBS; Tris Buffered Saline; 10 mM Tris-HCl (pH 7.5), 1 mM EDTA, 150 mM NaCl). A titration of phage were added using a minimum of 10¹⁰ phage/well in Conjugate Dilution Buffer (0.4%.Nonfat Dry Milk in TBS or, 0.4% M-TBS) for 1 hour at room temperature. Washes were performed using TBS-Tween-20 (0.1%). Anti-M13-horse radish peroxidase (HRP) Monoclonal Antibody Conjugate (Pharmacia Piscataway, NJ) was used at a 1/2000 dilution in 0.4% MTBS for 1.5 h at room temperature. Washes were performed 5 times with TBS-Tween-20 (0.1%). 2,2'-Azinobis(3-ethylbenzthiazoline-sulfonic acid) (ABTS) (Pierce, Rockford, IL) a colorimetric substrate for detection at OD405 was added. Positive controls for the detection of plated huOPG bp [143-317] was performed by addition of OPG[22-194]-Fc, followed by anti-Fc-alkaline phosphatase and para-nitrophenylphenol (pNPP) substrate for detection.

### PCR conditions and 2xTY-AG culture

A typical polymerase chain reaction (PCR) was performed in a 96-well Thermowell plate. Each well contains 20 *µ*l of PCR reaction mix [2 *µ*l 10X PCR buffer (Gibco BRL Products, Grand Island, NY), 17.3 *µ*l water, 0.2 *µ*l dNTPs (25 mM), 0.2 *µ*l Primer 870-02, 0.2 *µ*l Primer 2182-83 (primer stocks 10 pmol/*µ*l for insert amplification), 0.1 *µ*l Taq polymerase]. Individual colonies were picked and resuspended in a well and overlayed with 20 *µ*l of mineral oil, sealed, then placed in PCR machine.
870-02 5'-CCG ACT TTG CAC CTA GTT (SEQ ID NO: 22) 2182-83 5'-TTT GTC GTC TTT CCA GAC GTT AGT (SEQ ID NO: 23)

A duplicate plate for preparing cultures was generated by transferring the same picked colony to the corresponding well position in a second 96-deep well block. Cultures were grown in 0.3 to 1.0 ml 2xTY-AG (2xTY broth: (16 g bacto-tryptone / liter water, 10 g Yeast extract / liter water, 5 g NaCl/ liter water), containing 100 µg/ml ampicillin and 2% glucose). The block was sealed with air-permeable tape, centrifuged at 1000 rpm for 2 minutes to bring down the liquid, and 37°C incubator at 300 to 350 rpm overnight for culturing. The overnight cultures received 150 *µ*l/well of 50% glycerol, were mixed, and frozen at -80°C.

The PCR reaction conditions were 40 cycles of 45 sec. at 90°C, 45 sec at 55°C, 1.5 min at 72C, followed by a 72°C.extension for 10 min. After the PCR reaction was complete, 2.5 to 4.0 *µ*l were run on 25-well 1% agarose gels with 0.5 *µ*l/ml ethidium bromide, using DNA molecular weight standards (Gibco BRL Products, Grand Island, NY, or Stratagene, La Jolla, CA) for 90 min at 90 volts. Only full-length inserts of greater than 1.6 kb were considered.

A 16 *µ*l aliquot of the PCR reactions was BstNI digested 3 hours at 60°C with a 30 *µ*l total digestion mixture containing 10 *µ*l water, 3 *µ*l of 10X REact Buffer 2 (GIBCO BRL Products), 0.3 *µ*l BSA (10 mg/ml)., 0.7 *µ*l BstNI (GIBCO; 10,000 units/ml). Digested samples were run on a 25-well 3% agarose gels for 3.5 hours at 80 volts.

### RAW cell assays

Varying concentrations of Fab test samples were mixed with a constant amount of human OPGbp[143-317] and incubated for at least one hour at room temperature in DMEM, 10% fetal bovine serum and 1x glutamine-penicillin-streptomycin mixture. The concentrations of Fab samples and OPGbp are indicated for each experiment. After incubation, the mixture was added to 2 x 10⁴ RAW 264.7 cells/well (American Type Culture Collection, Manassas, VA, Accession No. TIB-71) in a 96 well flat bottom tissue culture plate. RAW cells were cultured in DMEM with 10% fetal bovine serum and 1x glutamine-penicillin-streptomycin. After three days at 37°C and 5%CO2, the media was aspirated from the wells and the cells were stained for Tartrate Resistant Acid Phosphatase (TRAP), an osteoclast differentiation marker, by addition of 100 *µ*l per well of 0.1M citrate buffer with 0.1% Triton X-100, incubation for five minutes at room temperature, addition of para-nitrophenylphosphate (pNPP) substrate and tartrate in citrate buffer containing Triton X-100 (substrate concentration was 20 mM pNPP and 335 mM tartrate) and incubation for an additional five minutes at room temperature. The reaction was stopped by addition of NaOH to a concentration of 0.05M. Acid phosphatase converts the pNPP substrate to para-nitrophenol which is detected by absorbance at 405 nm. The change in absorbance at 405 nm was plotted as a function log dose for both controls and test samples. An analysis of Variance (ANOVA) and relative potency with 95% confidence limits was calculated. Positive controls included varying concentrations of OPG[22-194]-Fc fusion protein or an anti-OPGbp polyclonal antibody preparation preincubated with OPGbp[143-317] and incubated with RAW264.7 cells as described above.

### Bone Marrow Assay

A murine bone marrow assay for osteoclast formation was carried out essentially as described in Lacey et al. (Cell 93, 165-176 (1998)) and Kong et al. (Nature 397, 315-323 (1999)). Briefly, the assay is a modification of the murine bone marrow coculture assay described in PCT WO97/23614 in which non-adherent murine bone marrow cells were cultured in media for about seven days in the presence of human OPGbp (143-317) but without addition of the stromal cell line ST2, 1,25 (OH)₂ vitamin D3 and dexamethsone. Cells having an osteoclast phenotype were detected by the appearance of TRAP-positive cells. TRAP activity was measured in solution or by histochemical staining.

For detection of TRAP activity in solution, adult bone marrow cells were lysed in 100 mM Citrate Buffer (Sigma, Cat # 91-5) + 0.1% Triton X-100, pH 5.0, 3-5 min. 20 mM pNPP, 80 mM Tartrate, and 100 mM Citrate + 0.1% Triton X-100, pH 5.0 were added and incubated at RT, 3-5 min, and measured at 405 nm after stopping the reaction with 50 *μ*l of 500 mM NaOH /well. A positive response was a concentration dependent decrease in absorbance at 405 nm from ∼2.0 OD to -0.6 OD.

For histochemical staining, cells were fixed in a formaldehyde-based fixative solution, then stained with Fast Garnet GBC (2-methyl-4-[(2-methylphenyl)-azo]benzenediazonium)solution + Naphthol AS-BI (C₁₈H1₅BrNO₆P) phosphate solution + Acetate Solution + Tartrate Solution, incubated 1 hour at 37oC, then rinsed, dried, and evaluated microscopically. A cell that was TRAP positive and contained three or more nuclei (TRAP-positive MNC) was considered an osteoclastic cell.

### Example 2

### Screening of a Human Fab library

A library of about 4 x10¹⁰ unique human Fab fragments prepared in bacteriophage M13 was obtained from Target Quest, NV (Amsterdam, Netherlands). General procedures for construction and screening human Fab libraries were described in de Haard et al. (Advanced Drug Delivery Reviews 31, 5-31 (1998); J. Biol. Chem. 274, 18218-18230 (1999)). The library was screened for Fab fragments which bind to OPGbp[143-317] by the following procedures.

### Solid phase direct plating

OPGbp [143-317] prepared as described above was immobilized on a solid phase using Nunc Maxisorb immunotubes (12 x 75mm, 5ml capacity) by directly plating on the solid phase at a protein concentration of 1.5 μg/ml in TBS, pH 8.0 (TBS was Tris buffered saline: 10 mM Tris (pH 7.5), 150 mM NaCl) at room temperature for 2 hours. These conditions permitted 80% of maximum plating of the solid phase at 2 hours (maximum at 2 hours was still nonsaturating) while retaining binding capabilities to OPG [22-194]-Fc. After the 2 hour incubation, the tube was washed three times with PBS. The plated target was blocked by filling the immunotube with 2% nonfat dry milk, (Marvel or Carnation) in PBS (MPBS) for 1 to 4 hours at room temperature, washed two times each in PBS-Tween 20 (0.1%) and PBS. The PEG-concentrated phage (approximately 10") were pre-blocked in 2% MPBS to adsorb milk binding phage prior to exposure of the phage to the solid phase target. The pre-blocked phage were incubated in 4 ml with the plated target at room temperature for 2 h, (30 min rotating end-over-end and 90 min standing). The contents bound to the tube were washed 20 times with PBS-Tween 20 (0.1%) and 20 times with PBS to remove unbound phage and to reduce nonspecific binding. Phage were eluted from the solid phase by a ten minute total phage elution with 1 ml of 100 mM triethylamine (TEA) pH 12, rotating the tube end-to-end, followed by neutralization with 0.5 ml of 1 M Tris-HCl pH 7.4. Alternatively, specific phage binders were recovered by elution with 1 ml of either 1 uM OPGbp[143-317] or 1 uM OPG[22-194]-Fc in 0.4% MPBS, pH 8.0 or pH 7.4, respectively.

Eluted phage (binders) were titered on E. coli strain TG1 (Pharmacia, Piscataway, NJ.). Titering was performed in duplicate by a modification of the "Track-Dilution" method (Huycke et al. BioTechniques 23, 648-650 (1997)) by 10 μl phage dilution in 2xTY broth into 90 ul log phase (A600 0.2 to 1.0 ODs) TG1 cells, mixed and incubated 20-30 minutes at room temperature. Ten *μ*l were streaked horizontally in a lane, 6 lanes per 2xTY-AG square petri dish (2xTY broth, containing 2% glucose, 100 *μ*l /ml ampicillin and 15 g/liter agar), and incubated overnight at 37°C.

The eluted phage (binders) were amplified through bacterial infection in TG1 cells. Twenty-five ml of 2xTY broth were inoculated with *E. coli* TG1 cells and grown at 30°C for more than 12 h, 270 rpm. The overnight culture was inoculated 1:100 in 50 ml of 2xTY broth, and grown -1.5 hr, 270 rpm to an OD600 of 0.5. For amplification of selected phage, 5 volumes of exponential *E. coli* TG1 cells were added, 4 volumes of 2xTY broth and 1 volume of eluted (neutralized) phage together and incubated in a waterbath at 37 °C for 30 min. To reduce the volume for plating the cells were centrifuged at 4,000 rpm and the pellet was resuspended in 2x TY-AG broth (100 ug/ml ampicillin, 2% glucose). In the first round of selection, the sample was plated onto two to four 16 cm² 2x TY-AG plates (2xTY broth, containing 2% glucose, 100 ug/ml ampicillin and 15 g agar) to maintain diversity. For later rounds of selection, one plate was sufficient. The plates were incubated overnight at 30 °C. After overnight growth, 5 mls of 2xTY-AG was added to each large plate, and bacteria were scraped loose with a sterile spreader. After complete resuspension and concentration by spinning down at 4,000 rpm, 10 min, a concentrated sample was transferred to a Nunc Cryotube. Sterile glycerol was added to 15% final concentration and immediately stored at -70 °C.

Amplified cells were resuspended in 2x TY-AG broth to ∼0.1 OD and grown for 1.5-2.5 h at 37°C, 270 rpm, to an 0D600 of 0.5 and transferred (5 ml) to a 50-ml Falcon tube containing an appropriate amount of M13K07 helper phage (Gibco BRL Products, Grand Island, NY), with a 20 to 1 ratio of phage to bacteria. The mixture of phage and bacteria were incubated at 37°C for 30 min without agitation followed by centrifugation for 15 min, 3,700 rpm. The supernatant was removed and the bacterial pellet was resuspended in 25 ml of 2xTY-AK (100 ug/ml ampicillin, 25 ug/ml kanamycin) and transferred to a 250 ml flask for overnight incubation at 30°C with shaking at 270 rpm. Next day, the culture was centrifuged in a 50-ml Falcon tube for 20 min at 3,700 rpm to pellet the bacteria. To the supernatant, 1/5th of the volume of a polyethylene glycol (PEG) solution (20% PEG 8000, 2.5M NaCl) was added and kept on ice for at least 1 hr. Phage were pelleted 20 min, 3,700 rpm at 4°C. Supernatant was discarded and the pellet was resuspend in ∼1.0 ml sterile PBS and transferred to a 1.5 ml eppendorf tube. The sample was microcentrifuged 2 min. -14,000 rpm to remove the remaining bacteria and the supernatant was transferred to a new tube. The PEG precipitation was repeated. The concentrated PEG precipitated phage were used in selection or screening assays. The standard yield was about 1-5 x 10¹³ phage from a 25 ml culture. For longer storage, glycerol was added to the phage (15% final concentration) and the phage were stored at -70°C.

This procedure describes one round of screening, comprising the steps of binding, elution and amplification. Typically, three to five rounds of screening were performed in order to obtain an eluted phage pool which bound OPGbp [143-317] in an ELISA assay at a level at least four fold over background. After screening was completed, the final eluted phage were plated for individual colonies and the inserted DNA analyzed by colony PCR and BstNI digestion as described below.

### Solution phase

Phage were preblocked for 60 min on a rotator at room temperature in 2% MPBS. Biotinylated OPGbp b-b' loop peptide (500 nM) was added directly into the equilibrated phage mix and incubated for 30 min to 1 hour on a rotator (end-over-end) at room temperature. The loop peptide had the sequence [biotin(LC)-TDIPSGSHKVSLSSWYHDRG] (SEQ ID NO: 24) where the LC (linear chain i.e., (CH₂)₅-NH₂)) was used to link the OPGbp b-b_{'} loop sequence to biotin. Streptavidin-coated Dynabeads (100 *μ*l per selection in 1.5 ml eppendorf tubes) were used for solution phase capture of biotinylated antigen-phage complexes (3X for negative and 1X for target antigen selection). Streptavidincoated beads were pre-equilibrated by being drawn to the side of the tube using a Dynal magnet, buffer was removed and beads resuspended in 1 ml of 2% MPBS. Equilibration at room temperature was for 1-2 h on an end-over-end rotator.

Three negative selections were performed in rounds 2 and 3. For negative selection preblocked phage were added to a tube of streptavidin coated Dynabeads pre-equilibrated in 2% MPBS and incubated for 30 min on an end-over-end rotator at room temperature (repeated two times). Beads were drawn to the side and unbound phage transferred into a fresh eppendorf tube to be used for antigen selection. Biotinylated huOPGbp b-b' loop peptide (500 nM) was added directly into the equilibrated phage mix and incubated for 30 min to 1 hour on an end-over-end rotator at RT. Equilibrated beads were drawn to the side of the tube, buffer was removed and resuspended with the phage-biotinylated peptide mix followed by incubation for 15 min on an end-over-end rotator at RT. Tubes were placed in a magnetic rack for 1 min, aspirated and beads were washed 6x with 1 ml of 2% MPBS-Tween-20 (0.1%), 6x with 1 ml of PBS-Tween-20 (0.1%), and 2x with 1 ml of PBS. Phage were eluted with 1 ml of 100 mM TEA pH 12 for 5-10 min on a rotator at room temperature followed by neutralization with 0.5 ml of 1M Tris-HCl, pH 7.4.

### Example 3

### Identification of Fab clones which bind OPGbp

*E. coli* TG1 cells were infected with the phage pool from an ELISA responsive round and individual colonies were picked for PCR analysis. Typically one to four plates of 96 colonies were picked for each selection. Fab cDNAs were amplified by PCR by a specific set of primers and analyzed on an agarose gel for Fab insert length. Fab insert lengths > 1.6 kb were full length. cDNAs were also digested with BstNI restriction enzyme and the banding pattern analyzed by electorphoresis on agarose gels. Clones which exhibited identical size PCR full-length inserts and identical BstNI banding patterns in two or more isolates were candidates for further analysis. Using the above criteria, the following Fabs were identified.

Fab pattern "P" was identified after solution phase screening using three rounds of elution with triethylamine, pH 12, followed by solid phase screening as described above using one round of elution with 1 uM OPGbp[143-317].

Fab pattern "S' was identified by solution phase screening using three rounds of elution with triethylamine, pH 12, followed by solid phase screening as described above using two rounds of elution with 1 uM OPG[22-194]-Fc.

Fab pattern "AT" was identified by solid phase screening as described above using four rounds of elution with 1 uM OPG[22-194]-Fc.

Fab pattern "Y" was identified by solid phase screening as described above using three rounds of elution with OPGbp[143-317].

Phage were prepared from individual colonies exhibiting Fab AT, Y, P and S patterns by the following procedure. Plasmid preparations were made and transformed into TG1 cells. PCR analysis confirmed the transformation of a full length insert. The cells were grown in either a deep well block (0.5 ml volume) or as a 10 ml culture. Phage were rescued by a 20:1 ratio of M13K07 helper phage/cells infection, PEG precipitated 1 time (as in the solid phase direct plating protocol) and resuspended in ∼ 200 ul from a 2-ml well sized deep well block or -500 ul from the 10 ml culture in PBS.

Phage titers were in the range of 10¹¹-10¹⁴ phage/ml into the ELISA. Titrations based on volume using a maximum of 50 μl/well additions were performed giving a typical range 10⁹-10¹¹ phage/well in an ELISA. Phage ELISA was performed as previously described. The ELISA uses anti-M13-HRP conjugate for detection of bound phage with ABTS, a colorimetric substrate at 405 nm. Anti-M13 HRP conjugate was specific for the major coat protein VIII on the phage. Values were from single point determinations.

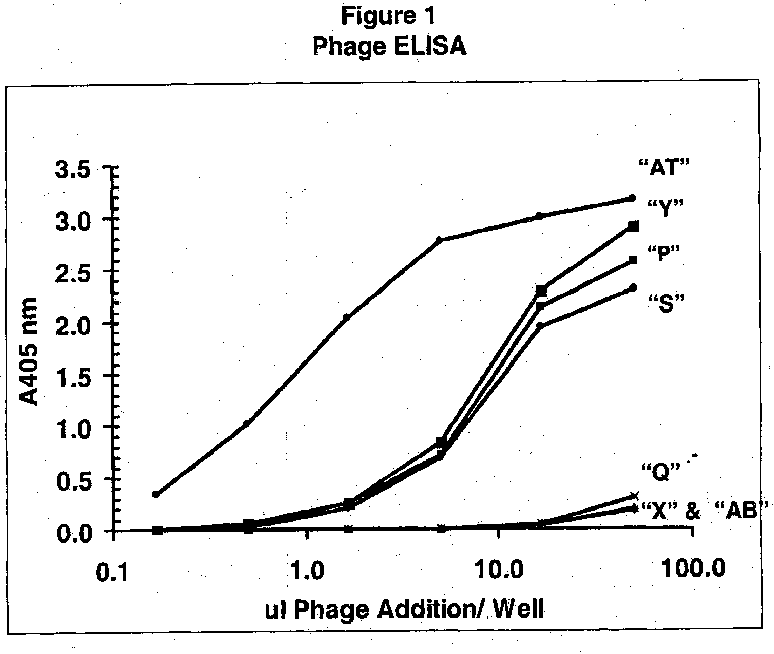

Results of an ELISA of a representative clone from each of the major patterns "AT", "Y", "P" and "S" was shown in Figure 1. All four Fab clones demonstrated significant reactivity with OPGbp[143-317]. All clone members from patterns "AT" and "Y" (27 members from 672 clones and 9 members from 96 clones, respectively) were sequenced found identical within their patterns. Therefore any pattern member will be representative of the entire pattern for patterns "AT" and "Y".

Patterns "Q" (3 members from 96 clones), "X" (3 members from 96 clones) and "AB" (2 members from 96 clones) were also ELISA positive to plated OPGbp[143-317] as determined by a representative clone (Figure 1). Since the ELISA signals were considerably lower than patterns "AT", "Y", "P", and "S", and represented by only two to three members in 96 clones, they were assumed to have Kds in the *μ*M range and were not analyzed further. Pattern "X" was only ELISA positive when the concentration of Tween-20 in the washes was reduced from 0.1% to 0.01%.

### Example 4

### Purification of soluble Fabs

Phage containing Fabs "AT", "Y", "P" and "S" were infected into E. coli HB2151 (Pharmacia, Piscataway, NJ) and expression of Fab fragments was induced by addition of IPTG to 1 mM generally for at least 5 h, except that for pattern Y the IPTG levels were reduced to 0.25 mM. After induction, the cells (750 ml) were harvested by centrifugation and Fabs were released from the periplasmic space by osmotic shock.

The total pellet was resuspended in 8 ml of ice cold TES (0.2 M Tris, 0.5 mM EDTA, 17.1 % sucrose, pH 8.0), transferred to a 50 ml tube and incubated for 5 to 10 min on ice with occasional gentle shaking. Meanwhile, the empty tubes were washed with 8.8 ml TES/H₂O (1:3) to recover the remaining cell pellet and added to the other cells and incubated another 20 min on ice. Cells were centrifuged at 14,000 rpm for 3 min and supernatant transferred from the slightly sloppy cell pellet to another 50-ml tube. The supernatant was again centrifuged at 14,000 rpm for 10 min at 4°C to remove residual cell contamination. The supernatant was referred to as the TES-released periplasmic fraction. The bacterial pellet was resuspended in 10 ml TES plus 15 mM MgSO4, incubated on ice for 15 min and centrifuged twice as above. The supernatant was referred to as the Mg-released periplasmic fraction. Bovine serum albumin (BSA; RIA grade, Sigma) was added as a carrier and stabilizer to each periplasmic fraction to a final concentration of 1 mg/ml and dialyzed overnight at 4°C in 2 L with 1 exchange of Talon column buffer (20 mM Tris-HCl/0.1 M NaCl, pH 8.5) plus protease inhibitors at the final concentrations, Pefabloc 0.05 mg/ml, leupeptin 50 nM, aprotinin 0.06 *μ*g/ml and pepstatin A 0.9 *μ*g/ml.

The Fab-containing periplasmic extracts (TES and Mg-released) were subjected separately to batch method binding 1 h rocking at 4C with 0.8 ml to 1.5 ml (1/20^{th} the extract volume) preequilibrated Talon resin (Clontech), then batch method washing in at least 2Xs 20 column volumes of column buffer. The Talon resin was column packed, washed with 10 column volumes of column buffer, and 2 column volumes of column buffer plus 50 mM imidazole to release nonspecifically bound proteins. Purified Fabs were eluted with 2 to 3 column volumes of 200 mM imidazole, 4% glycerol. Purified extracts were then concentrated/exchanged in a Centricon 10 (Amicon, Inc. Beverly, MA) into PBS, pH 7.4 to a final concentration of 0.5 to 5 mg/ml. Purity of soluble Fab "AT" was determined on a Novex (San Diego, CA) 10% Bis Tris NuPAGE Gel with NuPAGE MOPS SDS Running Buffer (Nonreducing) and 4X LDS Sample Buffer (pH 8.45). Purified Fab samples containing the LDS Sample Buffer were heated at 70°C for 10 min and loaded 40 ul/lane. The gel was run at 200 volts, 20 min, then reduced to 50 volts -1.5 h, stained with Novex Colloidal Coomassie Blue Stain ∼14 h, and destained. Soluble Fab "AT" was determined to have greater than 98% purity.

### Example 5

### Activity of purified anti-OPGbp Fabs

The activity of purified soluble Fabs "AT" and "Y" was analyzed by the following assays.

### OPGbp/ODAR interaction inhibition assay

The interaction of human OPGbp [143-317] and its receptor ODAR was measured in a fluorescence energy transfer (FRET) assay. A 1 hour preincubation with OPGbp was added for increased sensitivity. A soluble extracellular domain of human ODAR was constructed as a fusion polypeptide with an amino terminal FLAG tag and a carboxy terminal human Fc region. The Fc region is recognized and bound by a polyclonal goat anti-human-Fc specific allophycocyanin (APC) which is detected by absorbance at 665 nm. OPGbp [143-317] was labeled with Eu2+ which is detected by absorbance at 620 nm. Binding of OPGbp to ODAR in this assay triggers a fluorescent energy transfer and is highly sensitive for competition curves. A decrease in the A665/A620 absorbance ratio indicates inhibition of OPGbp binding to ODAR.

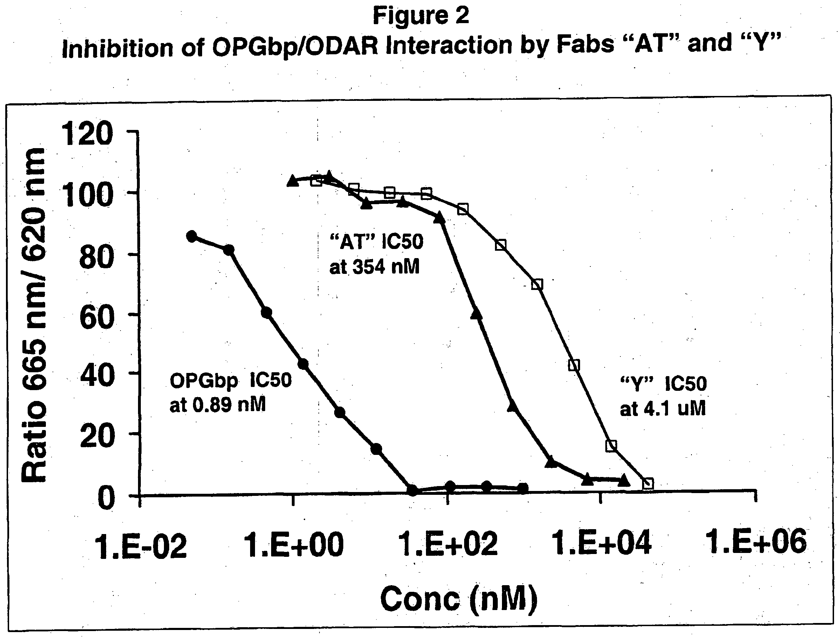

A concentration dependent inhibition of OPGbp-Eu binding to sODAR-Fc was observed for duplicate preparations of soluble Fabs "AT" and "Y". Soluble Fab "AT" (preparation A) inhibited OPGbp binding to soluble ODAR-Fc fusion with an IC50 of 550 nM. Soluble Fab "Y" inhibited with an IC50 of 6 *μ*M. The Fab "AT" clone 1B5 was from the predominant 27 member pattern from seven 96-well plates (all with same amino acid sequence) obtained by elution from OPGbp target using a solution of 1 *μ*M OPG[22-194]-Fc for 90 min. The Fab "Y" clone 1B4 was from the predominant 9 member pattern from one 96-well plate (all with same amino acid sequence) from the optimized plated OPGbp screen using a 1 *μ*M OPGbp elution for 90min. A second purification of Fab"AT", clone 6F11, (designated preparation B) yielded a similar IC50 of 440nM for PBS and IC50 of 354nM for TBS. A second purification of Fab "Y" (preparation B) yielded a similar IC50 of 4.1 *μ*M for TBS. The positive control was OPGbp[143-317] in TBS or PBS with corresponding IC50s of 0.89 and 0.93 nM, respectively. Similar IC50's were obtained with the duplicate preparations "A" and "B". Results of preparation "B" of Fabs "AT" and "Y" in TBS are shown in figure 2.

### Bone Marrow assay

Soluble Fabs "AT" "Y" and "P" were purified as in Example 4 followed by two sequential endotoxin removal steps using a Polymyxin affinity column (∼1 ml; BioRad, Hercules, CA) in PBS at room temperature according to the manufacturers instructions except as follows. To increase the probability of removing endotoxin bound to the Fab, after sample addition, a 100 *µ*l to 150 *μ*l aliquot was recycled from the bottom to the top of the column every 5 min for 2 to 2.5 hours. Fab was eluted with 3 column volumes of PBS with 4% glycerol.

The samples were tested for endotoxin using E-Toxate (Limulus Amebocyte Lysate) detection system (Sigma, St. Louis, MO) according to manufacturer's instructions. The samples were then filter sterilized (0.2 μm). Fab "P" was denatured after purification and bound poorly to OPGbp [143-317] in an ELISA. It was used as a negative control in these experiments.

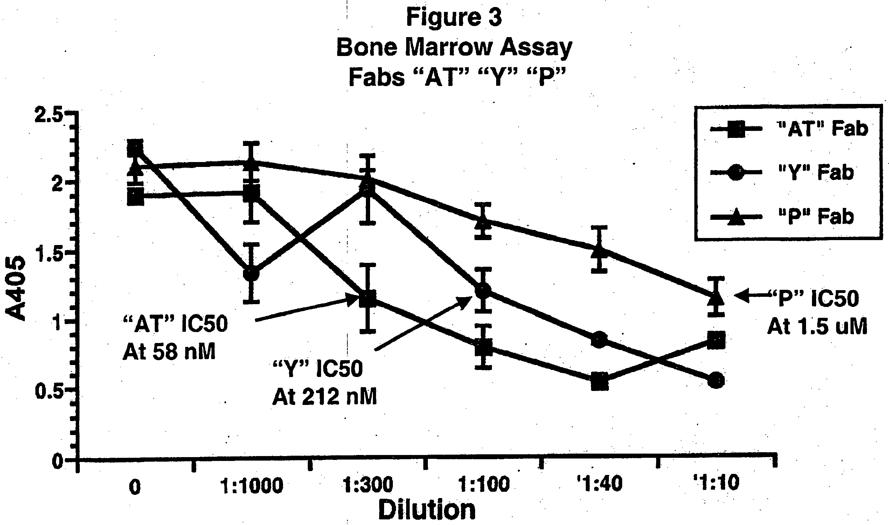

The assay format includes a 1 hour preincubation of the anti-OPGbp Fab with 10 ng/ml of human OPGbp [143-317] (final cell well concentration). TRAP assays were carried out in solution using pNPP chromogenic substrate. The results are shown in Figure 3. Fab "AT" gave a 50% decrease (IC50) at 57.8 nM; Fab "Y" gave a 50% decrease at 212 nM; Fab "P" gave a 50% decrease at 1.5 *μ*M. An estimated Fab molecular weight of 50,000 was used in these calculations to give a conversion factor of 1 *μ*g/ml = 20 nM.

The IC50's as determined by TRAP histochemical staining were similar to those above as determined in the pNPP assay.

### RAW Cell Assay

The effects of adding soluble Fabs "AT", "Y" and "P" to the RAW cell assay are shown in Figure 4.

The 50% point for the graph was taken as 1.65 OD₄₀₅ nm. Fab "AT" has an IC50 of 15 *μ*g/ml, 300 nM (assuming a Fab molecular weight of 50,000). Fab "Y" has a decrease in signal to 2.15 OD₄₀₅ from 2.5 OD₄₀₅, an 80% point. By extrapolation, a 50% point OD4₀₅ of 1.65 for Fab "Y" would be reached at ∼ 400 *μ*g/ml x 20 = ∼8 *μ*M. "P" does not show any detectable reactivity in the assay.

### Example 6

### Sequencing and Analysis of Fab clones

The DNA and predicted amino acid sequences for the light chains of Fabs "AT", "Y", "P" and "S" were shown in Figures 5, 6, 7, and 8 respectively. The DNA and predicted amino acid sequences for the heavy chains of Fabs "AT", "Y", "P" and "S" were shown in Figures 9, 10, 11, and 12, respectively. Figure 13 shows an amino acid sequence comparison matrix of the heavy and light chains respectively of the four predominant Fab pattern clones based on identity and similarity. Identity and similarity were obtained by either the GCG program or calculated by hand. The heavy chain sequences of Fabs "AT" and "Y" has the closest match as they differed by a single amino acid (conservative change) and thus had an identity of 99.6% and a similarity of 100%. The light chain amino acid sequences were compared among the top four patterns for both identity and similarity to each other. Fabs "AT", "Y" and "P" showed an identity of at least 85% and a similarity of at least 89%. Pattern "S" was the most dissimilar being of the rarer V lambda family.

A comparison of the amino acid sequences of the complementary determining regions (CDRs) was shown in Figure 14. Fabs "AT" and "Y" had identical heavy chain amino acid sequences in CDR1, CDR2 and CDR3. The heavy chain CDR1 of Fabs "P" and "S" each had 3 amino acid residues identical to the "AT" and "Y" CDR1 sequence. The CDR2 sequence of "P" and "S" showed greater identity to each other than to the "AT" and "Y" CDR2 sequence. The light chains of Fabs "AT" and "Y" were showed identical lengths of CDR1, CDR2 and CDR3. Patterns "AT" and "Y" light chain CDRs 1 and 3 showed identity in 7 of 11 residues (64%) and 3 of 5 residues (60%), respectively. Although patterns "AT" and "Y" light chain CDR2 show no identity to each other, each contain part of the consensus sequence for light chain CDR2. When the first 4 residues of pattern "AT" were combined with the last 3 residues of pattern "Y", the light chain CDR2 of pattern "P" was obtained. The light chain CDR3 can vary in length from 5 to 25 residues. Therefore, the light chain CDR3s obtained in patterns "AT", "Y" and "P" were very short. The most unique of the four predominant pattern clones was Fab "S."

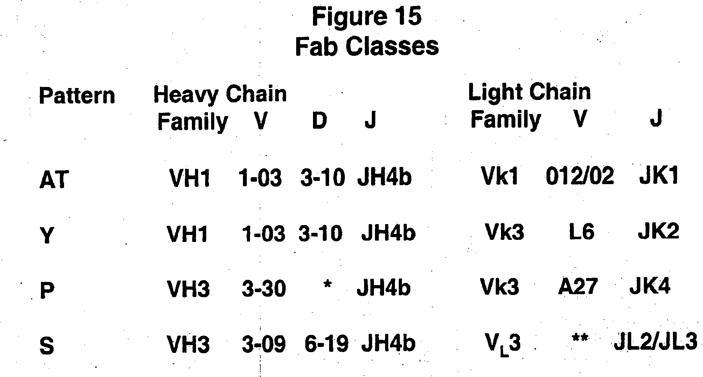

A comparison of the Fab classes represented in the four predominant patterns was shown in Figure 15. These results were obtained from V-Base DNA PLOT Analysis. As expected Fabs "AT" and "Y" were of the same VH1 family (Variable Heavy 1 family) with the same VDJ regions (Variable, Diversity and Joining). Fabs "AT" and "Y" belong to different light chain families. Fabs "P" and "S" belong to the same VH3 heavy chain family but different light chain families. All heavy chains have the same JH4b joining regions.

An alignment of the Fab sequences and the corresponding germ line sequences is shown in Figures 16-18 for the heavy chains and Figure 19-22 for the light chains. Changes in the heavy and light chains of "AT", "Y", "P" and "S" result from naturally occurring sometic matastis that arise in antibody germline sequences during an antibody response. Variable regions of the "AT" and "Y" heavy chains (the amino terminal 127 amino acids in Figures 9 and 10, respectively) had 17 and 18 amino acid changes, respectively, compared to the corresponding VDJ germline sequences. The variable region of the "P" heavy chain (the amino terminal 117 amino acids in Figure 11) has 16 amino acid changes compared to corresponding VDJ germline sequence. The variable region of the "S" heavy chain (the amino terminal 124 amino acids in Figure 12) had 14 amino acid changes compared to germline sequences. The variable region of the "AT" light chain (residues 6-108 in Figure 5) had 16 amino acid changes compared the corresponding VJ germline sequence,; the "Y" light chain (residues 6-108 in Figure 6) had 14 amino acid changes; the "P" light chain (residues 5-108 in Figure 7) had 14 amino acid changes; and the "S" light chain (residues 5-112 in Figure 8) had 12 amino acid changes. In general, amino acid differences in the occurred with the most frequency in the CDR3 regions of both heavy and light chains.

### Example 7

### Cloning and Expression of full-length human OPGbp antibodies

Fab clones were converted to full-length antibodies by the following procedures.

### Construction of pDSRα19:hCH

The plasmid pDSRα19:EPO was digested with HindIII and SalI to remove the coding region for erythropoietin. Plasmid pCRBluntCHl-3 containing the human IgG1 C_{H}1, hinge, C_{H}2 and C_{H}3 domains inserted into the vector pCRBlunt (Invitrogen) was used to obtain a 1.4 kb human IgG1 constant domain. pCRBluntCH1-3 was digested with HindIII and Sal I and the constant domain sequences were inserted into pDRSα19 to generate pDSRα19:hCH.

### Construction of pDSRα19:AT-VH21

Four anti-huOPGbp Fab heavy chain cDNAs were cloned into pDSRα19:hCH to convert the Fabs into full length IgGs. The construction of a plasmid encoding "AT" heavy chain is described here. The other Fab heavy chains were cloned using similar procedures. To generate the Fab with a signal sequence, a three-step PCR was performed. First, primers 2249-25 and 2248-22 were used with the Fab cDNA template Conditions were: 94°C for 1 min, (95°C for 30 sec., 50°C for 1 min., 68°C for 1 min) for 20 cycles, 68°C for 10 min. with Pfu polymerase and the appropriate buffer and nucleotides. The PCR product was then amplified with primers 2209-21 and 2248-22 followed by amplification with primers 2209-20 and 2248-22. The final PCR product was cleaned, cut with HindIII and BsmBI, and gel purified. This fragment contains the Fab with a 5' Kozak (translational initiation) site and the following signal sequence for mammalian expression of Fab "AT" heavy chain:
MEWSWVFLFFLSVTTGVHS (SEQ ID NO: 25)
This signal sequence is designated the VH2₁ signal sequence.

The plasmid pDSRα19:hCH was digested with HindIII and BamBI to remove the polylinker before the IgG C_{H}1-3 domains and the Fab PCR product was inserted to generate pDSRα19:AT-VH21.

Primers:

### Construction of πDSRα19:AT-L

Fabs "AT", "Y", "P" and "S" light chain cDNAs were cloned into pDSRα19 to convert the Fabs into full length antibodies. The construction of a plasmid encoding the "AT" light chain is described here. The other Fabs were cloned using similar procedures. To generate Fab "AT" with a signal sequence, a three-step PCR was performed. First, primers 2233-50 and 2233-51 were used with the Fab cDNA template. The PCR conditions were: 94°C for 1 min., (95°C for 30 sec., 50°C for 1 min., 68°C for 2 min.) for 20 cycles, 68°C for 10 min. with Pfu polymerase and the appropriate buffer and nucleotides. The PCR product was then amplified with primers 2148-98 and 2233-51 followed by amplification with primers 2148-97 and 2233-51. The final PCR product was cleaned, cut with HindIII and SalI, and gel purified. This fragment contains the Fab with a 5' Kozak (translational initiation) site and the following signal sequence for mammalian expression of the "AT" light chain:
MDMRVPAQLLGLLLLWLRGARC (SEQ ID NO: 33)
This signal sequence is designated the "light" signal sequence.

The plasmid pDSRα19:EPO was digested with HindIII and SalI to remove the EPO gene before the IgG light chain PCR product was inserted to generate of pDSRα19:AT-L.

Primers:

### Construction of pDSRa19:AT-tPA

Expression vectors for production of "AT", "Y", "P" and "S" full-length heavy chains were constructed as described above except that primers were modified to introduce the following signal sequence:
MDAMKRGLCCVLLLCGAVFV7SPSRGRFRR (SEQ ID NO: 42)
This signal sequence is designated the "tPA-RGR" signal sequence.

### Construction of pLDH-AT

An expression vector was constructed that included both "AT" heavy and light chains. The plasmid pDSRα19:AT-Vh21 was digested with AatII and NdeI and the ends filled by T4 DNA polymerase. The fragment containing the "AT" heavy chain expression cassette ("AT" heavy chain coding sequence flanked by the promoter and polyadenylation site from pDSRα19) was gel purified and ligated to of pDSRα19:AT-L which had been linearized with NheI, filled with T4 DNA polymerase and dephosphorylated with alkaline phosphatase. The heavy chain expression cassette was in the same transcriptional orientation as the light chain and DHFR genes.

### Antibody Preparation

Expression vectors containing cDNA encoding heavy and light chain "AT", "Y", "S" and "P" full-length antibodies (either in separate vectors or in a single vector) were transfected into CHO cells and cultured under conditions to allow expression of heavy and light chains and secretion into the cell media. The conditioned media was filtered through a 0.45 μm cellulose acetate filter (Corning, Acton, MA) and applied to a Protein G sepharose (Amersham Pharmacia Biotech, Piscataway, NJ) column which had been equilibrated with PBS - Dulbecco's Phosphate Buffered Saline without calcium chloride and without magnesium chloride (Gibco BRL Products, Grand Island, NY). After sample application the column was washed with PBS until absorbency at 280 nm reached baseline. Elution of protein was achieved using 100 mM Glycine, pH 2.5. Fractions were collected and immediately neutralized by addition of 1M Tris-HCl, pH 9.2. Antibodies were detected by SDS-polyacrylamide gels visualized by Commassie staining.

Fractions containing antibody were pooled, concentrated and diafiltered into PBS using either Centricon 10 (Amicon) or for larger volumes Centriprep 10 (Amicon).

The isolated antibody was characterized by gel filtration on Superose 6 (Amersham Pharmacia Biotech, Piscataway, NJ) and was shown to run as a monomeric IgG.

### Example 8

### BIAcore Analysis of Fab and antibody binding to OPGbp

The binding constant (Kd), on rate constant (ka) and off rate constant (kd) for Fabs "AT" and "Y" and "AT" antibody were determined by surface plasmon resonance techniques (BIAcore, Pharmacia, Piscataway, New Jersey) and the results are shown in Table II. Fabs were prepared as described in Example 4 and "AT" antibody prepared as described in Example 7. OPGbp was immobilized on a CM5-chip by standard amine coupling. The Kd and rate constants were determined by BIAEVALUATION 3.0 software (Pharmacia).

**TABLE II**

| BIAcore Analysis of Fabs "AT" and "Y" and "AT" antibody | | | |
|---|---|---|---|
| | Kₐ(M⁻¹s⁻¹) | Kₐ(s⁻¹) | K_{D}(nM) |
| Fab "Y" | 9.44 x 10³ | 5.91 x 10⁻³ | 594 |
| Fab "AT" | 2.2 x 10⁶ | 0.31 | 140 |
| "AT" Ab (147 RU) | 5.7 x 10⁶ | 2.4 x 10⁻³ | 0.42 |
| "AT" Ab (80 RU) | 8.1 x 10⁶ | 2.7 x 10⁻³ | 0.33 |
| "AT" Ab (47 RU) | 7.3 x 10⁶ | 3.1 x 10⁻³ | 0.43 |

The binding affinity of Fab "AT" increased from about 140 nM to about 0.33 to 0.43 nM, or about 350 to 400-fold, when an Fc IgG₁ constant region was added as described in Example 7.

### Example 9

### Activity of anti-OPGbp antibodies

### RAW Cell Assay

"AT" antibody preparations designated 405, 406 and 407 were tested in a RAW cell assay as described in Example 1. "AT" 405, "AT" 406 and "AT" 407 differ only in the leader sequences used for expression ("AT" 405 was expressed using the "light" signal sequence, "AT" 406 used the tPA-RGR signal sequence, and "AT" 407 used the VH21 signal sequence). The purified mature antibodies were identical in each preparation. The results are shown in Figure 23.

IC50's for "AT" 405, "AT" 406 and "AT" 407 were 20.1 nM, 60.3 nM & 21.4 nM, respectively corresponding to 3.0 ug/ml, 9.0 ug/ml and 3.2 ug/ml, respectively (assuming a molecular weight of 150,000). The positive controls of OPG[22-194]-Fc and anti-OPGbp polyclonal antibody were at 33 ng/ml and 150 ng/ml, respectively.

The difference in IC50 in the Raw cell assay of the "AT" Fab fragment was 300 nM as compared to about 20 nM for the "AT" full-length, or about a 15-fold increase for the "AT" full-length antibody. Taking into account the differences in OPGbp concentrations in the two experiments (150 ng/ml / 60 ng/ml = 2.5 fold), the increase in cell function avidity for the "AT" full-length antibody was about 15 x 2.5 = 37.5 fold.

### Bone Marrow Assay

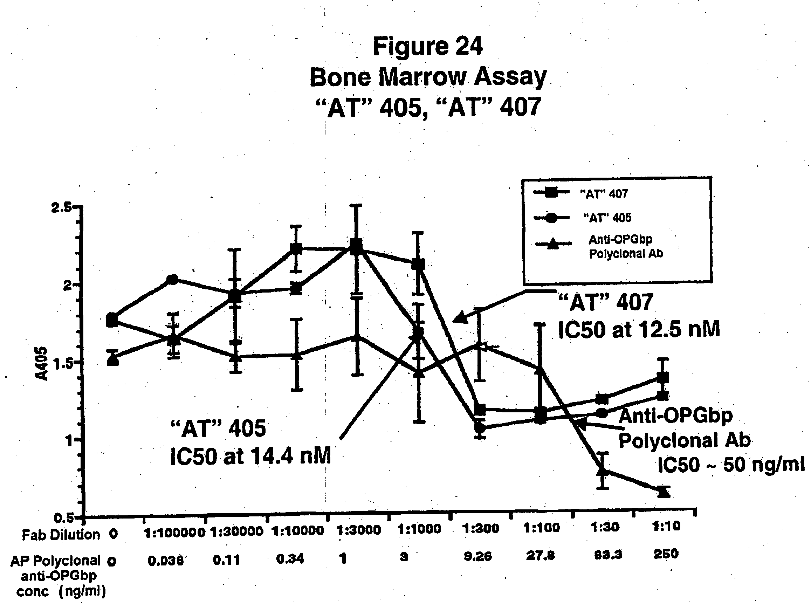

The effects of adding "AT"405 or "AT" 407 preparations to the murine bone marrow assay were shown in Figure 24. IC50's for "AT" 405 and "AT" 407 were 2.15 ug/ml (14.4 nM), and 1.85 ug/ml (12.5 nM), respectively, (MW = 150,000). A rat anti-OPGbp polyclonal antibody inhibited TRAP formation with an IC50 at ∼50 ng/ml.

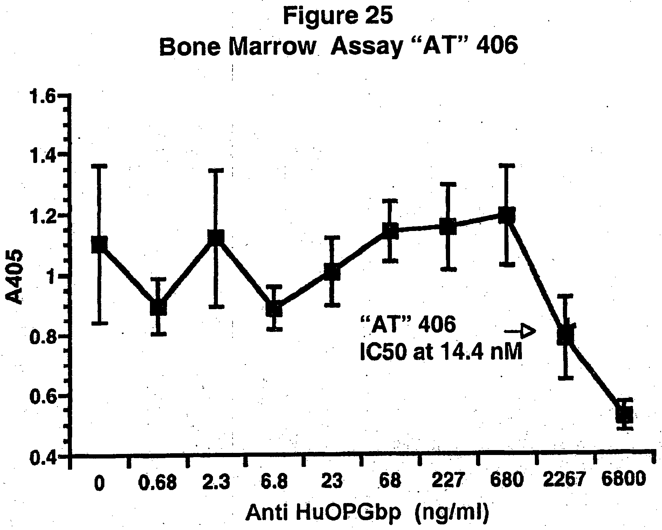

In a separate experiment, "AT" 406 was tested in the bone marrow coculture assay and inhibited TRAP formation with an IC50 of 2.35 *μ*g/ml (14.4 nM) assuming an antibody molecular weight of 150,000 (see Figure 25).

The difference in IC50 in the bone marrow assay for "AT" Fab fragment was about 50 nM as compared to about 13 nM for "AT" full-length antibody, or about a 3.85 fold increase. Taking into account the differences in OPGbp concentrations in the two experiments (50 nM / 9 nM = 5.55 fold), the approximate gain in cell function avidity from "AT" full-length antibody was about 3.85 x 5.55 = 21.4 fold.

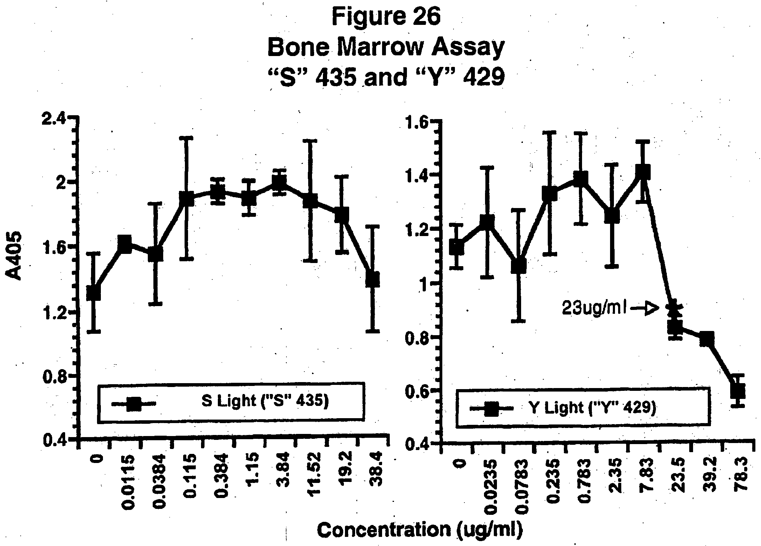

cDNAs encoding Fabs "Y", "P" and "S" were also fused to human IgG1 CH1, CH2, and CH3 sequences as described in Example 7 and transfected into CHO cells. The resulting antibodies were expressed and purified from conditioned medium and exdotoxin removed as before. "S" and "Y" light chain antibodies (referred to as "S Light" and "Y Light") were tested in the bone marrow assay and the results shown in Figure 26. "S light" and "Y light" antibodies were expressed using the corresponding light chain leader sequences and were designated "S" 435 and "Y" 429, respectively. The "Y" 429 ("Y Light") had an IC50 of 23 ug/ml or 154 nM. "S" 435 ("S Light") did not exhibit sufficient activity for a determination of an IC50.

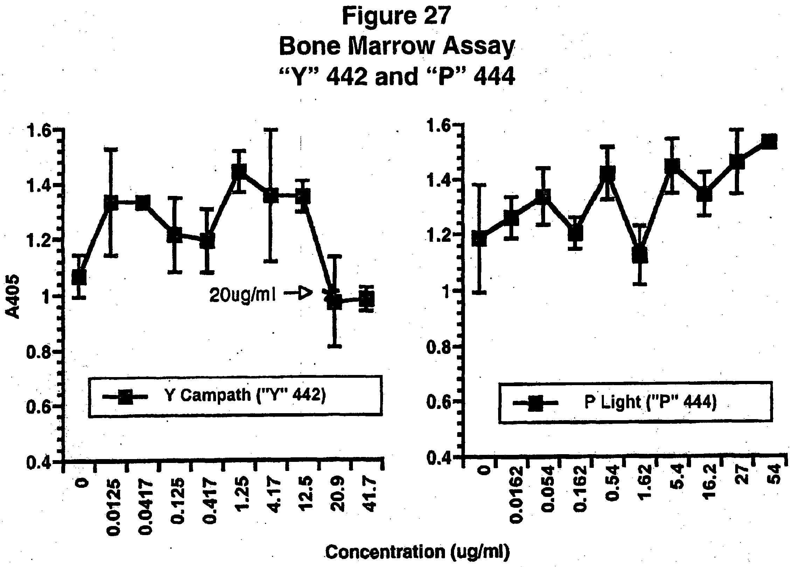

The "Y Campath" and "P Light" were Hu-IgG1 sequence "Y" and "P", respectively with the leader sequence from the "Campath" and "Light" Chain, and were designated "Y" 442 and "P" 444, respectively. The "Y" 442 ("Y Campath") had an IC50 of 20 *μ*g/ml or 134 nM. "P" 444 ("P Light") did not show detectable activity (see Figure 27).

The difference in IC50 in the bone marrow assay of "Y" Fab fragment was 212 nM compared to 134-154 nM for "Y" full -length antibody or about a 1.38 to 1.58 fold increase. Taking into account the differences in OPGbp concentrations in the two experiment (50 nM / 9 nM = 5.55 fold), the approximate gain in cell function avidity from "Y" Fab to "Y" full-length antibody was therefore about (1.38 to 1.58) x 5.55 = 7.7 to 8.8 fold or about 8-fold.

### Example 10

### Identification of an Epitope for "AT" Antibody on OPGbp

### Production of variant murine OPGbp

Human OPGbp[143-317] was produced as described in Example 1. Murine OPGbp[158-316] containing amino acid residues 158 through 316 of as shown in Figure 1 of PCT WO98/46751 preceded by an introduced N-terminal methionine residue was produced in *E. coli* and purified from the soluble fraction of bacteria as described previously (Lacey et al. Cell 93, 165-176 (1998)). FLAG-murine OPGbp[158-316] was generated by introduction of nucleic acid residues encoding an N-terminal methionine followed by a FLAG-tag sequence (DYKDDDDKKL (SEQ ID NO: 99)) fused to the N-terminus of residues 158-316 as shown in Figure 1 of PCT WO98/46751 using methods known to one skilled in the art. The FLAG-OPGbp[158-316] molecule was cloned into bacterial expression vector pAMG21 (deposited with the American Type Culture Collection and having accession no. 98113).

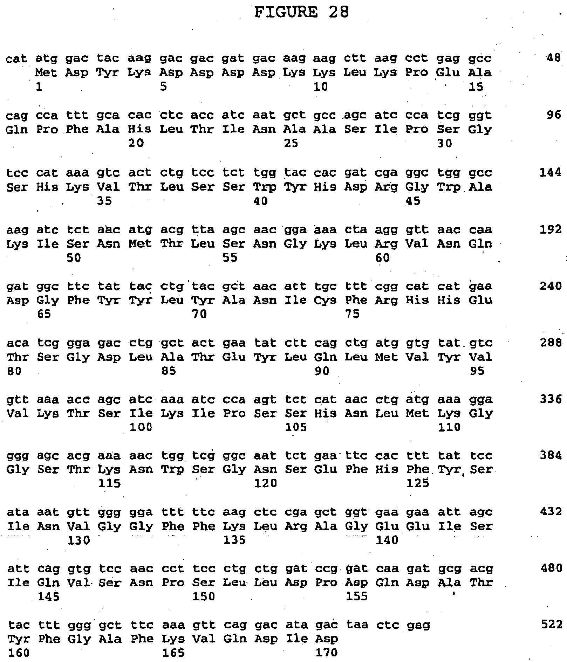

A FLAG-murine OPGbp[158-316] polypeptide variant was constructed in which amino acid residues SVPTD at positions 229-233 inclusive as shown in Figure 1 (SEQ ID NO: 1) of WO98/46751 were substituted with corresponding amino acid residues DLATE at positions 230-234 inclusive as shown in Figure 4 (SEQ ID NO: 3) of WO98/46751. The resulting construct referred to as "FLAG-murine OPGbp[158-316]/DE" has the nucleic acid and protein sequence as shown in Figure 28. The amino acid sequence changes are located in a region of OPGbp between the D and E regions. Figure 29 shows a comparison of murine, human, and murine DE variant amino acid sequences in this region. The sequence changes in the murine variant are S229D, V230L, P231A and D233E with the T at position 234 unchanged. Flanking sequences in this region are virtually identical between murine and human OPGbp.

This molecule was constructed using a two step PCR reaction where the first step contained two separate PCR reactions, designated reaction A and reaction B. For both reaction A and reaction B, pAMG21-FLAG-murine OPGbp[158-316] DNA was used as a template for PCR. Reaction A employed oligonucleotides #2640-90 and #2640-91 for PCR, whereas reaction B employed oligonucleotides #2640-92 and 2640-93. Thermocycling was performed and PCR products from reactions A and B were purified from an agarose gel using methods available to one skilled in the art. The second step PCR reaction, designated reaction C, utilized purified reaction A and reaction B PCR products as a template and oligonucleotides #2640-90 and #2640-93 as primers. Following thermocycling, the product from reaction C was cloned into the pCRII-TOPO cloning vector (Invitrogen) & electroporated into DH10b (Gibco) cells using methods provided by the manufacturer. Clones were selected and sequence confirmed verifying that the introduced mutations resulted in changing the amino acid sequence SVPTD in murine OPGbp[158-316] to DLATE. The sequence verified DNA was then digested with NdeI and XhoI, purified, and subcloned into bacterial expression vector pAMG21 giving rise to plasmid pAMG21-FLAG-murine OPGbp[158-316]/DE.
2640-90: CCTCTCATATGGACTACAAGGAC (SEQ ID NO: 100)
2640-91: AGTAGCCAGGTCTCCCGATGTTTCATGATG (SEQ ID NO: 101)
2640-92: CTGGCTACTGAATATCTTCAGCTGATGGTG (SEQ ID NO: 102)
2640-93: CCTCTCCTCGAGTTAGTCTATGTCC (SEQ ID NO: 103)

*E. coli* host GM94 (deposited with the American Type Culture Collection under accession number 202173) containing plasmid pAMG21-FLAG-murine OPGbp[158-316]/DE was grown in 2XYT media to an exponential growth phase and induced to express the FLAG-murine OPGbp[158-316]/DE protein by addition of V. *fischeri* synthetic autoinducer to 100 ng/ml. Approximately 3-6 hours following induction, the cells were pelleted and recombinant FLAG-murine OPGbp[158-316]/DE protein was purified from the soluble fraction of *E. coli* using methods described in Lacey et al. ibid.

### Binding of "AT" Antibody to human OPGbp[143-317], murine OPGbp[158-316], and FLAG-murine OPGbp[158-316]/DE

Costar E.I.A./R.I.A. Plates (Flat Bottom High Binding, Cat.#3590) were coated with 100 μl/well of either human OPGbp[143-317]protein, murine OPGbp[158-316] protein, or FLAG-murine OPGbp[158-316]/DE protein at 3 μg/ml in PBS, overnight at 4°C with agitation. After overnight incubation, the protein solutions were removed from the plate and 200 μl of 5% Chicken Serum (Gibco/BRL Cat# 16110-082) in PBST (PBS plus 0.05% Tween 20) was added to each well of the plate and plates were incubated at room temperature (RT) for 90 min with agitation. After incubation and blocking, plates were washed 4 times with 1X K-P wash solution in dH₂0 (Cat#50-63-00, Kirkegaard & Perry Laboratories) and dried. Purified "AT" antibody or human OPG [22-194]-Fc protein was serially diluted 1:1 from 2 ug/ml down to 1.953 ng/ml in PBST and 100 ul/well was added to appropriate wells of the microtiter plate coated with either human OPGbp[143-317], murine OPGbp[158-316], or FLAG-murine OPGbp[158-316]/DE protein. Plates were incubated for three hours at room temperature with agitation, washed four times with 1X K-P wash solution and dried. Goat anti-human IgG (Fc) (Jackson ImmunoResearch, Cat# 109-036-098) was diluted 1:5000 in 5% Chicken Serum in PBST and 100 μl was added to each well. Plates were incubated for 1.25 hours at room temperature with agitation, washed six times with 1X K-P wash solution, and dried. 100 μl of undiluted ABTS substrate (Kirkegaard & Perry; Cat# 50-66-00) was added to each well and the dish was incubated at room temperature until sufficient blue-green color developed. Color development was stopped by addition of 100 μl 1% SDS. Quantitation of color development was performed using a microtiter plate reader with detection at 405 nm.

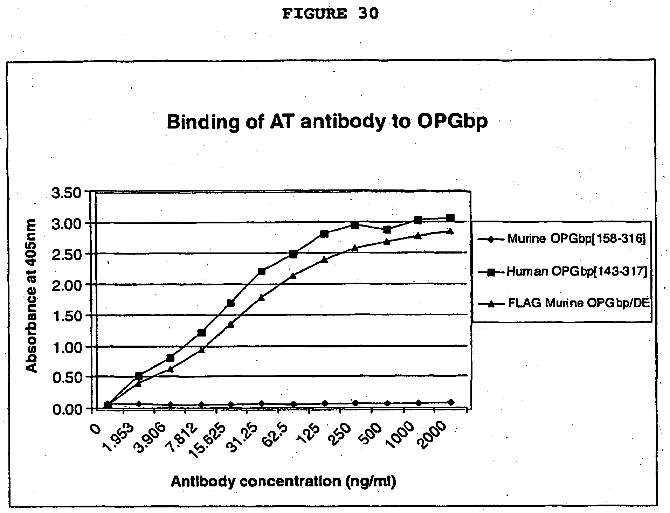

The results of the EIA are shown in Figure 30. The "AT" antibody binds to human OPGbp[143-317] but does not show detectable binding to murine OPGbp[158-316] over the antibody concentration range tested. However, the "AT" antibody binds to both FLAG-murine OPGbp[158-316]/DE and to human OPGbp[143-317] under the assay conditions above. It was concluded that the amino acid changes in murine OPGbp[158-316]/DE compared to murine OPGbp[158-316] were involved in binding of "AT" antibody.

The FLAG-murine OPGbp[158-316]/DE was assayed for activity in a RAW cell assay as described in Example 1 and observed to have a similar ED50 for osteoclast formation as human OPGbp[143-317], indicating that the DE variant is active in promoting osteoclast activity in vitro. Therefore, the binding of "AT" antibody to murine OPGbp[158-316]/DE is likely to inhibit osteoclast formation.

The epitope of the "AT" antibody is located to a region of human OPGbp which includes at least amino acids residues DLATE (residues 230 through 234 of human OPGbp as shown in Figure 4 of PCT WO98/46751).

### Example 11

### Construction of "AT" Fab and Antibody Variants

Three approaches were used to generate CDR variants of "AT" antibody: alanine scanning mutagenesis of heavy chain CDR3 region, substitution mutagenesis of selected sites in heavy chain CDR3 region, and insertion mutagenesis of the light chain CDR3 region.

### Alanine variants of heavy chain CDR3

Alanine scanning mutagenesis was done on the CDR3 region of the AT heavy chain. The amino acid sequence used for alanine scanning was:
DSSNMVRGIIIAYYFDY (SEQ ID NO:_104)
Primers 12 and 15 were annealed to each other and extended by polymerase chain reaction (PCR) under the following conditions: 25 pmol of each primer, 6 cycles of 30 sec at 94°, 2 min at 55°, 20 sec at 74°.
15) 5' GTA GTC AAA ATA GTA CGC TAT AAT AAT TCC CCG AAC (SEQ ID NO: 106)
The PCR product of this reaction is termed Template A.

Template A was extended by PCR using primers 11 and 14 under the following conditions: 0.5 microliters of template A, 10 pmol each primer, 15 cycles of 30 sec at 94°, 2 min at 43°, 20 sec at 74°.
11) 5' GTG TAT TAC TGT GCG AGA GAT TCC TCA AAT ATG (SEQ ID NO: 107)
14) 5' CAG GGT GCC CTG GCC CCA GTA GTC AAA ATA GTA CGC (SEQ ID NO: 108)
The PCR product of this reaction is termed Template B.

Alanine variants of CDR3 were then generated using either template A or B. Two rounds of PCR were done (20 cycles of 94° C for 20 sec and 74°C for 40 sec) using a primer containing the desired alanine codon. The primer containing the alanine codon was in the forward orientation for CDR3 residues (numbering follows Kabat system, see Kabat et al. Sequences of Proteins of Immunological Interest, U.S. Department of Health and Human Services, 4th edition (1991)) D95, S96, V100, R100a. The primer containing the alanine codon was in the reverse orientation for residues S97, N98, M99, G100b, I100c, I100d, I100e, Y100g, Y100h, F100i, D101, Y102.
The mutagenesis reactions were then followed by up to three extension reactions using six common primers. These reactions extended the product to encompass the entire CDR3 region up to and including unique flanking restriction sites BglII and BstEII.

Primers for alanine substitution are shown below in the 5' to 3' orientation. The alanine codon is shown in bold type.

The six common primers for extension PCR are shown below. The sequences of the flanking BglII (top strand) and BstEII (bottom strand) sites are shown in bold type.

### Forward Common primers

11) 5' GTG TAT TAC TGT GCG AGA GAT TCC TCA AAT ATG (SEQ ID NO: 126)

### Reverse Common primers

13) 5' CTT GAG ACG GTG ACC AGG GTG CCC TGG CCC CA (SEQ ID NO: 128)
14) 5' CAG GGT GCC CTG GCC CCA GTA GTC AAA ATA GTA CGC (SEQ ID NO: 129)
15) 5' GTA GTC AAA ATA GTA CGC TAT AAT AAT TCC CCG AAC (SEQ ID NO: 130)

The following table shows the starting template and primer pairs used sequentially to build synthetic DNA fragments containing substituted alanine residues.

**TABLE III**

| | | PCR reactions | | | |
|---|---|---|---|---|---|
| CDR Residue | Template | Alanine Primer pair | Extension Primers | Extension Primers | Extension Primers |
| D95 | A | 60 + 14 | 10 + 13 | | |
| S96 | A | 59 + 14 | 10 + 13 | | |
| S97 | B | 10 + 58 | 10 + 15 | 10 + 14 | 10 + 13 |
| N98 | B | 10 + 57 | 10 + 15 | 10 + 14 | 10 + 13 |
| M99 | B | 10 + 56 | 10 + 15 | 10 + 14 | 10 + 13 |
| V100 | A | 55 + 14 | 10 + 14 | 10 + 13 | |
| R100a | A | 54 + 14 | 10 + 14 | 10 + 13 | |
| G100b | A | 11 + 53 | 10 + 14 | 10 + 13 | |
| I100c | A | 11 + 52 | 10 + 14 | 10 + 13 | |
| I100d | A | 11 + 51 | 10 + 14 | 10 + 13 | |
| I100e | A | 11 + 50 | 10 + 14 | 10 + 13 | |
| Y100g | B | 11 + 20 | 10 + 13 | | |
| Y100h | B | 11 + 19 | 10 + 13 | | |
| F100i | B | 11 + 18 | 10 + 13 | | |
| D101 | B | 11 + 17 | 10 + 13 | | |
| Y102 | B | 11 + 16a | 10 + 13 | | |

For primers used for alanine scanning, the PCR conditions were 20 cycles at 94° C for 20 sec, then 74°C for 40 sec. For extension reactions with primer pair 10 + 15, the conditions where 20-25 cycles of 94° C for 20 sec, 42° for 1 min 30 sec, 74°C for 20 sec. For extension reactions with primer pair 10 + 14, the conditions were 20-25 cycles of 94° C for 20 sec, 48-50° for 1 min 30 sec, 74°C for 20 sec. For extension reactions with primer pair 10 + 13, the conditions were 20-25 cycles of 94° C for 20 sec, 64° for 1 min 30 sec, 74°C for 20 sec. The PCR products were digested with BglII and BstEII restriction enzymes and cloned into FabAT which had been digested with BglII and BstEII, thereby replacing the CDR3 of AT with the alanine substituted CDR3. The alanine substituted constructs were verified by DNA sequencing.

### Substitution variants of heavy chain CDR3

A similar strategy to that used for the alanine scanning mutagenesis was utilized for the randomization of positions S96, S97 and N98 of the heavy chain CDR3 of "AT". Templates A and B were generated as previously described. Positions 96, 97 and 98 were randomized with a set of four primer for each position. The positions in parentheses have variable nucleotides as indicated.

For position S96.

For position S97

For position N98

PCR reactions were done using the reverse randomization primer and primer 10. The resulting product was extended by four sequential PCR reactions using primer pairs 10 + 15, 10 + 14, 10 + 13 and 10 + 22. The 5' end of the heavy chain variable region of "AT" was amplified from a full length clone of the "AT" heavy chain variable region using primers 16 and 72. All PCR products were gel purified. The randomization products were overlapped with the 16/72 product, with flanking primers being 16 and 22. The full-length variable region was then cloned into a vector containing the IgG1 constant region as a HindIII/BsmBI fragment. Full-length antibody clones were selected by sequencing.

Primers used the overlap PCR for_randomization mutants.
72 5' CAC AGC CGT GTC TTC AGA TCT CAG ACT GCG CAG CTG (SEQ ID NO: 145)
22 5' GTG GAG GCA CTA GAG ACG GTG ACC AGG GTG (SEQ ID NO: 146)

The S96A, S97A, and N98A variants were converted from Fabs to full length antibodies by PCR amplification of the Fab clone having the bacterial signal sequence replaced with a mammalian signal sequence. Plasmid DNA from the Fab containing the desired mutation was used as template. Sequential primer pairs used were 21 + 22, 98 + 22, and 16 + 22. Correct clones were selected by DNA sequence analysis.

Multiple alanine variants (S96A, S97A; S96A, N98A; S97A, N98A and S96A, S97A and N98A) were generated using the converted AT heavy chain full length IgG1 plasmid as template. Initial PCR reaction were carried out with one of the primers listed below and primer 22. These products were then extended using primer pairs 10 + 22. That product was then overlapped with the 16/72 product using flanking primers 16 and 22, and cloned into the IgG1 constant region as before. Correct clones were selected by DNA sequence analysis.
S96A, S97A S96A, N98A S97A, N98A S96A, S97A, N98A

### Insertion Variants of light chain CDR3 region

The AT light chain CDR3 contains a five amino acid loop having the sequence QHTRA (SEQ ID NO: 09). Light chain CDR3 sequence variants were constructed as follows. The following primers were used for PCR using a plasmid containing Fab "AT" light chain cDNA as a template:

(HincII)CCG GTC AAC ACA CT(ACGT) (ACGT) (GT)G CGG CGG CGC GGG CGT TCG GCC AAG GG (SEQ ID NO: 153) where (ACGT) indicates a mixed four bases in this position.
CCG GGC GCG CCT TAT TAA CAC TC(AscI) (SEQ ID NO: 154)
The resulting PCR product had a CDR3 loop sequence increased from five to nine amino acids and changed from QHTRA to GHTXAAARA where X can be any amino acid. The AT clone was digested with HincII and AscI digestion and the "AT" light chain CDR3 region was replaced with the variant sequence. Clones containing all twenty amino acid residues in the X position along with three alanine residues inserted in the CDR3 loop were isolated and their identity confirmed by DNA sequencing.

### Purification of Fab Variants

Alanine substitution variants of heavy chain CDR3 and insertion variants of light chain CDR3 were produced and purified as Fab fragments by the following procedure. Each variant was grown in 50 ml of 2XYT with 2% glucose and 100µg/ml amplicillin while shaking at 37°C up to an OD₆₀₀ of 0.8-1.0. Each culture was then spun down and resuspended in 50ml of 2XYT with 100µg/ml Amplicillin with 1mM IPTG at 30°C to induce production of soluble Fabs. The soluble Fabs were then migrated to periplasmic area and concentrated over-night prior to release by osmotic shock. Osmotic shock was carried on by washing the cells by cold 0.5M sucrose solution in Tris buffer and EDTA to break the bacterial cell wall and then quickly diluting it into cold solution of low osmotic strength. The released soluble Fabs were purified on TALON metal affinity chromatography via 6X His-tagged residues on the expressed Fab. The impurities was washed away with NaCl and lower Imidazole concentrations prior to eluting the protein by Imidazole. Expression and purification of each mutant was analyzed by reducing, non-reducing and anti-His western blots. Total protein concentration was determined by A₂₀₀.

### Example 12

### BIAcore Analysis of "AT" variants

The binding constant (Kd) on rate constant (Kₐ) and off rate constant (kd) for "AT" antibody variants, and off rate constant (K_{d}) for "AT" Fab variants were determined by surface plasmon resonance techniques (BIAcore) using immobilized OPGbp [143-317]. The results are shown in Tables IV - VIII. The light chain CDR3 variants described in Example 11 did not demonstrate detectable binding.

**TABLE IV**

| BIAcore Analysis of "AT" Fabs from Alanine Scanning Mutagenesis of Heavy Chain CDR3 | |
|---|---|
| | Kd (s⁻¹) |
| AT (No substituted alanines) | 0.284 |
| D95A | No detectable binding |
| S96A | 7.20x10⁻³ |
| S97A | 6.20x10⁻³ |
| N98A | 1.20x10⁻² |
| M99A | 1.12 |
| V100A | 0.261 |
| R(100a)A | 0.303 |
| G(100b)A | 0.657 |
| I(100c)A | 0.53 |
| I(100d)A | 0.622 |
| I(100e)A | 6.90x10⁻³ |
| A(100f) | 0.197 |
| Y(100g)A | 7.40x10⁻² |
| Y(100h)A | 0.368 |
| F(100i)A | 0.251 |
| D110A | 0.127 |
| Y111A | 0.414 |

**TABLE V**

| BIAcore Analysis of "AT" Abs from Alanine Scanning Mutagenesis of Heavy Chain CDR3 | | | |
|---|---|---|---|
| | | | |

| | Kₐ (M-¹s⁻¹) | K_{d} (s⁻¹) | K_{D} (nM) |
|---|---|---|---|
| AT | 1.70x10⁵ | 2.00x10⁻³ | 1.18 |
| S96A | 2.60x10⁵ | 6.40x10⁻⁴ | 2.50 |
| S97A | 5.30x10⁵ | 1.50x10⁻³ | 2.90 |
| N98A | 1.30x10⁵ | 9.00x10⁻⁴ | 0.67 |
| S96A, S97A | 3.17x10⁵ | 2.84x10⁻³ | 8.90 |
| S97A, N98A | | | No detectable binding |
| S96A, S97A, N98A | | | No detectable binding |
| S96A, N98A | | | No detectable binding |

**TABLE VI**

| BIAcore Analysis of "AT" Abs Substituted at S96 of Heavy Chain CDR3 | | | |
|---|---|---|---|
| | Kₐ (M⁻¹s⁻¹) | K_{d}(s⁻¹) | K_{D} (nM) |
| S96F | 3.17x10⁵ | 2.68x10⁻³ | 8.45 |
| S96Q | 1.16x10⁶ | 3.59x10⁻³ | 3.09 |
| S96M | 3.57x10⁵ | 8.70x10⁻³ | 2.43 |
| S96V | 5.37x10⁶ | 1.21x10⁻² | 2.25 |
| S96I | 2.99x10⁶ | 4.66x10⁻³ | 1.56 |
| S96N | 1.40x10⁶ | 2.15x10⁻³ | 1.53 |
| S96P | 1.45x10⁶ | 1.88x10⁻³ | 1.29 |
| S96W | 1.61x10⁶ | 2.03x10⁻³ | 1.26 |
| S96T | 3.14x10⁶ | 1.67x10⁻³ | 0.53 |
| S96D | 2.28x10⁶ | 1.18x10⁻³ | 0.51 |
| S96R | 6.97x10⁶ | 2.62x10⁻³ | 0.38 |
| S96E | 2.54x10⁶ | 9.01x10⁻⁴ | 0.35 |
| S96K | 4.55x10⁶ | 1.41x10⁻³ | 0.31 |
| S96L | 5.84x10⁶ | 1.75x10⁻³ | 0.30 |
| S96H | 4.12x10⁶ | 7.64x10⁻³ | 1.86 |
| S96G | 3.87x10⁵ | 1.50x10⁻² | 3.85 |
| S96Y | 2.75x10⁵ | 1.85x10⁻³ | 0.67 |

**TABLE VII**

| BIAcore Analysis of "AT" Abs Substituted at S97 of Heavy Chain CDR3 | | | |
|---|---|---|---|
| | Kₐ (M⁻¹s⁻¹) | Kd (s⁻¹) | K_{D} (nM) |
| S97F | | | No detectable binding |
| S97Y | | | No detectable binding |
| S97G | | | No detectable binding |
| S97W | | | No detectable binding |
| S97D | | | No detectable binding |
| S97E | Fast on | Fast off | 1940 |
| S97R | 4.09x10⁵ | 1.18x10⁻¹ | 28.90 |
| S97P | 1.10x10⁵ | 2.69x10⁻³ | 24.50 |
| S97M | 2.33x10⁶ | 2.90x10⁻² | 12.40 |
| S97Q | 7.30x10⁵ | 5.98x10⁻³ | 8.19 |
| S97N | 8.95x10⁵ | 4.11x10⁻³ | 4.59 |
| S97H | 8.39x10⁵ | 2.48x10⁻³ | 3.00 |
| S97T | 4.63x10⁵ | 1.16x10⁻³ | 2.51 |
| S97V | 3.04x10⁶ | 3.58x10⁻³ | 1.18 |
| S97L | 7.50x10⁵ | 6.90x10⁻⁴ | 0.92 |
| S97I | 2.10x10⁵ | 1.80x10⁻³ | 0.87 |
| S97K | 1.45x10⁷ | 5.78x10⁻³ | 0.40 |

**TABLE VIII**

| BIAcore Analysis of "AT" Abs Substituted at N98 of Heavy Chain CDR3 | | | |
|---|---|---|---|
| | Kₐ (M⁻¹s⁻¹) | K_{d} (s⁻¹) | K_{D} (nM) |
| N98K | | | No detectable binding |
| N98F | 3.70x10³ | 9.90x10⁻² | 26600 |
| N98Y | 2.72x10⁵ | 1.12x10⁻² | 41.30 |
| N98M | 2.96x10⁵ | 8.14x10⁻² | 27.50 |
| N98T | 2.46x10⁵ | 6.62x10⁻² | 26.90 |
| N98G | 2.30x10⁵ | 5.59x10⁻³ | 24.30 |
| N98I | 5.68x10⁵ | 4.36x10⁻³ | 7.60 |
| N98W | 2.62x10⁵ | 7.68x10⁻³ | 2.93 |
| N98E | 2.72x10⁵ | 3.59x10⁻³ | 1.32 |
| N98P | 1.51x10⁶ | 1.71x10⁻³ | 1.13 |
| N98H | 2.20x10⁶ | 2.34x10⁻³ | 1.06 |
| N98L | 1.48x10⁶ | 1.22x10⁻³ | 0.82 |
| N98Q | 1.86x10⁶ | 1.51x10⁻³ | 0.81 |
| N98S | 2.67x10⁶ | 1.54x10⁻³ | 0.58 |
| N98D | 6.85x10⁶ | 3.12x10⁻³ | 0.46 |
| N98V | 4.56x10⁵ | 1.97x10⁻⁴ | 0.43 |
| N98R | 3.59x10⁶ | 1.08x10⁻³ | 0.30 |

Disclosures
1. An antibody or antigen binding domain, or fragment, variant or derivative thereof, which binds to an osteoprotegerin binding protein and is an antagonist antibody.
2. The antibody of 1 wherein the osteoprotegerin binding protein is mammalian osteoprotegerin binding protein.
3. The antibody of 2 wherein the osteoprotegerin binding protein is human osteoprotegerin binding protein or an immunogenic fragment thereof.
4. The antibody of 3 wherein the immunogenic fragment comprises at least part of the extracellular domain of a human osteoprotegerin binding protein.
5. The antibody of 1 which inhibits the binding of osteoprotegerin binding protein to osteoclast differentiation and activation receptor.
6. The antibody of 1 which inhibits the formation or activation of osteoclasts.
7. The antibody of 1 which inhibits bone resorption.
8. The antibody of 1 which is selected from the group consisting of Fv, scFv, Fab, Fab' and F(ab')₂.
9. The antibody of 1 which is a human antibody.
10. An antibody or antigen binding domain which comprises:
   (a) a Fab heavy chain amino acid sequence as shown in Figure 9 (SEQ ID NO: 51) or Figure 10 (SEQ ID NO: 53);
   (b) a heavy chain amino acid sequence comprising conservative amino acid substitutions of the sequence in (a);
   (c) a heavy chain amino acid sequence which is at least about 80% identical to the sequence in (a); or
   (d) a fragment or derivative of (a), (b) or (c);
   wherein the antibody or antigen binding domain binds selectively to OPGbp.
11. The antibody of 10 further comprising a kappa or lambda light chain.
12. The antibody of 10 further comprising an human Fc region.
13. An antibody or antigen binding domain which recognizes an epitope on human OPGbp recognized by an antibody or antigen binding domain comprising the Fab heavy chain amino acid sequence as shown in Figure 9 (SEQ ID NO: 51) or Figure 10 (SEQ ID NO: 53) and Fab light amino acid sequence as shown in Figure 5 (SEQ ID NO: 43) or Figure 6 (SEQ ID NO: 45)
14. An antibody or antigen binding domain comprising a variable light (V₁) chain and a variable heavy (Vₕ) chain:
   wherein each V₁ chain comprises CDR amino acid sequences designated CDR1(V₁), CDR2(V₁) and CDR3(V₁) separated by framework amino acid sequences, CDR1(V₁) being selected from the group consisting of:
      RASQSISRYLN (SEQ ID NO: 01);
      RASQSVGSYLA (SEQ ID NO: 02);
      RASQSVSSSSLA (SEQ ID NO: 03); and
      SQDALPKQY (SEQ ID NO: 04);
      CDR2(V₁) being selected from the group consisting of:
      GASSLQS (SEQ ID NO: 05);
      DATNRAT (SEQ ID NO: 06);
      GASSRAT (SEQ ID NO: 07); and
      EDSERPS (SEQ ID NO: 08);
      and CDR3(V₁) being selected from the group consisting of:
      QHTRA (SEQ ID NO: 09);
      QHRRT (SEQ ID NO: 10);
      QQYGA (SEQ ID NO: 11); and
      QSTDSSGTYVV (SEQ ID NO: 12);
      wherein CDR1 (V₁), CDR2 (V₁) and CDR3 (V₁) are selected independently of each other; and
   wherein each Vₕ chain comprises CDR amino acid sequences designated CDR1(Vₕ), CDR2(Vₕ) and CDR3(Vₕ) separated by framework amino acid sequences, CDR1(Vₕ) being selected from the group consisting of:
      NYAIH (SEQ ID NO: 13);
      NYPMH (SEQ ID NO: 14); and
      DYAMH (SEQ ID NO: 15)
      CDR2(Vₕ) being selected from the group consisting of:
      WINAGNGNTKFSQKFQG (SEQ ID NO: 16);
      VISYDGNNKYYADSVKG (SEQ ID NO: 17); and
      GISWNSGRIGYADSVKG (SEQ ID NO: 18)
      CDR3(Vₕ) being selected from the group consisting of:
      DSSNMVRGIIIAYYFDY (SEQ ID NO: 19);
      GGGGFDY (SEQ ID NO: 20); and
      GGSTSARYSSGWYY (SEQ ID NO: 21)
   wherein CDR1(Vₕ), CDR2(Vₕ) and CDR3(Vₕ) are selected independently of each other.
15. The antibody of 14 comprising a variable light (V₁) chain and a variable heavy (Vₕ) chain wherein:
   the V₁ chain comprises CDR1 having the sequence RASQSISRYLN (SEQ ID NO: 01), CDR2 having the sequence GASSLQS (SEQ ID NO: 05), and CDR3 having the sequence QHTRA (SEQ ID NO: 09); and
   the Vₕ chain comprises CDR1 having the sequence NYAIH (SEQ ID NO: 13), CDR2 having the sequence WINAGNGNTKFSQKFQG (SEQ ID NO: 16), and CDR3 having the sequence DSSNMVRGIIIAYYFDY (SEQ ID NO: 19);
   wherein CDR1, CDR2 and CDR3 on each V1 and Vₕ chain are separated by framework amino acid sequences.
16. The antibody of 14 or 15 further comprising a human Fc region.
17. An antibody comprising a variable light (V₁) chain and a variable heavy (Vₕ) chain wherein:
   the V₁ chain comprises a rearranged or somatic variant of the germline sequence of Figure 19 (SEQ ID NO: 66); and
   the Vₕ chain comprises a rearranged or somatic variant of the germline sequence of Figure 16 (SEQ ID NO: 59); and the antibody binds selectively to an osteoprotegerin binding protein.
18. An antibody comprising a variable light (V₁) chain and a variable heavy (Vₕ) chain wherein:
   the V₁ chain comprises a rearranged or somatic variant of the germline sequence of Figure 20 (SEQ ID NO: 68); and
   the Vₕ chain comprises a rearranged or somatic variant of the germline sequence of Figure 16 (SEQ ID NO: 59); and the antibody binds selectively to an osteoprotegerin binding protein.
19. An antibody comprising a variable light (V₁) chain and a variable heavy (Vₕ) chain wherein:
   the V₁ chain comprises a rearranged or somatic variant of the germline sequence of Figure 21 (SEQ ID NO: 70); and
   the Vₕ chain comprises a rearranged or somatic variant of the germline sequence of Figure 17 (SEQ ID NO: 62); and the antibody binds selectively to an osteoprotegerin binding protein.
20. An antibody comprising a variable light (V₁) chain and a variable heavy (Vₕ) chain wherein:
   the V₁ chain comprises a rearranged or somatic variant of the germline sequence of Figure 22 (SEQ ID NO: 72); and
   the Vₕ chain comprises a rearranged or somatic variant of the germline sequence of Figure 18 (SEQ ID NO: 64); and the antibody binds selectively to an osteoprotegerin binding protein.
21. The antibody of 1 which is a monoclonal antibody, a humanized antibody, a bispecific antibody, a single chain antibody, or a heteroantibody.
22. An isolated nucleic acid molecule encoding the antibody of any of 1, 10, 13, 14, 15, 17, 18, 19, 20 or 21.
23. An expression vector comprising the nucleic acid molecule of 22.
24. A host cell comprising the expression vector of 23.
25. The host cell of 24 which is a CHO cell.
26. A method of producing an antibody comprising culturing the host cell of 25 under conditions which allow expression of the nucleic acid molecule.
27. The antibody of 1, 10, 13, 14, 15, 17, 18, 19, 20 or 21 wherein the IgG isotype is selected from IgG, IgM, IgA, IgE and IgD.
28. The antibody of 27 wherein the isotype is IgG₁, IgG₂, IgG₃ or IgG₄.
29. A composition comprising the antibody or antigen binding domain, or fragment, variant or derivative thereof, of any of 1, 10, 13, 14, 15, 17, 18, 19, 20 or 21 and a pharmaceutically acceptable carrier.
30. A method of inhibiting osteoclast formation or activation comprising administering to a mammal an effective amount of the composition of 29.
31. A method of inhibiting bone resorption comprising administering to a mammal an effective amount of the composition of 29.
32. A method of preventing or treating loss of bone mass comprising administering to a mammal a therapeutically or prophylactically effective amount of the composition of 29.
33. The method of 32 wherein loss of bone mass results from osteoporosis, metastasis of cancer to bone; rheumatoid arthritis, hypercalcemia of malignancy, and steroid-induced osteoporosis.
34. The method of any of 30, 31, 32 or 33 further comprising administering a composition comprising at least one additional bone anti-resorptive agent.
35. The method of 34 wherein the anti-resorptive agent is estrogen, a bisphosphonate, or a selective estrogen receptor modulator.
36. The method of any of 30, 31, 32 or 33 further comprising administering a composition comprising an anabolic agent for bone.
37. The method of 36 wherein the anabolic agent is parathyroid hormone or a complex of insulin-like growth factor and insulin-like growth factor binding protein.
38. The method of any of 30, 31, 32 or 33 further comprising administering an interleukin-1 inhibitor or a tumor necrosis factor-alpha inhibitor.
39. A method of preventing or treating tumor cell growth in bone comprising administering to a mammal an effective amount of the composition of 29.
40. An antibody or antigen binding domain which recognizes a DE epitope on human osteoprotegerin binding protein (OPGbp).
41. An antibody or antigen binding domain which recognizes a DE epitope on human OPGbp.
42. The antibody or antigen binding domain of 40 or 41 wherein the DE epitope comprises the sequence DLATE.
43. An antibody or antigen binding domain which binds to murine OPGbp comprising the amino acid substitutions S229D, V230L, P231A, and D233E, but does not bind to murine OPGbp lacking said substitutions.
44. The antibody or antigen binding domain of 40, 41, 42 or 43 which inhibits the formation of activation of ostoeclasts.
45. The antibody or antigen binding domain of 40, 41, 42 or 43 which inhibits bone resorption.
46. An antibody comprising a variable light (Vl) and a variable heavy (Vh) chain, wherein the Vl chain comprises CDR1 having the sequence RASQSISRYLN (SEQ ID NO: 01), CDR2 having the sequence GASSLQS (SEQ ID NO: 05), and CDR3 having the sequence QHTRA (SEQ ID NO: 09), wherein CDR1, CDR2 and CDR3 on each Vl chain are separated by framework amino acid sequences,
   and wherein the antibody binds selectively to an osteoprotegerin binding protein.
47. An antibody comprising a variable light (Vl) chain and a variable heavy (Vh) chain wherein the Vₕ chain comprises CDR1 having the sequence NYAIH (SEQ ID NO: 13), CDR2 having the sequence WINAGNGNTKFSQKFQG (SEQ ID NO: 16), and CDR3 having the sequence DSSNMVRGIIIAYYFDY (SEQ ID NO: 19), wherein CDR1, CDR2 and CDR3 on each Vh chain are separated by framework amino acid sequences,
   and wherein the antibody binds selectively to an osteoprotegerin binding protein.
48. An antibody comprising a variable light (V₁) chain and a variable heavy (Vₕ) chain wherein:
   the V₁ chain comprises CDR1 having the sequence RASQSISRYLN (SEQ ID NO: 01), CDR2 having the sequence GASSLQS (SEQ ID NO: 05), and CDR3 having the sequence QHTRA (SEQ ID NO: 09); and
   the Vₕ chain comprises CDR1 having the sequence NYAIH (SEQ ID NO: 13), CDR2 having the sequence WINAGNGNTKFSQKFQG (SEQ ID NO: 16), and CDR3 having the sequence selected from the group consisting of:
      XSSNMVRGIIIAYYFDY (SEQ ID NO: 80);
      DXSNMVRGIIIAYYFDY (SEQ ID NO: 81);
      DSXNMVRGIIIAYYFDY (SEQ ID NO: 82);
      DSSXMVRGIIIAYYFDY (SEQ ID NO: 83);
      DSSNXVRGIIIAYYFDY (SEQ ID NO: 84);
      DSSNMXRGIIIAYYFDY (SEQ ID NO: 85);
      DSSNMVXGIIIAYYFDY (SEQ ID NO: 86);
      DSSNMVRXIIIAYYFDY (SEQ ID NO: 87);
      DSSNMVRGXIIAYYFDY (SEQ ID NO: 88);
      DSSNMVRGIXIAYYFDY (SEQ ID NO: 89);
      DSSNMVRGIIXAYYFDY (SEQ ID NO: 90);
      DSSNMVRGIIIXYYFDY (SEQ ID NO: 91);
      DSSNMVRGIIIAXYFDY (SEQ ID NO: 92);
      DSSNMVRGIIIAYXFDY (SEQ ID NO: 93);
      DSSNMVRGIIIAYYXDY (SEQ ID NO: 94);
      DSSNMVRGIIIAYYFXY (SEQ ID NO: 95); and
      DSSNMVRGIIIAYYFDX (SEQ ID NO: 96);
      wherein CDR1, CDR2 and CDR3 on each V1 and Vₕ chain are separated by framework amino acid sequences and X is any amino acid different from the amino acid normally resident at that position;
   and wherein the antibody binds selectively to an osteoprotegerin binding protein.
49. An antibody comprising a variable light (V₁) chain and a variable heavy (Vₕ) chain wherein:
   the Vₕ chain comprises CDR1 having the sequence NYAIH (SEQ ID NO: 13), CDR2 having the sequence WINAGNGNTKFSQKFQG (SEQ ID NO: 16), and CDR3 having the sequence selected from the group consisting of:
      XSSNMVRGIIIAYYFDY (SEQ ID NO: 80);
      DXSNMVRGIIIAYYFDY (SEQ ID NO: 81);
      DSXNMVRGIIIAYYFDY (SEQ ID NO: 82);
      DSSXMVRGIIIAYYFDY (SEQ ID NO: 83);
      DSSNXVRGIIIAYYFDY (SEQ ID NO: 84);
      DSSNMXRGIIIAYYFDY (SEQ ID NO: 85);
      DSSNMVXGIIIAYYFDY (SEQ ID NO: 86);
      DSSNMVRXIIIAYYFDY (SEQ ID NO: 87);
      DSSNMVRGXIIAYYFDY (SEQ ID NO: 88);
      DSSNMVRGIXIAYYFDY (SEQ ID NO: 89);
      DSSNMVRGIIXAYYFDY (SEQ ID NO: 90);
      DSSNMVRGIIIXYYFDY (SEQ ID NO: 91);
      DSSNMVRGIIIAXYFDY (SEQ ID NO: 92);
      DSSNMVRGIIIAYXFDY (SEQ ID NO: 93);
      DSSNMVRGIIIAYYXDY (SEQ ID NO: 94);
      DSSNMVRGIIIAYYFXY (SEQ ID NO: 95); and
      DSSNMVRGIIIAYYFDX (SEQ ID NO: 96);
      wherein CDR1, CDR2 and CDR3 on each V1 and Vₕ chain are separated by framework amino acid sequences and X is any amino acid different from the amino acid normally resident at that position;
   and wherein the antibody binds selectively to an osteoprotegerin binding protein.
50. An antibody comprising a variable light (Vₗ) chain and a variable heavy (Vₕ) chain wherein:
   the Vₗ chain comprises CDR1 having the sequence RASQSISRYLN (SEQ ID NO: 01), CDR2 having the sequence GASSLQS (SEQ ID NO: 05), and CDR3 having the sequence QHTRA (SEQ ID NO: 09); and
   the Vₕ chain comprises CDR1 having the sequence NYAIH (SEQ ID NO: 13), CDR2 having the sequence WINAGNGNTKFSQKFQG (SEQ ID NO: 16), and CDR3 having one or more amino acid substitutions in the sequence DSSNMVRGIIIAYYFDY (SEQ ID NO: 19);
   wherein CDR1, CDR2 and CDR3 on each Vl and Vₕ chain are separated by framework amino acid sequences, and wherein the antibody binds selectively to an osteoprotegerin binding protein.
51. An antibody comprising a variable light (Vₗ) chain and a variable heavy (Vₕ) chain wherein:
   the Vₕ chain comprises CDR1 having the sequence NYAIH (SEQ ID NO: 13), CDR2 having the sequence WINAGNGNTKFSQKFQG (SEQ ID NO: 16), and CDR3 having one or more amino acid substitutions in the sequence DSSNMVRGIIIAYYFDY (SEQ ID NO: 19);
   wherein CDR1, CDR2 and CDR3 on each Vl and Vₕ chain are separated by framework amino acid sequences, and wherein the antibody binds selectively to an osteoprotegerin binding protein.
52. An antibody or antigen binding domain which binds selectively to human OPGbp with a dissociation constant (KD) of about 1nM or less.
53. An antibody or antigen binding domain which binds selectively to human OPGbp with a dissociation rate constant (kd) of about 3 x 10-3 or less.

## Claims

1. A monoclonal antibody or antigen binding domain thereof which binds to a portion of the amino acid sequence:
GGFYYLYANICFRHHETSGDLATEYLQLMVYVTKTSIKIP (SEQ ID NO: 76) from amino acid residue 212 to amino acid residue 250 of human OPGbp (osteoprotegerin binding protein), wherein the antibody or antigen binding domain thereof inhibits the formation or activation of osteoclasts mediated by human OPGbp.

2. A monoclonal antibody or antigen binding domain thereof of Claim 1 which binds to the amino acid sequence DLATE within SEQ ID NO: 76.

3. The monoclonal antibody or antigen binding domain thereof of Claims 1 or 2 which is a recombinant antibody or antigen binding domain.

4. The monoclonal antibody or antigen binding domain thereof of Claims 1 or 2 which is a chimeric antibody or antigen binding domain.

5. The monoclonal antibody or antigen binding domain thereof of Claims 1 or 2 which is a humanized antibody or antigen binding domain.

6. The monoclonal antibody or antigen binding domain thereof of Claims 1 or 2 which is a fully human antibody or antigen binding domain.

7. The monoclonal antibody or antigen binding domain thereof of Claims 1 or 2 which is a single chain antibody or antigen binding domain.

8. The monoclonal antibody or antigen binding domain thereof of Claims 1 or 2 which is a bispecific antibody or antigen binding domain.

9. The monoclonal antibody or antigen binding domain thereof of Claim 1 or 2 which is selected from Fab, Fab', F(ab')2, Fv and scFv.

10. A method of producing the monoclonal antibody or antigen binding domain thereof of Claim 1 or 2 comprising culturing a host cell expressing the antibody or antigen binding domain and recovering the antibody or antigen binding domain from the culture medium or from the host cell.

11. The method of Claim 10 wherein the host cell is a eukaryotic cell.

12. The method of Claim 10 wherein the host cell is a prokaryotic cell.

13. A composition comprising the monoclonal antibody or antigen-binding domain thereof of Claims 1 or 2 and a pharmaceutically acceptable carrier.

14. Use of a monoclonal antibody or an antigen-binding domain thereof of Claims 1 or 2 for the preparation of a pharmaceutical composition for inhibiting osteoclast formation or activation.

15. Use of a monoclonal antibody or an antigen-binding domain thereof of Claims 1 or 2 for the preparation of a pharmaceutical composition for inhibiting bone resorption.

16. of a monoclonal antibody or an antigen-binding domain thereof of Claims 1 or 2 for the preparation of a pharmaceutical composition for preventing or treating loss of bone mass.

17. The use of Claim 16 wherein loss of bone mass results from osteoporosis, metastasis of cancer to bone; rheumatoid arthritis, hypercalcemia of malignancy, and steroid-induced osteoporosis.

18. The use of any of Claims 14 to 17 wherein a composition comprising at least one bone anti-resorptive agent is further administered.

19. The use of Claim 18 wherein the anti-resorptive agent is estrogen, a bisphosphonate, or a selective estrogen receptor modulator.

20. The use of any of Claims 14 to 17, wherein a composition comprising an anabolic agent for bone is further administered.

21. The use of Claim 20, wherein the anabolic agent is parathyroid hormone or a complex of insulin-like growth factor and insulin-like growth factor binding protein.

22. The use of any of Claims 14 to 17, wherein an interleukin-1 inhibitor or a tumor necrosis factor-alpha inhibitor is further administered.

## Patentansprüche

1. Ein monoklonaler Antikörper oder eine Antigen-Bindungsdomäne davon, welcher einen speziellen Teil der folgenden Aminosäuresequenz bindet:
GGFYYLYANICFRHHETSGDLATEYLQLMVYVTKTSIKIP (SEQ ID NO: 76) aus dem Abschnitt von Aminosäurerest 212 bis Aminosäurerest 250 von menschlichem OPGbp (Osteoprotegerin-Bindung-Bindungsprotein), wobei der Antikörper oder die Antigenbindungsdomäne davon die Ausbildung oder Aktivierung von Osteoklasten, mediatisiert durch menschliches OPGbp, inhibiert.

2. Ein monoklonaler Antikörper oder eine Antigen-Bindungsdomäne davon gemäß Anspruch 1, welche an die Aminosäuresequenz DLATE innerhalb SEQ ID NO: 76 bindet.

3. Der monoklonale Antikörper oder die Antigen-Bindungsdomäne davon gemäß Anspruch 1 oder 2, welche eine rekombinanter Antikörper oder eine Antigenbindungsdomäne ist.

4. Der monoklonale Antikörper oder die Antigen-Bindungsdomäne davon gemäß Anspruch 1 oder 2, welcher eine chimärer Antikörper oder eine Antigen-Bindungsdomäne ist.

5. Der monoklonale Antikörper oder eine Antigen-Bindungsdomäne davon gemäß Anspruch 1 oder 2, welcher ein humanisierter Antikörper oder eine Antigen-Bindungsdomäne ist.

6. Der monoklonale Antikörper oder die Antigen-Bindungsdomäne davon gemäß Anspruch 1 oder 2, welcher ein vollständiger humaner Antikörper oder eine Antigen-Bindungsdomäne ist.

7. Der monoklonale Antikörper oder eine Antigen-Bindungsdomäne davon gemäß Anspruch 1 oder 2, welcher ein einzelkettiger Antikörper oder eine Antigen-Bindungsdomäne ist.

8. Der monoklonale Antikörper oder eine Antigen-Bindungsdomäne davon gemäß Anspruch 1 oder 2, welcher ein bispezifischer Antikörper oder eine Antigen-Bindungsdomäne ist.

9. Der monoklonale Antikörper oder die Antigen-Bindungsdomäne davon gemäß Anspruch 9, wobei das Fragment ausgewählt ist aus Fab, Fab', F(ab')2, Fv und scFv.

10. Ein Verfahren zum Herstellen eines monoklonalen Antikörpers oder einer Antigen-Bindungsdomäne davon gemäß Anspruch 1 oder 2 umfassend das Kultivieren einer Wirtszelle, welche den Antikörper oder die Antigen-Bindungsdomäne davon exprimiert und Rückgewinnen des Antikörpers oder der Antigen-Bindungsdomäne aus dem Kulturmedium oder aus der Wirtszelle.

11. Das Verfahren gemäß Anspruch 11, wobei die Wirtszelle eine eukaryontische Zelle ist.

12. Das Verfahren gemäß Anspruch 11, wobei die Wirtszelle eine prokaryontische Zelle ist.

13. Eine Zusammensetzung umfassend den monoklonalen Antikörper oder ein Antigen-Bindungsdomäne davon gemäß Anspruch 1 oder 2 und einen pharmazeutisch verträglichen Träger.

14. Verwendung eines monoklonalen Antikörpers oder einer Antigen-Bindungsdomäne davon gemäß Anspruch 1 oder 2 zur Herstellung einer pharmazeutischen Zusammensetzung zum Inhibieren der Osteoklasten-Ausbildung oder -Aktivierung.

15. Verwendung eines monoklonalen Antikörpers oder Antigen-Bindungsdomäne davon von Anspruch 1 oder 2 zur Herstellung einer pharmazeutischen Zusammensetzung zum Inhibieren der Knochenresorption.

16. Verwendung eines monoklonalen Antikörpers oder Antigen-Bindungsdomäne davon gemäß Anspruch 1 oder 2 zur Herstellung einer pharmazeutischen Zusammensetzung zum Vorbeugen oder Behandeln des Verlustes von Knochenmasse.

17. Die Verwendung gemäß Anspruch 17, wobei der Verlust an Knochenmasse von Osteoporose, Metastasen von Krebs im Knochen; rheumatoider Arthritis, Hyperkalzemie durch bösartige Wucherungen und steroidinduzierte Osteoporose herrührt.

18. Die Verwendung gemäß irgendeiner der Ansprüche 15 bis 18, wobei eine Zusammensetzung, welche zumindest einen Knochen-Anti-Resorptions-Wirkstoff umfasst, weiter verabreicht werden soll.

19. Die Verwendung gemäß Anspruch 19, wobei der Anti-Resorptions-Wirkstoff Östrogen ist, ein Bisphosphonat, oder ein selektiver Östrogen-Rezeptor Modulator.

20. Die Verwendung gemäß irgendeiner der Ansprüche 15 bis 18, wobei die Zusammensetzung umfassend einen anabolen Wirkstoff für Knochen weiter verabreicht werden soll.

21. Die Verwendung gemäß Anspruch 21, wobei der anabole Wirkstoff Parathyroidhormon oder ein Komplex eines Insulin-ähnlichen Wachstumsfaktors und einem Insulinähnlichem Wachstumsfaktor bindenden Protein darstellt.

22. Die Verwendung gemäß irgendeiner der Ansprüche 15 bis 18, wobei ein Interleukin-1 Inhibitor oder ein Tumornekrosefaktor-Alpha-Inhibitor weiter verabreicht werden soll.

## Revendications

1. Anticorps monoclonal ou domaine de liaison à l'antigène de celui-ci qui se lie à une partie de la séquence d'acides aminés :
GGFYYLYANICFRHHETSGDLATEYLQLMVYVTKTSIKIP (SEQ ID n° : 76) du résidu d'acides aminés 212 au résidu d'acides aminés 250 de l'OPGbp humaine (protéine de liaison ostéoprotégérine), l'anticorps ou un domaine de liaison à l'antigène de celui-ci inhibant la formation ou l'activation des ostéoclastes à médiation par l'OPGbp humaine.

2. Anticorps monoclonal ou domaine de liaison à l'antigène de celui-ci selon la revendication 1 qui se lie à la séquence d'acides aminés DLATE à l'intérieur de SEQ ID n° : 76.

3. Anticorps monoclonal ou domaine de liaison à l'antigène de celui-ci selon les revendications 1 ou 2 qui est un anticorps recombinant ou un domaine de liaison à l'antigène.

4. Anticorps monoclonal ou domaine de liaison à l'antigène de celui-ci selon les revendications 1 ou 2 qui est un anticorps chimère ou un domaine de liaison à l'antigène.

5. Anticorps monoclonal ou domaine de liaison à l'antigène de celui-ci selon les revendications 1 ou 2 qui est un anticorps humanisé ou un domaine de liaison à l'antigène.

6. Anticorps monoclonal ou domaine de liaison à l'antigène de celui-ci selon les revendications 1 ou 2 qui est un anticorps ou un domaine de liaison à l'antigène entièrement humain.

7. Anticorps monoclonal ou domaine de liaison à l'antigène de celui-ci selon les revendications 1 ou 2 qui est un anticorps à chaîne unique ou un domaine de liaison à l'antigène.

8. Anticorps monoclonal ou domaine de liaison à l'antigène de celui-ci selon les revendications 1 ou 2 qui est un anticorps bispécifique ou un domaine de liaison à l'antigène.

9. Anticorps monoclonal ou domaine de liaison à l'antigène de celui-ci selon les revendications 1 ou 2 qui est sélectionné parmi Fab, Fab', F(ab')2, Fv et scFv.

10. Procédé de production de l'anticorps monoclonal ou de son domaine de liaison à l'antigène selon les revendications 1 ou 2 comprenant la culture d'une cellule hôte exprimant l'anticorps ou le domaine de liaison à l'antigène et la récupération de l'anticorps ou du domaine de liaison à l'antigène du milieu de culture ou de la cellule hôte.

11. Procédé selon la revendication 10 dans lequel la cellule hôte est une cellule eucaryote.

12. Procédé selon la revendication 10 dans lequel la cellule hôte est une cellule procaryote.

13. Composition comprenant l'anticorps monoclonal ou un domaine de liaison à l'antigène de celui-ci selon les revendications 1 ou 2 et un support pharmaceutiquement acceptable.

14. Utilisation d'un anticorps monoclonal ou d'un domaine de liaison à l'antigène de celui-ci selon les revendications 1 ou 2 pour la préparation d'une composition pharmaceutique pour inhiber la formation ou l'activation des ostéoclastes.

15. Utilisation d'un anticorps monoclonal ou d'un domaine de liaison à l'antigène de celui-ci selon les revendications 1 ou 2 pour la préparation d'une composition pharmaceutique d'inhibition de la résorption osseuse.

16. Utilisation d'un anticorps monoclonal ou d'un domaine de liaison à l'antigène de celui-ci selon les revendications 1 ou 2 pour la préparation d'une composition pharmaceutique de prévention ou de traitement de la perte de masse osseuse.

17. Utilisation selon la revendication 16, dans laquelle la perte de masse osseuse résulte de l'ostéoporose, de métastases d'un cancer des os ; de l'arthrite rhumatoïde, de l'hypercalcémie d'origine tumorale, et de l'ostéoporose induite par les stéroïdes.

18. Utilisation selon l'une quelconque des revendications 14 à 17, dans laquelle une composition comprenant au moins un agent anti-résorption osseuse est en outre administrée.

19. Utilisation selon la revendication 18, dans laquelle l'agent anti-résorption est un oestrogène, un bisphosphonate, ou un modulateur sélectif du récepteur des oestrogènes.

20. Utilisation selon l'une quelconque des revendications 14 à 17, dans laquelle une composition comprenant un agent anabolique osseux est en outre administrée.

21. Utilisation selon la revendication 20, dans laquelle l'agent anabolique est l'hormone parathyroïdienne ou un complexe de facteur de croissance type insuline et d'une protéine de liaison au facteur de croissance type insuline.

22. Utilisation selon l'une quelconque des revendications 14 à 17, dans laquelle un inhibiteur de l'interleukine-1 ou un inhibiteur du facteur de nécrose de tumeur-alpha est en outre administré.
